(19) Europäisches Patentamt
European Patent Office
Office européen des brevets

(11) Publication number: **0 395 768 A1**

# (12) EUROPEAN PATENT APPLICATION published in accordance with Art. 158(3) EPC

(21) Application number: 89906430.7

(22) Date of filing: 25.05.89

(86) International application number: PCT/JP89/00522

(87) International publication number: WO 90/05132 (17.05.90 90/11)

(51) Int. Cl.[5]: C07D 207/325, C07D 207/33, C07D 213/24, C07D 233/56, C07D 239/26, C07D 249/06, C07D 261/08, C07D 263/32, C07D 271/10, C07D 275/02

(30) Priority: 11.11.88 JP 285381/88

(43) Date of publication of application: 07.11.90 Bulletin 90/45

(84) Designated Contracting States: AT BE CH DE FR GB IT LI LU NL SE

(71) Applicant: BANYU PHARMACEUTICAL CO., LTD.
2-3, Nihonbashi Honcho 2-chome
Chuo-ku Tokyo 103(JP)

(72) Inventor: TAKEZAWA, Hiroshi
879-91, Uchikoshi-machi Hachioji-shi
Tokyo 192(JP)
Inventor: HAYASHI, Masahiro
19-15-311, Fukuei 3-chome Ichikawa-shi
Chiba 272-01(JP)
Inventor: IWASAWA, Yoshikazu
235, Nishikoiso Oiso-machi
Naka-gun Kanagawa 255(JP)
Inventor: HOSOI, Masaaki
1-51, Kajigaya 5-chome Takatsu-ku
Kawasaki-shi Kanagawa 213(JP)
Inventor: IIDA, Yoshiaki
922, Imai-cho Hodogaya-ku
Yokohama-shi Kanagawa 240(JP)
Inventor: TSUCHIYA, Yoshimi
45-7-402, Higashifunabashi 3-chome
Funabashi-shi Chiba 273(JP)
Inventor: HORIE, Masahiro
5-17, Tokiwadai 2-chome
Itabashi-ku Tokyo 174(JP)
Inventor: KAMEI, Toshio
11-8, Hagoromo-cho 2-chome
Tachikawa-shi Tokyo 190(JP)

(74) Representative: Kraus, Walter, Dr. et al
Patentanwälte Kraus, Weisert & Partner
Thomas-Wimmer-Ring 15
D-8000 München 22(DE)

EP 0 395 768 A1

(54) **SUBSTITUTED ALLYLAMINE DERIVATIVES, PROCESS FOR THEIR PREPARATION AND THEIR USE.**

(57)

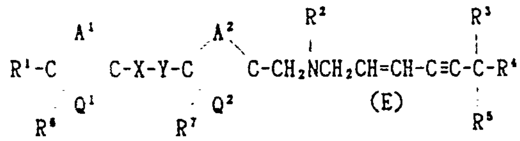

[I]

The invention relates to substituted allylamine derivatives and nontoxic salts thereof useful as a cholesterol-depressing agent and represented by general formula (I), [wherein $A^1$ and $A^2$ each represents a methine group, N, O or S; $Q^1$ and $Q^2$ may contain 1 to 2 hetero atoms and form a 5- or 6-membered aromatic ring together with $A^1$ or $A^2$; X and Y each represents O, S, a carbonyl group, $-CHR^a-$ (wherein $R^a$ represents H or an alkyl group) or $-NR^b-$ (wherein $R^b$ represents H or an alkyl group) or, when taken together, X and Y represent a vinylene group or an ethynylene group; $R^1$ represents a 5- or 6-membered heterocyclic ring containing 1 to 4 hetero atoms; $R^2$ represents an alkyl, allyl, propargyl or cyclopropyl group; $R^3$ and $R^4$ each represents an alkyl group or, when taken together, $R^3$ and $R^4$ represent a cycloalkane together with the adjacent carbon atom; $R^5$ represents H, an alkyl or alkoxy group; and $R^6$ and $R^7$ each represents H, halogen, -OH, -CN, an alkyl or alkoxy group, provided that when one of X and Y represents O, S or $-NR^b-$ (wherein $R^b$ is the same as described above), the other represents a carbonyl group or $-CHR^a-$ (wherein $R^a$ is the same as described above)], a process for their preparation, and their use for treatment of hypercholesteremia, hyperlipemia and arteriosclerosis.

MODIFIED
front page



SPECIFICATION

Substituted Allylamine Derivatives, Processes for Production Thereof and Use Thereof

**Technological Field**

This invention relates to novel substituted allylamine derivatives, and more specifically substituted allylamine derivatives and their salts which are useful in the field of pharmaceuticals, particularly for the treatment and prevention of hypercholesterolemia, hyperlipemia and arteriosclerosis, processes for production thereof, and their use.

**Background Technology**

Arteriosclerosis is a degenerative arterial disease which has closely to do with aging and diet, and is regarded as the cause of coronary and cerebral arterial diseases, the principal cause of death in the present day. Arteriosclerosis begins in early ages as deposition of lipid on the endothelia of large vessels, and with age, its degree increases. It will finally show clinical symptoms as ischemic heart diseases such as myocardial infarction and angina pectoris, cerebral arteriosclerosis such as cerebral infarction, and aneurism. It is known that the increase of various blood lipids is involved in this lipid deposition. In particular, the increase of blood cholesterol is the most prominent risk factor, and decreasing the blood cholesterol level to a normal value is the most effective therapeutic and prophylactic means against arteriosclerosis. It is said that in humans, more than 50 % of cholesterol is derived from de novo biosynthesis. Nowadays, lovastatin and eptastatin which are inhibitors of enzymes in the process of de novo biosynthesis are clinically used as hypocholesterolemic agents [see, for example, A. W. Alberts et al., Proc. Natl. Acad. Sci., vol. 77, page 3957 (1980); and Tsujita et al., Biochim. Biophs. Acta, vol. 877, page 50 (1986)]. However, since

3-hydroxy-3-methyl glutaryl-coenzyme A reductase, a target enzyme of these inhibitors, is positioned in the early stage of the cholesterol biosynthesis pathway, the administration of these drugs will also inhibit formation of dolichol and ubiquinone which are other biologically important metabolites. Furthermore, it was reported that triparanol, an inhibitor of the later stage of the cholesterol biosynthesis pathway, becomes the cause of cataract due to the accumulation of desmosterol. Since squalene epoxidase is positioned in the middle stage of the cholesterol biosynthesis pathway, an inhibitor of this enzyme is expected to solve these problems and serve as a hypocholesterolemic agent with high safety.

Some compounds have already been known as inhibitors of squalene epoxidase [see G. Petranyi et al., Science, vol. 224, page 1239 (1984)]. All of these, however, were developed as antimycotic agents which inhibit fungal squalene epoxidase selectively. No inhibitor has been known which inhibits mammalian enzyme and has utility as an hypocholesterolemic agent.

It is a primary object of this invention to provide a hypolipemic agent, and a therapeutic and prophylactic agent for arteriosclerosis which is safer and better than conventional hypolipemic agents.

The present inventors investigated squalene epoxidase inhibitors having hypocholesterolemic activity in order to develop a novel antiarteriosclerotic agent, and have found that substituted allylamine derivatives of general formula [I] given below selectively inhibit squalene epoxidase of mammals, and have strong hypocholesterolemic activity.

**Disclosure of the Invention**

This invention provides a substituted allylamine derivative represented by the following general formula

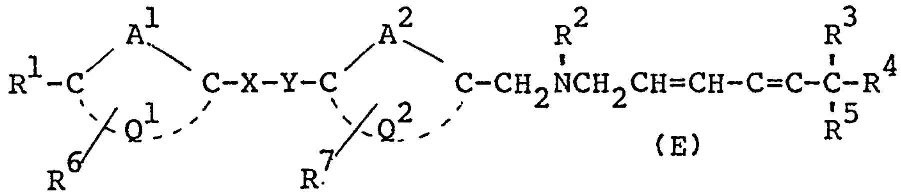

[I]

wherein

$A^1$ and $A^2$ are identical or different and each represents a methine group or a nitrogen, oxygen or sulfur atom;

$Q^1$ and $Q^2$ are identical or different and each represents a group which may contain 1 or 2 hetero atoms selected from the class consisting of nitrogen, oxygen and sulfur atoms and which forms a 5- or 6-membered aromatic ring together with the adjoining carbon atoms and $A^1$ or $A^2$;

X and Y are identical or different, and each represents an oxygen or sulfur atom, a carbonyl group, a group of the formula $-CHR^a-$ in which $R^a$ represents a hydrogen atom or a lower alkyl group, or a group of the formula $-NR^b-$ in which $R^b$ represents a hydrogen atom or a lower alkyl group, or when taken together, X and Y form a vinylene or ethynylene group;

$R^1$ represents a 5- or 6-membered heterocyclic group containing 1 to 4 hetero atoms selected from the class consisting of nitrogen, oxygen and sulfur atoms;

$R^2$ represents a lower alkyl group, an allyl group, a propargyl group or a cyclopropyl group;

$R^3$ and $R^4$ are identical or different and each represents a lower alkyl group, or they represent groups which when taken together, form a cycloalkane together with the adjoining carbon atom;

$R^5$ represents a hydrogen atom, a lower alkyl group or a lower alkoxy group; and

$R^6$ and $R^7$ are identical or different and each represents a hydrogen atom, a halogen atom, a hydroxyl group, a cyano group, a lower alkyl group, or a lower alkoxy group;

with the proviso that when one of X and Y represents an oxygen atom, a sulfur atom or the group $-NR^b-$ (where $R^b$ is as defined), the other represents a carbonyl group or the group $-CHR^a-$ (where $R^a$ is as defined);

and its nontoxic salt, processes for production thereof, and its use in the treatment of hypercholesterolemia, hyperlipemia and arteriosclerosis.

The present invention will be described in detail.

It is known that allylamine derivatives typified by naftifine and terbinafine having the following structural formulae

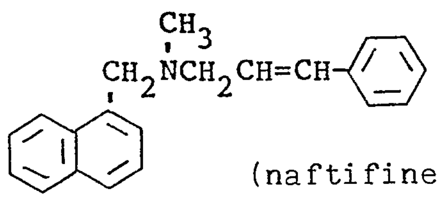 and

(naftifine)

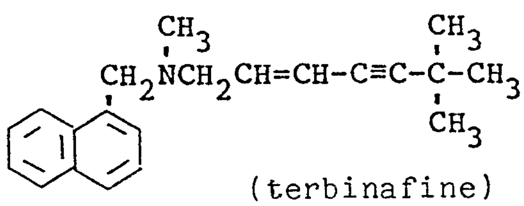

(terbinafine)

show strong inhibitory activity against the squalene-epoxidase of Eumycetes, and are consequently useful as antimycotic agents [see G. Petranyi et al.: Science, Vol. 244, page 1239 (1984)]. However, these compounds hardly show inhibitory activity against the squalene-epoxidase of mammals including man, and cannot be an inhibitor of cholesterol synthesis [see N. S. Ryder et al.: Biochemical, Journal, Vol. 230, page 765 (1985)].

The present inventors made extensive investigations in order to develop a drug which acts selectively on mammalian squalene-epoxidase and shows hypocholesterolemic activity. Consequently, the present

inventors found that a compound which shows strong inhibitory activity against mammalian squalene-epoxidase can be obtained when the naphthalene ring of the naftifine and terbinafine mentioned above is replaced by a 1,3-substituted 5- or 6-membered aromatic group of the following formula

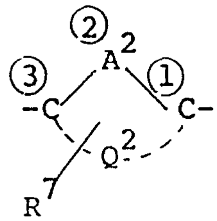

wherein $R^7$, $A^2$ and $Q^2$ are as defined, and its 3-position is substituted by a group of the formula

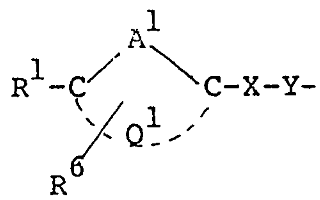

in which $R^1$, $R^6$, $A^1$, $Q^1$, X and Y are as defined.

The present inventors also found that the squalene-epoxidase inhibitory activity of the compound of general formula [I] is very selective and scarcely shows activity against enzymes of Eumycetes, and that this compound is very valuable as a drug for treating or preventing hypercholesterolemia, hyperlipemia and arteriosclerosis.

Now, the definitions and specific examples of the various terms mentioned in the description of this specification will be explained.

The term "lower", used to qualify a group or a compound, means that the group or compound so qualified has not more than 6, preferably not more than 4, carbon atoms.

Accordingly, the lower alkyl group includes, for example, linear or branched alkyl groups having 1 to 6 carbon atoms, such as methyl, ethyl, propyl, isopropyl, butyl, isobutyl, sec-butyl, tert-butyl, pentyl, isopentyl, neopentyl and hexyl groups. Examples of the lower alkoxy groups preferably include linear or branched alkoxy groups having 1 to 4 carbon atoms such as methoxy, ethoxy, propoxy, isopropoxy, butoxy, isobutoxy, sec-butoxy and tert-butoxy groups. The cycloalkane means a cycloalkane having 3 to 6 carbon atoms, and its specific examples are cyclopropane, cyclobutane, cyclopentane and cyclohexane. The halogen atom means a fluorine, chlorine, bromine or iodine atom.

To disclose the compound of this invention represented by general formula [I] more specifically, the various symbols used in formula [I] will be explained in detail by citing preferred examples.

Examples of the 5- or 6-membered heterocyclic group containing 1 to 4 hetero atoms selected from nitrogen, oxygen and sulfur atoms which can be represented by $R^1$ include aromatic heterocyclic groups such as pyrrolyl, furyl, thienyl, oxazolyl, isoxazolyl, thiazolyl, isothiazolyl, imidazolyl, pyrazolyl, oxadiazolyl, thiadiazolyl, triazolyl, tetrazolyl, furazanyl, pyridyl, pyridazinyl, pyrimidinyl, pyrazinyl and triazinyl groups; non-aromatic heterocyclic groups such as dihydrothienyl, tetrahydrothienyl, pyrrolinyl, pyrrolidinyl, oxazolinyl, oxazolidinyl, isoxazolinyl, isoxazolidinyl, thiazolinyl, thiazolidinyl, isothiazolinyl, isothiazolidinyl, 1,2-dithiolanyl, 1,3-dithiolanyl, 1,2-dithiolyl, 1,3-dithiolyl, dihydrothiopyranyl, tetrahydrothiopyranyl, 1,4-dithianyl, 1,4-dithiinyl, 1,4-oxathiinyl and thiomorpholinyl groups. Of these, thienyl, pyrrolyl, oxazolyl, isoxazolyl, thiazolyl, isothiazolyl, imidazolyl, pyridyl and dihydrothienyl groups are preferred. Especially preferred are 3-thienyl, 1-pyrrolyl, 5-oxazolyl,

4-isoxazolyl, 5- isoxazolyl, 4-thiazolyl, 5-thiazolyl, 3-isothiazolyl, 4-isothiazolyl, 5-isothiazolyl, 3-pyridyl, 2,3-dihydro-4-thienyl and 2,5-dihydro-3-thienyl groups.

X and Y may be identical or different as stated above, and each represents an oxygen atom, a sulfur atom, a carbonyl group, a group of the formula $-CHR^a-$ (in which $R^a$ represents a hydrogen atom or a lower alkyl group) or a group of the formula $-NR^b-$ (in which $R^b$ represents a hydrogen atom or a lower alkyl group; or when taken together, X and Y represent a vinylene group or ethynylene group, provided that when one of X and Y represents an oxygen atom, a sulfur atom, or a group of the formula $-NR^b-$, the other represents a carbonyl group or a group of the formula $-CHR^a-$. Specifically, the group represented by -X-Y- may be, for example, $-(CHR^a)_2-$, $-CHR^aO-$, $-OCHR^a-$, $-CHR^aS-$, $-SCHR^a-$, $-CHR^aNR^b-$, $-NR^bCHR^a-$, $-CHR^aCO-$, $-COCHR^a-$, -COO-, -OCO-, -COS-, -SCO-, $-CONR^b-$, $-NR^bCO-$, -CH=CH- and -C≡C- (in the formulae, $R^a$ and $R^b$ are as defined above). Of these, an ethylene group, a (E)-vinylene group, a group of formula $-CH_2O-$, a group of the formula $-CH_2S-$ and a group of the formula $-CH_2NH-$ are preferred.

$R^2$ represents a lower alkyl group, an allyl group, a propargyl group or a cyclopropyl group. Examples of preferred lower alkyl groups are linear or branched lower alkyl groups having 1 to 5 carbon atoms such as methyl, ethyl, propyl, isopropyl, butyl, isobutyl and pentyl groups. Methyl, ethyl, propyl, allyl or propargyl groups are preferred as a substituent on $R^2$, and methyl, ethyl and propyl groups are most preferred.

$R^3$ and $R^4$ are identical or different and each represents a lower alkyl group, or when bonded together, they represent a group forming a cycloalkane together with the adjacent carbon atom. Preferred lower alkyl groups are linear lower alkyl groups having 1 to 4 carbon

atoms such as methyl, ethyl, propyl and butyl group. Examples of preferred cycloalkanes are those having 3 to 6 carbon atoms such as cyclopropane, cyclobutane, cyclopentane and cyclohexane.

As a substituent on $R^3$ and $R^4$, a methyl group, an ethyl group, a propyl group and a group which forms a cyclopropane ring together with the adjacent carbon atom are preferred, and the methyl group is most preferred.

$R^5$ represents a hydrogen atom, a lower alkyl group or a lower alkoxy group. Preferred lower alkyl groups are linear or branched lower alkyl groups having 1 to 5 carbon atoms such as methyl, ethyl, propyl, isopropyl, butyl, isobutyl, sec-butyl, tert-butyl and pentyl groups. Examples of preferred lower alkoxy groups are linear or branched alkoxy groups having 1 to 4 carbon atoms such as methoxy, ethoxy, propoxy, isopropoxy, butoxy, isobutoxy, sec-butoxy and tert-butoxy groups. Of these, methyl, ethyl, propyl, isopropyl, methoxy, ethoxy, propoxy and isopropoxy groups are preferred. The methyl, ethyl and methoxy groups are most preferred.

The aromatic rings represented by the formula

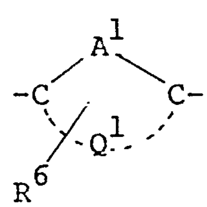 or 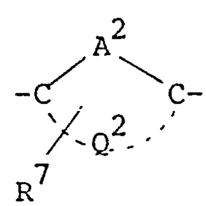

may be identical or different and preferably include aromatic rings which may contain 1 to 3 hetero atoms selected from the group consisting of nitrogen, oxygen and sulfur atoms, for example, benzene, pyrrole, furan, thiophene, oxazole, isoxazole, thiazole, isothiazole, imidazole, 1,3,4-oxadiazole, 1,3,4-thiadiazole, pyridine, pyridazine, pyrimidine, pyrazine and triazine rings. The aromatic ring represented by

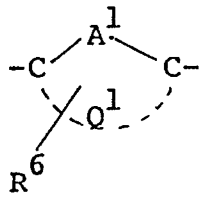

is especially preferably a benzene or thiophene ring.

Most preferably, the above aromatic rings are unsubstituted. If desired, they may have a substituent such as a halogen atom, a hydroxyl group, a cyano group, a lower alkyl group or a lower alkoxy group. Examples of the substituent are a hydroxyl group, a fluorine atom, a chlorine atom, a methyl group, an ethyl group and a methoxy group.

Thus, preferred examples of the compound provided by this invention are substituted allylamine derivatives of general formula [I] in which $R^1$ is a pyrrolyl, furyl, thienyl, oxazolyl, isoxazolyl, thiazolyl, isothiazolyl, imidazolyl, pyrazolyl, oxadiazolyl, thiadiazolyl, triazolyl, tetrazolyl, furazanyl, pyridyl, pyridazinyl, pyrimidinyl, pyrazinyl, triazinyl, dihydrothienyl, tetrahydrothienyl, pyrrolinyl, pyrrolidinyl, oxazolinyl, oxazolidinyl, isoxazolinyl, isoxazolidinyl, thiazolinyl, thiazolidinyl, isothiazolinyl, isothiazolidinyl, 1,2-dithiolanyl, 1,3-dithiolanyl, 1,2-dithiolyl, 1,3-dithiolyl, dihydrothiopyranyl, tetrahydrothiopyranyl, 1,4-dithianyl, 1,4-dithiinyl, 1,4-oxathiinyl or thiomorpholinyl group; the 5- or 6-membered aromatic rings represented by the formula

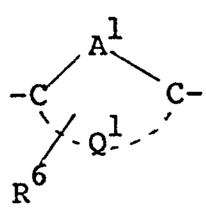 or 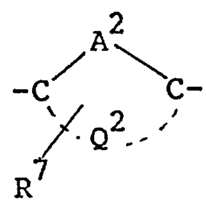

are identical or different, and each represents a benzene, pyrrole, furan, thiophene, oxazole, isoxazole, thiazole,

isothiazole, imidazole, 1,3,4-oxadiazole, 1,3,4-thiadiazole, pyridine, pyridazine, pyrimidine, pyrazine or triazine ring; X and Y are identical or different and each represents an oxygen atom, a sulfur atom, a carbonyl group, a group of the formula $-CHR^a-$ ($R^a$ represents a hydrogen atom or a lower alkyl group) or a group of the formula $-NR^b-$ ($R^b$ represents a hydrogen atom or a lower alkyl group), or taken together, X and Y represents a vinylene or ethynylene group [with the proviso that when one of X and Y is an oxygen atom, a sulfur atom or the group $-NR^b-$ ($R^b$ is as defined), the other is a carbonyl group or the group $-CHR^a-$ ($R^a$ is as defined)]; $R^2$ is a lower alkyl group, an allyl group, a propargyl group or a cyclopropyl group; $R^3$ and $R^4$ are identical or different and each represents a lower alkyl group, or the two are bonded to represent a group forming a cycloalkane together with the adjacent carbon atom; $R^5$ is a hydrogen atom, a lower alkyl group or a lower alkoxy group; and $R^6$ and $R^7$ are identical or different and each represents a hydrogen atom, a halogen atom, a hydroxyl group, a cyano group, a lower alkyl group or a lower alkoxy group.

A more preferred group of compounds of general formula [I] include those in which the aromatic ring of the formula

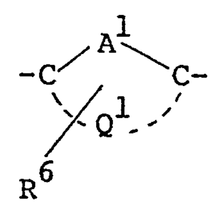

is a benzene or thiophene ring.

A further preferred group of the compounds of general formula [I] are those in which $R^1$ is a thienyl, pyrrolyl, oxazolyl, isoxazolyl, thiazolyl, isothiazolyl, imidazolyl, pyridyl or dihydrothienyl group; the aromatic ring of the formula

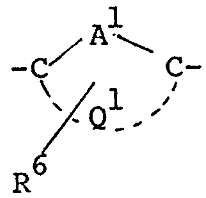

is a benzene or thiophene ring; and the aromatic ring represented by the following formula

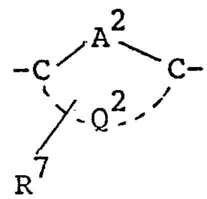

is a benzene, pyrrole, furan, thiophene, oxazole, isoxazole, thiazole, isothiazole, imidazole, 1,3,4-oxadiazole, 1,3,4-thiadiazole, pyridine, pyridazine, pyrimidine, pyrazine or triazine ring.

A still further preferred group of the compounds of general formula [I] are those in which $R^1$ is a 3-thienyl, 1-pyrrolyl, 5-oxazolyl, 4-isoxazolyl, 5-isoxazolyl, 4-thiazolyl, 5-thiazolyl, 3-isothiazolyl, 4-isothiazolyl, 5-isothiazolyl, 3-pyridyl, 2,3-dihydro-4-thienyl or 2,5-dihydro-3-thienyl group; and the aromatic ring represented by the formula

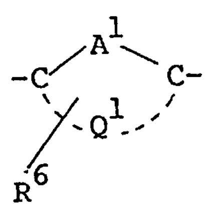

is a benzene or thiophene ring; and the aromatic ring of the formula

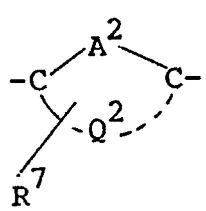

is a benzene, furan, thiophene, oxazole, isoxazole, thiazole, isothiazole, 1,3,4-oxadiazole, 1,3,4-thiadiazole, pyridine, pyridazine, pyrimidine or pyrazine ring.

An especially preferred group of compounds of formula [I] are those in which $R^1$ is a 3-thienyl, 1-pyrrolyl, 5-oxazolyl, 4-isoxazolyl, 5-isoxazolyl, 4-thiazolyl, 5-thiazolyl, 3-isothiazolyl, 4-isothiazolyl, 5-isothiazolyl, 3-pyridyl, 2,3-dihydro-4-thienyl or 2,5-dihydro-3-thienyl group; X is a methylene group; Y is a methylene group, an oxygen atom, a sulfur atom or an imino group or when taken together, X and Y represent an (E)-vinylene group; the aromatic ring represented by the following formula

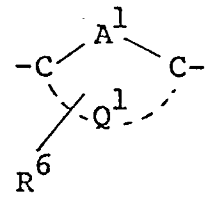

is a benzene or thiophene ring; and the aromatic ring of the following formula

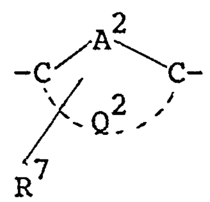

is a benzene, furan, thiophene, oxazole, isoxazole, thiazole, isothiazole, 1,3,4-oxadiazole, 1,3,4-thiadiazole, pyridine, pyridazine, pyrimidine, or pyrazine ring; $R^2$ is a lower alkyl group, an allyl group, a propargyl group or a cyclopropyl group; $R^3$ and $R^4$ are identical or different and each represents a lower alkyl group or the two are bonded to represent a group forming a cycloalkane together with the adjacent carbon atom; $R^5$ represents a hydrogen atom, a lower alkyl group or a

lower alkoxy group; and $R^6$ and $R^7$ are identical or different and each represents a hydrogen atom, a halogen atom, a hydroxyl group, a cyano group, a lower alkyl group or a lower alkoxy group.

A most preferred group of compounds of formula [I] are those in which $R^1$ is a 3-thienyl, 1-pyrrolyl, 5-oxazolyl, 4-isoxazolyl, 5-isoxazolyl, 4-thiazolyl, 5-thiazolyl, 3-isothiazolyl, 4-isothiazolyl, 5-isothiazolyl, 3-pyridyl, 2,3-dihydro-4-thienyl or 2,5-dihydro-3-thienyl group; the aromatic ring represented by the formula

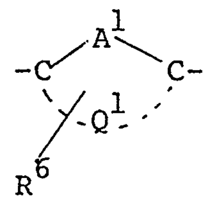

is a benzene or thiophene ring, and the aromatic ring represented by the following formula

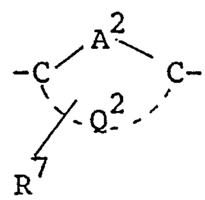

is a benzene, furan, thiophene, oxazole, isoxazole, thiazole, isothiazole, 1,3,4-oxadiazole, 1,3,4-thiadiazole, pyridine, pyridazine, pyrimidine or pyrazine ring; $R^2$ is a methyl, ethyl or propyl group; $R^3$ and $R^4$ are methyl groups; $R^5$ is a methyl, ethyl or methoxy group; and $R^6$ and $R^7$ are hydrogen atoms.

The substituted allylamine derivative of formula [I] may exist in the form of an acid addition salt. Examples of such an acid addition salt include inorganic acid salts such as hydrochlorides, hydrobromides, hydroiodides, sulfates, nitrates, perchlorides and phosphates; and organic acid salts such as p-toluenesulfonates, benzenesulfonates, methanesulfonates,

oxalates, succinates, tartrates, citrates, fumarates and maleates. Those which are a pharmaceutically acceptable nontoxic salts are preferred. The compounds of formula [I] provided by this invention may include stereoisomers such as geometric isomers and optical isomers. The present invention embraces all these stereoisomers and mixtures thereof.

General processes for the production of the compounds of this invention will be described.

The compounds of this invention represented by formula [I] can be produced by any of the following processes, A, B, C, D, E and F.

[Process A]

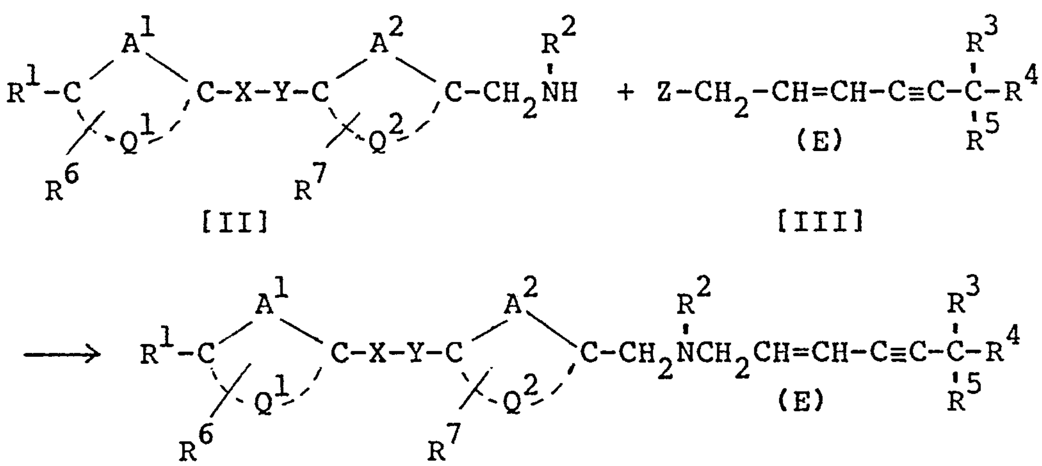

[II] [III]

[I]

[Process B]

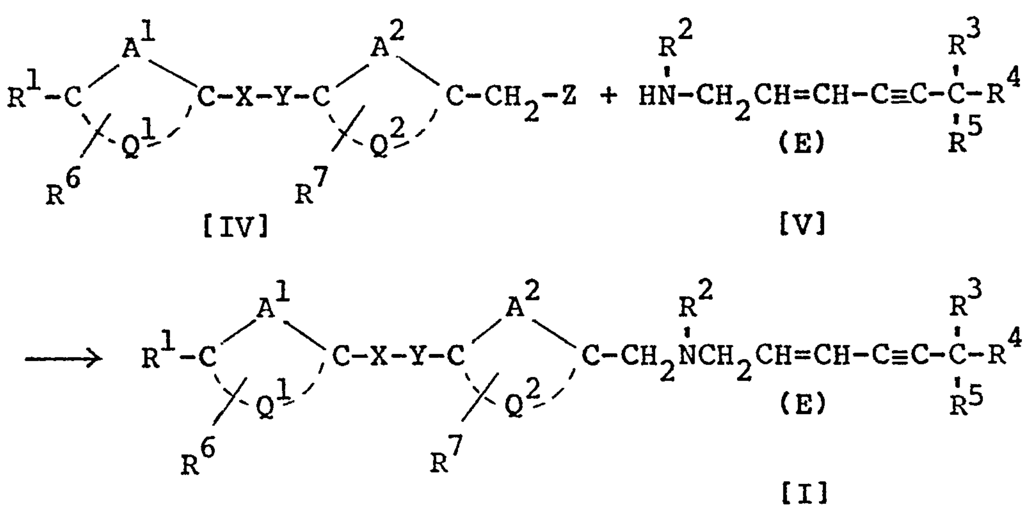

[IV] [V]

[I]

[Process C]

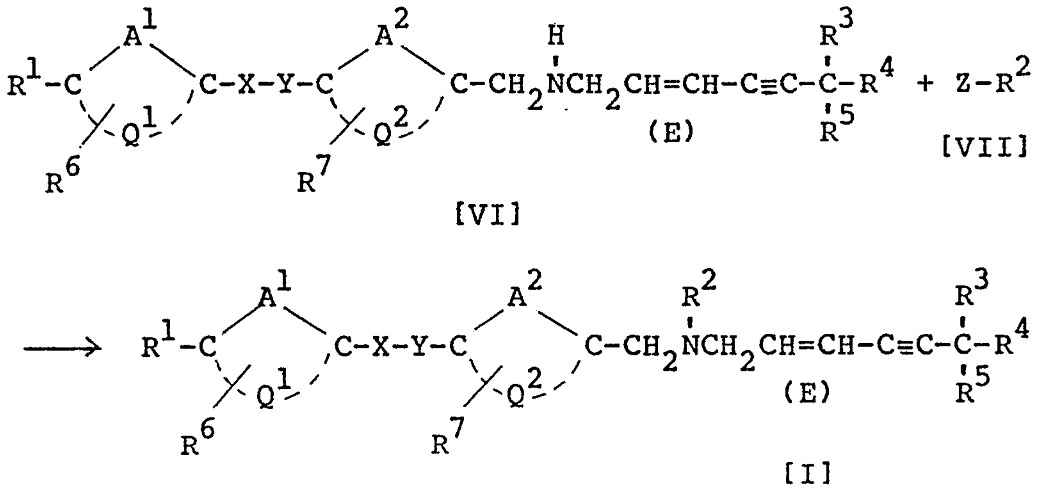

[VI] [VII]

[I]

[Process D]

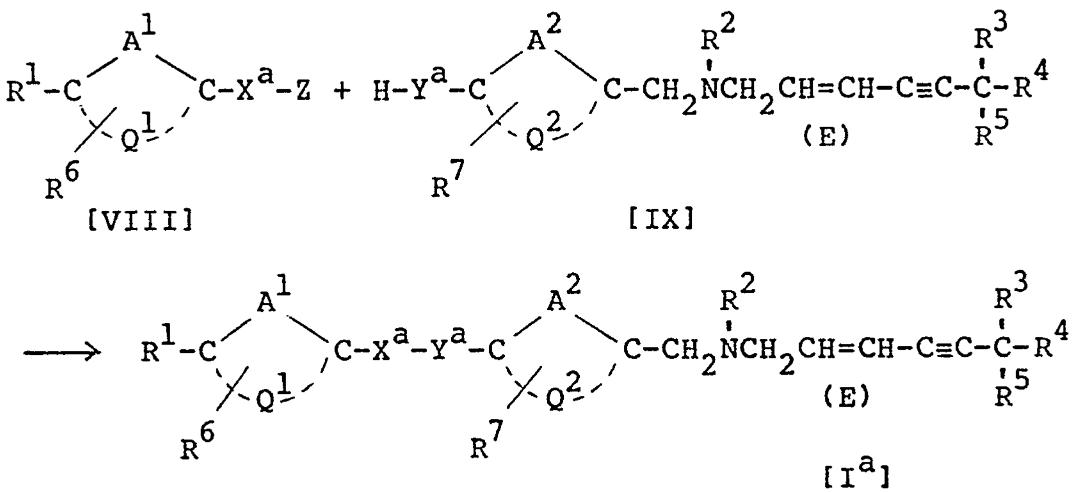

[VIII] [IX]

[$I^a$]

[Process E]

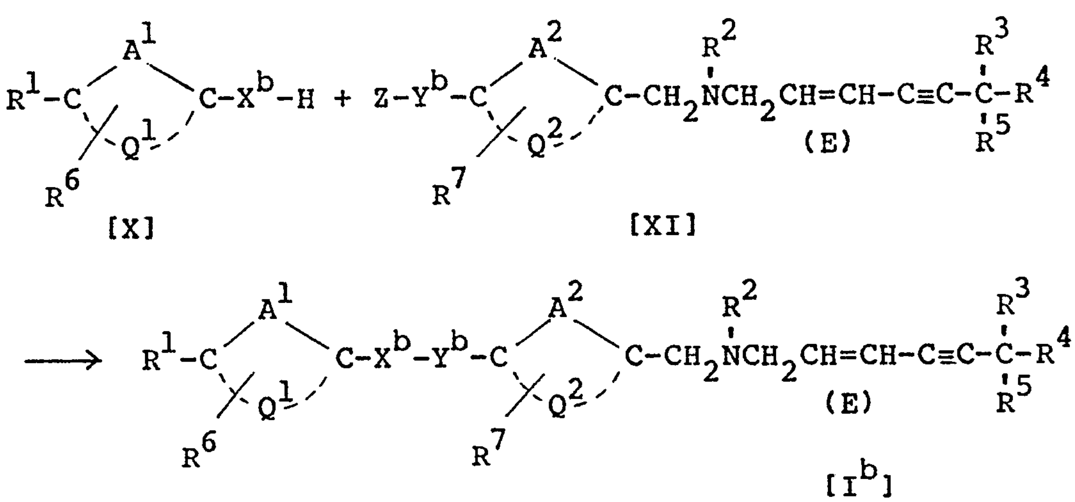

[X] [XI]

[$I^b$]

[Process F]

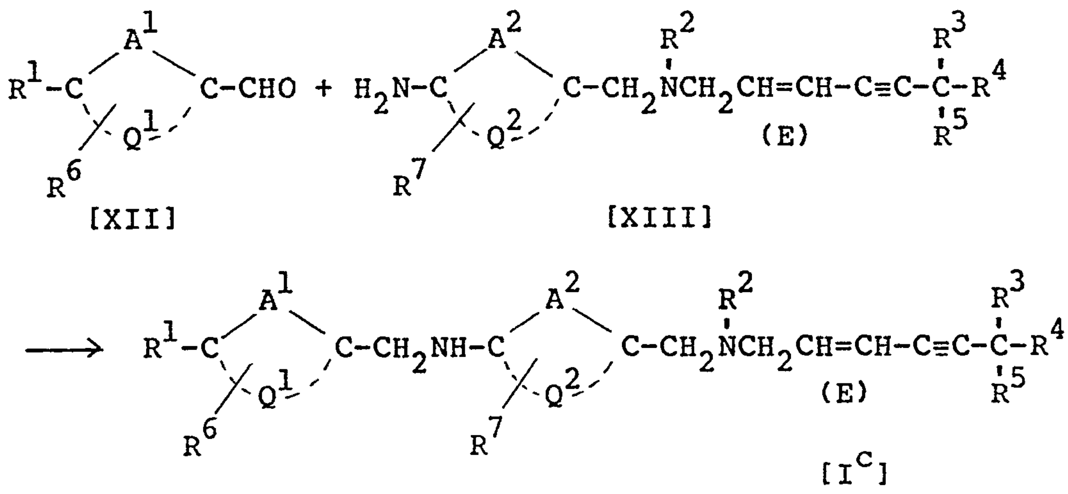

[XII] [XIII]

$[I^c]$

In the formulae, Z represents a leaving group; $X^a$ and $Y^b$ represent a carbonyl group or a group of the formula $-CHR^a-$ (where $R^a$ is as defined above); $X^b$ and $Y^a$ represent an oxygen atom, a sulfur atom or a group of the formula $-NR^b-$ (where $R^b$ is as defined above); and $A^1$, $A^2$, $Q^1$, $Q^2$, $R^1$, $R^2$, $R^3$, $R^4$, $R^5$, $R^6$ and $R^7$ are as defined above.

The processes A, B and C are alkylation of amines well known in the field of organic synthetic chemistry, and therefore can be carried out by using ordinary means known _per se_. The reactions in these processes may be carried out by using solvents which do not adversely affect the reactions. Examples of such solvents include aromatic hydrocarbons such as benzene, toluene and xylene; ethers such as ethyl ether, tetrahydrofuran and dioxane; halogenated hydrocarbons such as methylene chloride, chloroform and dichloroethane; alcohols such as ethanol and isopropanol; dimethylformamide, acetonitrile and dimethyl sulfoxide; and mixtures of these. Usually, in process A, compounds [II] and [III] are reacted in nearly equimolar proportions. In process B, compounds [IV] and [V] are reacted in nearly equimolar proportions. In process C, compounds [VI] and [VII] are reacted in nearly equimolar proportions.

Alternatively, one of the reactants may be in slight excess. The reaction conditions that can be used at this time are a reaction temperature of generally -20 to 150 °C, preferably from room temperature to the boiling point of the solvent, and a reaction time of usually 5 minutes to 10 days, preferably 1 to 24 hours. The above reactions are advantageously carried out in the presence of a base in order to permit the reactions to proceed smoothly. Examples of the base used at this time are alkali metal hydrides such as sodium hydride, lithium hydride and potassium hydride, alkali metal or alkaline earth metal hydroxides such as sodium hydroxide, potassium hydroxide and calcium hydroxide, alkali metal carbontates such as potassium carbonate and sodium hydrogen carbonate, and organic amines such as triethylamine and pyridine. The amount of the base is not critical, and can be varied over a wide range. Generally, it is at least 1 mole, preferably 1 to 2 moles, per mole of the starting compound.

Processes D and E are for the synthesis of compounds [$I^a$] or [$I^b$] corresponding to compounds of general formula [I] in which the group -X-Y- is -COO-, -OCO-, -CONR$^b$-, -NR$^b$CO-, -CHR$^a$O-, -OCHR$^a$-, -CHR$^a$S-, or -SCHR$^a$- (wherein R$^a$ and R$^b$ are as defined). Processes D and E are carried out in a solvent which does not adversely affect the reaction, such as tetrahydrofuran, dioxane, chloroform, benzene, acetone, dimethylformamide or dimethyl sulfoxide by reacting compounds [VIII] and [IX] in the case of process D or compounds [X] and [XI] in the case of process E in nearly equimolar proportions or one of the reactants being used in slight excess. The reaction conditions such as the reaction temperature and time used at this time differ depending upon the materials used. Generally, the reaction temperature is -70 to 100 °C, preferably -20 to 50 °C, and the reaction time is 1 minute to 24 hours, preferably 30 minutes to 5

hours. This reaction is preferably carried out in the presence of a base in order to allow the reaction to proceed smoothly. Examples of the base used at this time include inorganic bases such as sodium hydride, lithium hydride, sodium hydroxide, potassium hydroxide, sodium carbonate, potassium carbonate, sodium hydrogen carbonate and potassium hydrogen carbonate; and organic bases such as pyridine, triethylamine and dimethylaminopyridine. The amount of the base used is not strictly limited. Usually, it can be used in an amount of at least 1 mole, preferably 1 to 2 moles, per mole of the starting compound.

Process F is for the production of compounds of formula [$I^c$] corresponding to compounds of formula [I] in which the group -X-Y- is $-CH_2NH-$.

Process F can be carried out by condensing compound [XII] with compound [XIII] in benzene or alcohol to form an imine and thereafter, reducing the product. The reagent used in the reduction may be, for example, sodium borohydride, sodium cyanoborohydride or lithium aluminium hydride. The reaction is carried out, for example, in methanol, ethanol or tetrahydrofuran at 0 °C to room temperature for 1 to 6 hours. If the starting compounds of formulae [II], [III], [IV], [V], [VI], [VII], [VIII], [IX], [X], [XI], [XII] and [XIII] contain a reactive functional group such as a hydroxyl or amino group, in addition to the hydroxyl, mercapto or amino group which will be involved in the reaction, the reactive functional group, as required, can be protected, and after the reaction, the protecting group can be split off. The protecting group used at this time is one which can be easily eliminated by hydrolysis under acidic or alkaline conditions. Examples include methoxymethyl, tetrahydropyranyl, trityl, dimethyl-(tert-butyl)silyl, formyl, acetyl, methoxycarbonyl, ethoxycarbonyl and tert-butoxycarbonyl groups.

The desired compound of formula [I] so obtained may be isolated and purified by, for example, column chromatography, solvent extraction, precipitation, and recrystallization taken either alone or in combination. As required, the compound [I] as a free base may be converted into its acid addition salt, or the acid addition salt may be converted into the free base. The step of converting the free base into its acid addition salt, and the step of converting the acid addition salt into the free base may be easily carried out by ordinary methods using the corresponding acids or bases.

The leaving group represented by Z may be, for example, a halogen atom such as a chlorine, bromine or iodine atom, or an organic sulfonyloxy group such as a methanesulfonyloxy group or a p-toluenesulfonyloxy group.

The starting compounds of formulae [II] to [XIII] are commercially available or can be produced by the methods described in the literature [J. Med. Chem., vol. 27, page 1539 (1984), ibid., vol. 29, page 112 (1986), Japanese Laid-Open Patent Publications 32440/1981, 123177/1982, 208252/1983, 45/1986, 201850/1987 and 5059/1988], and the general methods shown below or methods substantially in accordance with them.

The starting compounds used in this invention can be produced, for example, by the following synthetic methods.

Production route (a)

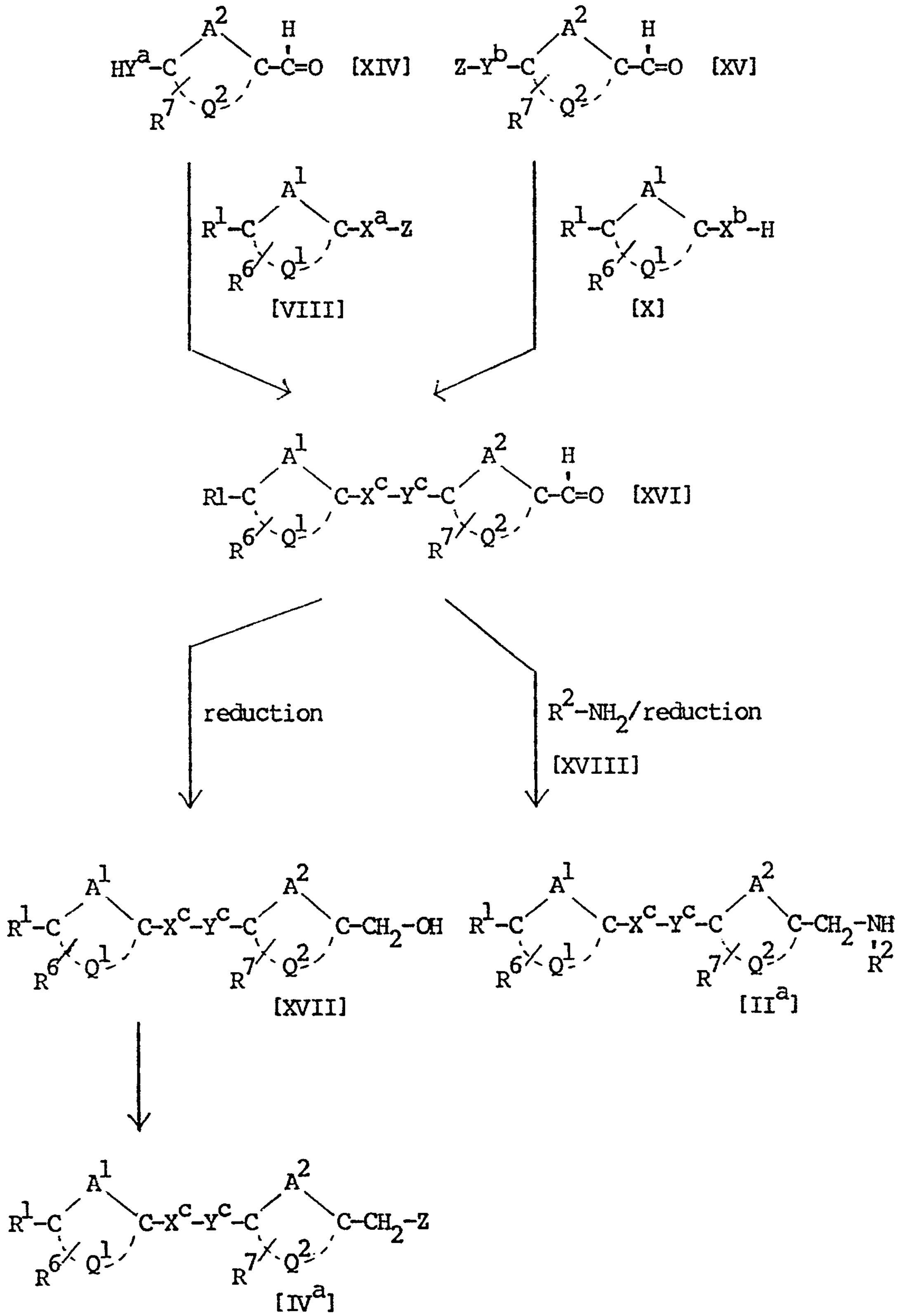


[XIV] [XV] [VIII] [X] [XVI] [XVII] [$II^a$] [$IV^a$]

Production route (b)

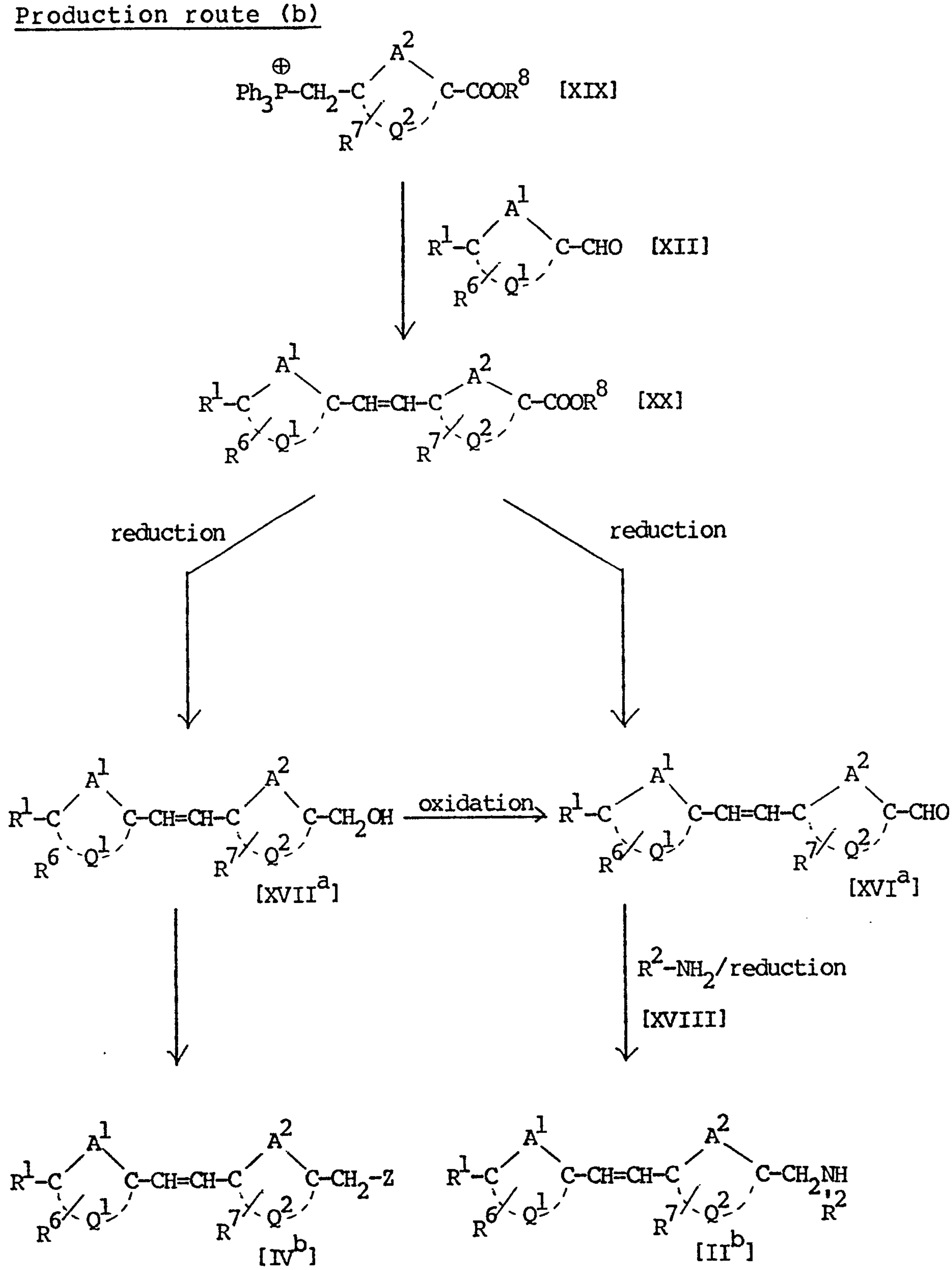


[XIX]

[XII]

[XX]

[XVII$^a$]

[XVI$^a$]

[IV$^b$]

[II$^b$]

Production route (c)

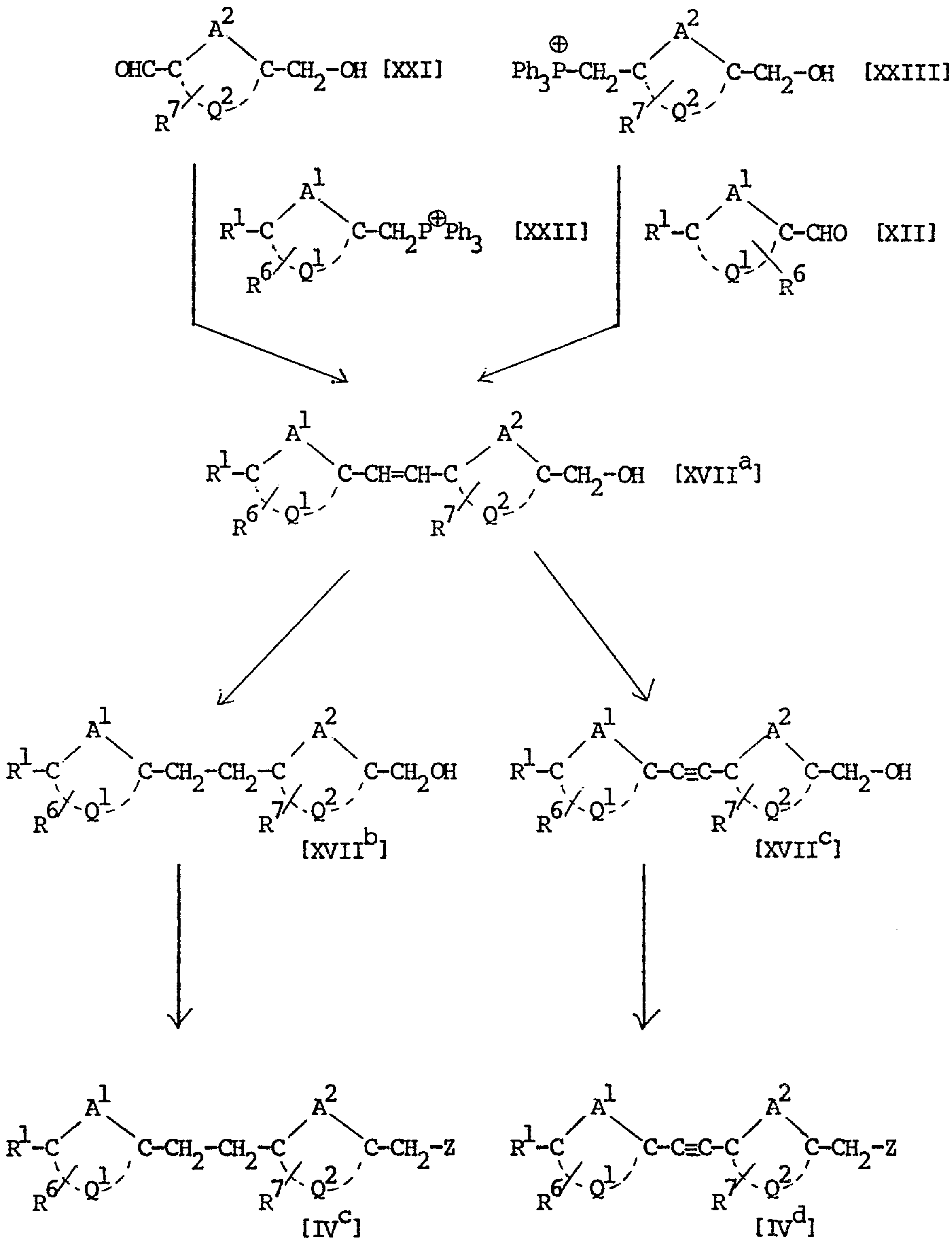

[XXI] [XXIII] [XXII] [XII] [XVII$^a$] [XVII$^b$] [XVII$^c$] [IV$^c$] [IV$^d$]

Production route (d)

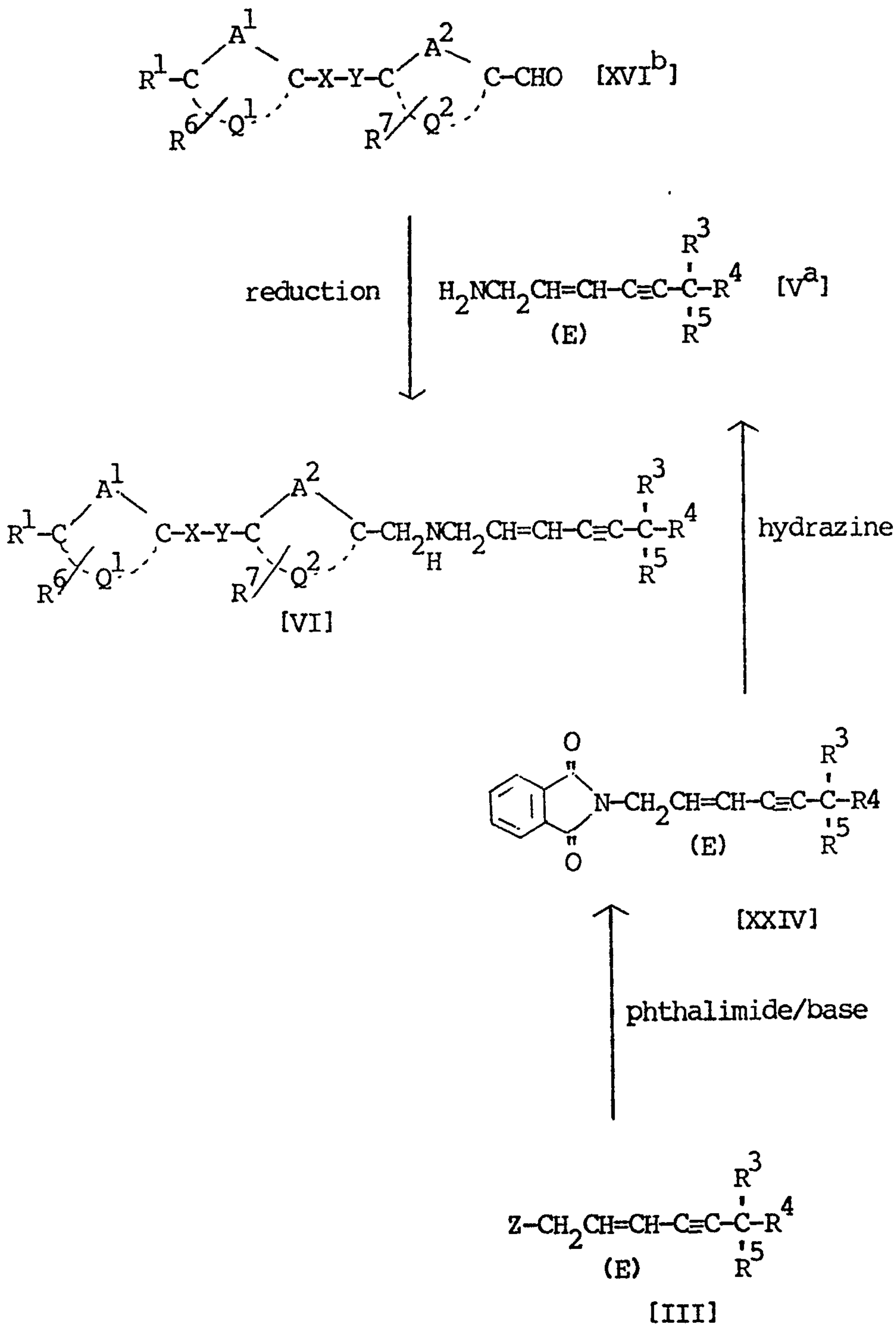


[XVI^b] [V^a] [VI] [XXIV] [III]

Production route (e)

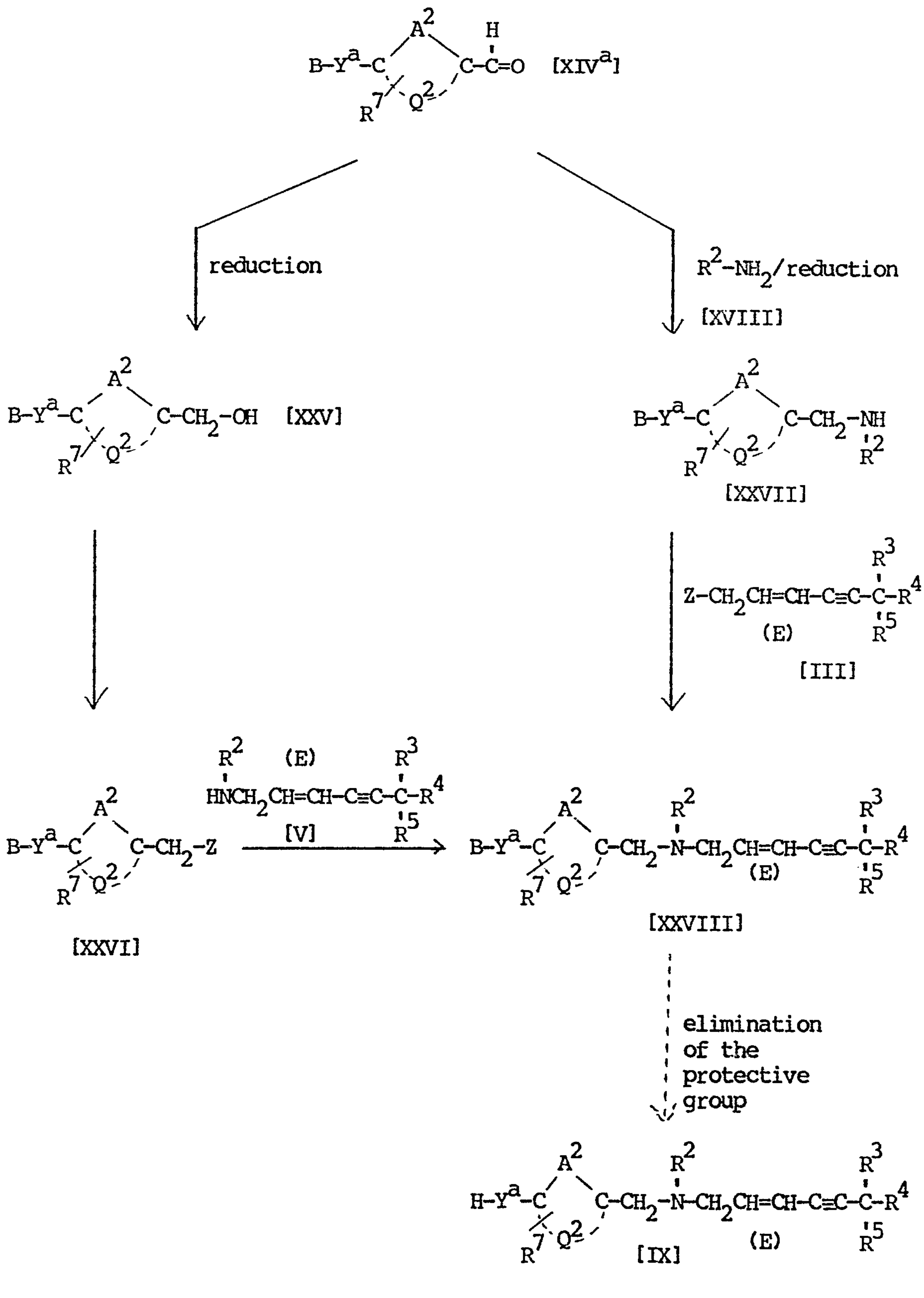

Production route (f)

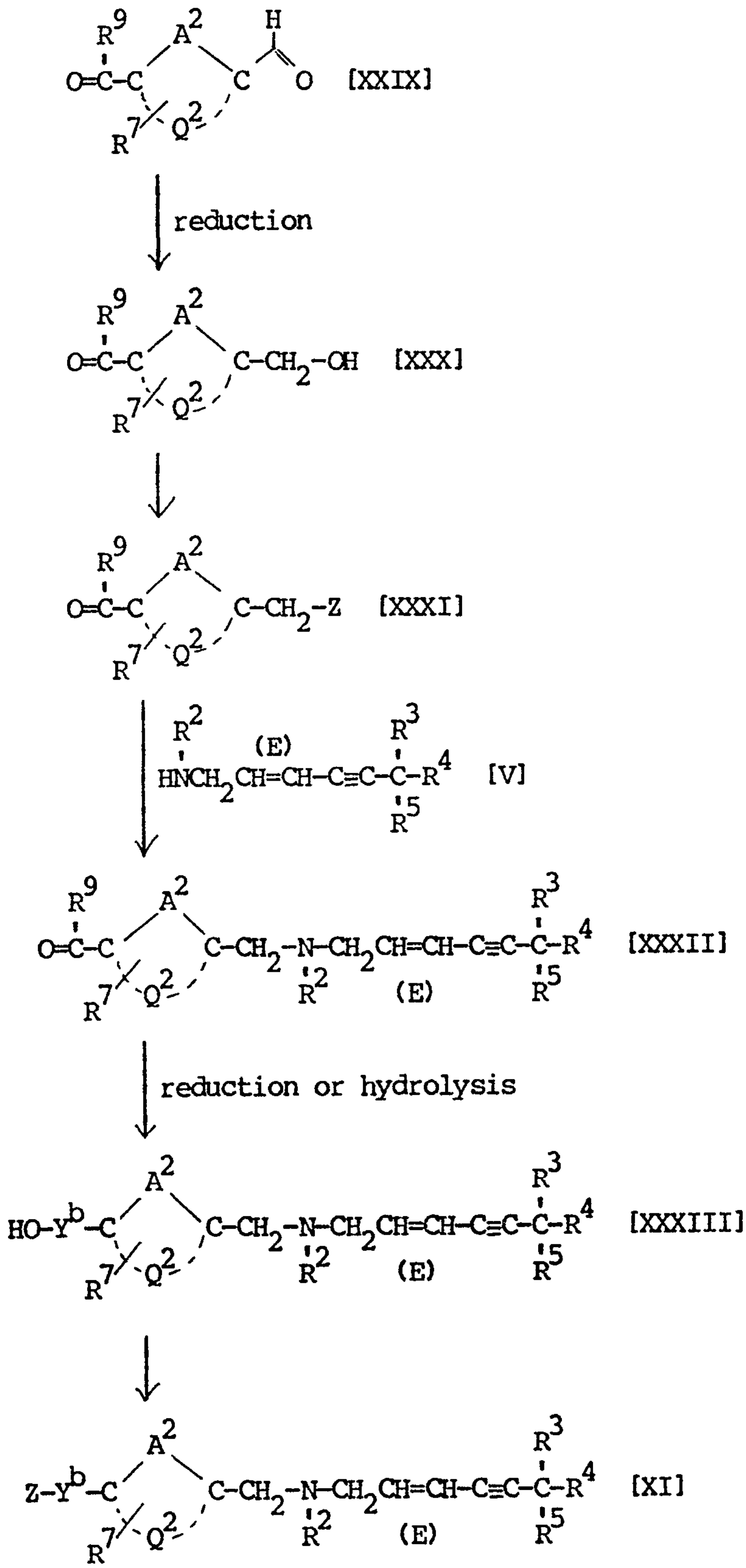

[In the formulae, $X^c$ and $Y^c$ are identical or different, and each represents an oxygen atom, a sulfur atom, a carbonyl group, a group of the formula $-CHR^a-$ (where $R^a$ is as defined above), or a group of the formula $-NR^b-$ (where $R^b$ is as defined above); $R^8$ represents a lower alkyl group; $R^9$ represents a hydrogen atom, a lower alkyl group or a lower alkoxy group; B represents a hydrogen atom or a protective group; and $A^1$, $A^2$, $Q^1$, $Q^2$, X, Y, $X^a$, $X^b$, $Y^a$, $Y^b$, Z, $R^1$, $R^2$, $R^3$, $R^4$, $R^5$, $R^6$ and $R^7$ are as defined hereinabove; with the proviso that when one of $X^c$ and $Y^c$ represents an oxygen atom, a sulfur atom or a group of the formula $-NR^b-$ (where $R^b$ is as defined), the other represents a carbonyl group or a group of the formula $-CHR^a-$ (where $R^a$ is as defined above).]

Production route (a)

The step of reacting compound [XIV] with compound [VIII] to produce compound [XVI] is carried out by reacting compounds [XIV] and [VIII] in nearly equimolar proportions at -20 to 100 °C for 2 to 3 hours in a solvent such as tetrahydrofuran, chloroform or dimethylformamide in the presence of a base such as sodium hydride, sodium hydroxide or potassium carbonate.

The step of producing compound [XVI] by reacting compounds [XV] and [X] can be carried out in the same manner as the step of reacting compounds [XIV] and [VIII].

The step of producing compound [$II^a$] by reacting compounds [XVI] and [XVIII] can be carried out, for example, by condensing compounds [XVI] and [XVIII] to form imine in benzene or alcohol and then reducing the product, or by reacting an excess of compound [XVIII] with compound [XVI] and simultaneously performing reduction. The reagent that is used in the reduction at this time may be, for example, sodium borohydride, sodium cyanoborohydride or lithium aluminium hydride. The reaction is carried out, for example, in methanol, ethanol

or tetrahydrofuran at 0 °C to room temperature for 1 to 6 hours.

The step of producing compound [XVII] by reducing compound [XVI] is carried out, for example, by treating compound [XVI] with a reducing agent such as sodium borohydride, sodium cyanoborohydride or lithium aluminium hydride in a solvent such as methanol, ethanol or tetrahydrofuran at 0 °C to room temperature for 1 to 5 hours.

The step of converting compound [XVII] into compound [$IV^a$] may be carried out, for example, by treating compound [XVII] with a sulfonylating agent such as a methanesulfonyl chloride and triethylamine or a halogenating agent such as thionyl chloride or phosphorus tribromide in a solvent such as chloroform or methylene chloride or in the absence of solvent at a temperature of -20 °C to room temperature for 1 to 5 hours.

Production route (b)

The step of producing compound [XX] by reacting compounds [XIX] and [XII] may be carried out, for example, by treating the compounds with a base such as n-butyllithium or sodium hydride in a solvent such as tetrahydrofuran at 0 °C to room temperature for 1 to 6 hours.

The step of producing compound [$XVII^a$] by reducing compound [XX] may be carried out by treating compound [XX] with a reducing agent such as lithium aluminium hydride in a solvent such as ethyl ether or tetrahydrofuran for 1 to 5 hours.

The step of producing compound [$XVI^a$] by reducing compound [XX] or oxidizing compound [$XVII^a$] may be carried out, for example, by reducing compound [XX] with diisobutyl aluminium hydride in toluene, or by oxidizing compound [$XVII^a$] with pyridinium chlorochromate in methylene chloride.

The step of converting compound [$XVII^a$] into compound [$IV^b$] and the step of producing compound [$II^b$]

by reacting compounds [XVI$^a$] and [XVIII] can be carried out in the same way as the step of converting compound [XVII] into compound [IV$^a$] and the step of producing compound [II$^a$] by reacting compound [XVI] with compound [XVIII] in production route (a).

Production route (c)

The step of producing compound [XVII$^a$] by reacting compound [XXI] with compound [XXII], or compound [XXIII] with compound [XII] can be carried out in the same way as the step of producing compound [XX] by reacting compound [XIX] with compound [XII] in production route (b).

The step of reducing compound [XVII$^a$] to compound [XVII$^b$] can be carried out, for example, by performing catalytic reduction in a solvent such as methanol or ethanol in the presence of a catalyst such as palladium-carbon at room temperature and atmospheric pressure for 1 to 10 hours.

The step of converting compound [XVII$^a$] into compound [XVII$^c$] may be carried out by reacting compound [XVII$^a$], either as such or after protecting it with a suitable protective group, with bromine in an organic solvent such as methylene chloride, chloroform or ethyl ether at 0 to 60 $^oC$ for 0.5 to 3 hours, concentrating the resulting solution to dryness, and thereafter, treating the residue in the presence of a base such as sodium hydroxide or potassium hydroxide in an alcohol solution such as methanol, ethanol or isopropanol at the boiling point of the solvent for 1 to 10 hours.

The step of converting compound [XVII$^b$] into compound [IV$^c$] and the step of converting compound [XVII$^c$] into compound [IV$^d$] may be carried out as in the step of converting compound [XVII] into compound [IV$^a$].

Production route (d)

The step of producing compound [VI] by reacting compound [XVI$^b$] with compound [V$^a$] may be carried out in

the same way as the step of producing compound [$II^a$] by reacting compound [XVI] with compound [XVIII] in production route (a).

The compound [$V^a$] used at this time may be produced, for example, by the Gabriel method which comprises reacting compound [III] with phthalimide in the presence of a base such as sodium hydroxide or potassium carbonate in a solvent such as tetrahydrofuran or dimethylformamide at 10 to 100 $^oC$ to produce compound [XXIV], and thereafter reacting it with hydrazine in, for example, ethanol or dimethylformamide, thereby forming compound [$V^a$].

Production route (e)

The step of producing compound [XXVII] by reacting compound [$XIV^a$] with compound [XVIII] may be carried out in the same way as in the step of producing compound [$II^a$] by reacting compound [XVI] with compound [XVIII]. The step of reducing compound [$XIV^a$] to produce compound [XXV] and then converting it into compound [XXVI] may be carried out in the same way as the step of reducing compound [XVI] to compound [XVII] and converting it into compound [$IV^a$] in production route (a).

The step of producing compound [IX] by reacting compound [XXVI] with compound [V] or reacting compound [XXVII] with compound [III] to produce compound [XXVIII], and thereafter, as required, deprotecting the product may be carried out in the same way as process A or B described above.

The protective group represented by B in the starting compound used in production route (e) may be any of various protective groups used in the field of organic synthetic chemistry as protective groups for a hydroxyl, mercapto or amino group. Specific examples include methoxymethyl, tetrahydropyranyl, trityl, tert-butoxycarbonyl and dimethyl(tert-butyl)silyl groups.

<u>Production route (f)</u>

The step of producing compound [XXX] by reducing compound [XXIX] is partial reduction which utilizes the difference in reactivity between coexisting carbonyl groups in reduction. It can be carried out, for example, by reducing compound [XXIX] with 1 to 2 equivalents of a reducing agent such as sodium borohydride or diisobutylaluminium hydride at -20 °C to room temperature for 1 to 5 hours in a solvent such as ethanol or tetrahydrofuran, or carrying out catalytic reduction in the presence of a catalyst such as palladium-carbon for 1 to 5 hours. The step of producing compound [XXXII] by converting compound [XXX] into compound [XXXI] and then reacting it with compound [V] may be carried out in the same way as the step of producing compound [XXVIII] by converting compound [XXV] into compound [XXVI] and then reacting it with compound [V] in production route (e).

The step of producing compound [XXXIII] by converting compound [XXXII] may be carried out by reduction in the case of an alcohol product [XXXIII] in which $Y^b$ is a group of the formula $-CHR^a-$ in which $R^a$ is as defined above, and by hydrolysis in the case of a carboxylic acid product [XXXIII] in which $Y^b$ is a carbonyl group. The step of producing the alcohol product [XXXIII] by reducing compound [XXXII] may be carried out in the same way as the step of producing compound [XVII] by reducing compound [XVI] in production route (a), or the step of producing compound [$XVII^a$] by reducing compound [XX] in production route (b). The step of hydrolyzing compound [XXXII] to produce the corresponding carboxylic acid compound [XXXIII] may be carried out, for example, by dissolving compound [XXXII] in a solvent such as aqueous ethanol or aqueous tetrahydrofuran containing an equimolar or an excessive molar of a base such as sodium hydroxide, and maintaining the solution at room temperature to 100 °C for 1 to 10 hours.

The step of converting compound [XXXIII] to produce compound [XI] may be carried out by treating compound [XXXIII] with a halogenating agent such as thionyl chloride or phosphorus tribromide in a solvent such as chloroform or methylene chloride or in the absence of solvent at a temperature of -20 °C to room temperature for 1 to 5 hours.

The products obtained in each of the steps described above can, if required, be purified or isolated by, for example, chromatography, recrystallization, solvent extraction, precipitation and distillation either alone or in suitable combinations.

Compounds which can be starting materials of the intermediates may be easily purchased as commercial goods, or produced easily by the methods described in the literature [for example, J. Med. Chem., vol. 27, page 1539 (1984); ibid., vol. 29, page 112 (1986); and Japanese Laid-Open Patent Publications Nos. 32440/1981, 123177/1982, 208252/1983, 45/1986, 201850/1987, and 5059/1988] and the synthesizing methods known in organic sythetic chemistry.

The compounds of this invention represented by general formula [I] are useful compounds which inhibit the mammalian squalene-epoxidase very selectively and strongly, and are expected to be used as a hypolipemic agent or an antiarteriosclerotic agent.

To demonstrate this, pharmacological test examples and an acute toxicity test examples will be given below.

PHARMACOLOGICAL TEST EXAMPLE 1

Squalene epoxidase inhibiting activity

(1) Preparation of squalene epoxidase

The rat squalene epoxidase was prepared by the method described in J. Biol. Chem., vol. 245, page 1670 (1970); ibid., vol. 250, page 1572 (1975).

SD-strain female rats were killed by exsangui-

nation. Livers were extracted and homogenized in the presence of 2 volumes of 0.1M Tris-HCl buffer (pH 7.5). The homogenate was centrifuged at 9750 Xg for 10 minutes. The supernatant fraction was further centrifuged at 105000 Xg for 1 hour. The sediment was washed with 0.1M Tris-HCl buffer (pH 7.5), and then centrifuged at 105000 Xg for 1 hour. The microsomes obtained were suspended in 0.1M Tris-HCl buffer (pH 7.5) so that the amount of proteins was 40 mg/ml, and under ice cooling, the suspension was stirred in the presence of 2 % Triton X-100 to solubilize the enzyme. After the solubilization, the solution was diluted to a Triton X-100 concentration of 0.5 % with 1 mM EDTA and 1 mM dithiothreitol, and centrifuged at 105000 Xg for 1 hour. The resulting supernatant fraction was used in the following test as a squalene epoxidase fraction.

(2) Method of assaying the squalene epoxidase activity

The squalene epoxidase activity was assayed in accordance with the method described in J. Biol. Chem., vol. 245, page 1670 (1970).

Three microliters of a dimethyl sulfoxide solution of a test drug was added to a solution composed of 0.2 ml of the squalene epoxidase fraction prepared in (1) [proteins 0.4 mg, 0.5 % Triton X-100, 20 $\mu$M Tris-HCl buffer (pH 7.5)], 100 $\mu$M FAD, 1 mM NADPH, 1 mM EDTA and 8 $\mu$M $^3$H-squalene-Tween 80 emulsion to adjust the total amount of the solution to 0.3 ml. The solution was incubated at 37 $^{o}$C for 30 minutes with shaking. Then, 0.3 ml of a 10 % methanolic potassium hydroxide was added to stop the reaction, and the reaction mixture was left to stand at room temperature for 1 hour. The non-saponified material was extracted with petroleum ether, and the solvent was evaporated to dryness under a nitrogen stream. The resulting residue was dissolved in a small amount of ethyl ether and spotted on pre-coated silica gel TLC plate, followed by developing with

benzene/ethyl acetate (99.5:0.5). The position of the resulting $^3H$-squalene-2,3-epoxide on TLC was determined using ergosterol acetate as a marker, and the $^3H$-squalene-2,3-epoxide portion on the TLC was cut off. The TLC strip was immersed in a toluene-type scintillator, and measured by a liquid scintillation counter. As a result, the 50 % inhibitory concentrations ($IC_{50}$ values) of the compounds of this invention on squalene epoxidase were determined. The results are shown in Table 1.

Table 1

Squalene epoxidase inhibitory activity

| Test drug | 50 % inhibitory concentration ($IC_{50}$, nM) |
|---|---|
| Compound of Example 2 | 7 |
| Compound of Example 3 | 4 |
| Compound of Example 8 | 11 |
| Compound of Example 9 | 6 |
| Compound of Example 15 | 60 |
| Compound of Example 19 | 27 |
| Compound of Example 25 | 33 |
| Compound of Example 26 | 31 |
| Compound of Example 31 | 26 |
| Compound of Example 33 | 26 |
| Compound of Example 47 | 26 |
| Compound of Example 48 | 23 |
| Compound of Example 51 | 34 |
| Compound of Example 54 | 21 |
| Compound of Example 56 | 34 |
| Compound of Example 70 | 11 |
| Compound of Example 74 | 22 |
| Compound of Example 78 | 33 |

PHARMACOLOGICAL TEST EXAMPLE 2

Inhibitory activity on cholesterol biosynthesis in cultured cells:-

Human hepatoma (Hep-G2) cells were cultured in 10 $cm^2$ dishes until they formed a monolayer. One milliliter of the culture medium was replaced, and 1 $\mu$Ci of [$^{14}$C] sodium acetate and 1 microliter of a dimethyl sulfoxide solution of a test drug were added, and the cells were cultured at 37 °C in air containing 5 % of carbon dioxide for 6 hours.

After the cultivation, the medium was aspirated, and the cells were cooled with ice, and washed with Dulbecco's phosphate buffered saline solution. The resulting cells were scraped by a rubber policeman, and collected by centrifugation. The cells collected were dissolved in 400 microliters of 0.3N sodium hydroxide. A 200 microliter aliquot of the solution was used for extraction, and the remainder, for protein determination.

To 200 microliters of the extracted cells, 15 % ethanolic potassium hydroxide was added, and saponification was carried out at 75 °C for 1 hour. Water (1 ml) was then added, and the mixture was extracted with 2 ml of petroleum ether twice to remove non-saponified materials. The petroleum ether extracts were washed with 1 ml of water, and evaporated to dryness under a nitrogen stream. The residue was spotted on a pre-coated silica gel TLC plate using a small amount of chloroform, and developed with hexane/ethyl ether/acetic acid (85:15:4). Cholesterol and squalene portions on the TLC were detected with iodine, and the corresponding TLC portions were cut out. The TLC strips were immersed in a toluene-type scintillator, and radioactivity was counted by a liquid scintillation counter. The results were corrected by the amount of proteins measured by the method described in J. Biol. Chem., vol. 193, page 265 (1951). The 50 % inhibitory concentration ($IC_{50}$ value)

of the compound of the invention on cholesterol biosynthesis in the cultured Hep-G2 cells was calculated. The results are shown in Table 2.

Table 2

Cholesterol biosynthesis inhibitory activity in cultured cells

| Test drug | 50 % inhibitory concentration ($IC_{50}$, nM) |
|---|---|
| Compound of Example 2 | 53 |
| Compound of Example 3 | 11 |
| Compound of Example 8 | 12 |
| Compound of Example 9 | 11 |
| Compound of Example 15 | 54 |
| Compound of Example 19 | 9 |
| Compound of Example 25 | 6 |
| Compound of Example 26 | 5 |
| Compound of Example 31 | 22 |
| Compound of Example 33 | 25 |
| Compound of Example 42 | 30 |
| Compound of Example 47 | 10 |
| Compound of Example 48 | 10 |
| Compound of Example 51 | 33 |
| Compound of Example 74 | 29 |
| Compound of Example 78 | 17 |
| Compound of Example 80 | 52 |

PHARMACOLOGICAL TEST EXAMPLE 3

Test on inhibition of cholesterol biosynthesis *in vivo*:-

Female SD rats, 5 weeks of age, were used in the *in vivo* test. The rats were kept for 9 days in an environment of reversed light cycle (i.e., dark between 6:00 am and 6:00 pm). The rats were allowed to take a solid diet and water freely. The test drug was orally

administered two hours before dark sixth hour when the cholesterol synthesis reached a maximum. Test drugs were dissolved in water containing 5 % of dimethyl sulfoxide and 2 % Tween 80 and administered orally at the dose of 3 mg/kg (1 ml/100 g of body weight).

An equal volume of the vehicle used above was administered to a control group. One hour after administration of the test drug, [$^{14}$C] sodium acetate (56 mCi/mmole) was intraperitoneally administered to the rats in a dose of 20 $\mu$Ci/100 g of body weight. At dark sixth hour, a blood sample was obtained from the abdominal artery under ether anesthesia, and the plasma was separated by centrifugation.

One milliliter of plasma was mixed with 2 ml of a 15 % methanolic potassium hydroxide and saponified by heating at 75 $^{o}$C for 3 hours. The resulting sample was extracted with 2 ml of petroleum ether twice. The extracts were washed with 2 ml of distilled water, and finally evaporated under a nitrogen stream. The resulting residue was dissolved in a small amount of ethyl ether, and all the solution was spotted on a pre-coated silica gel TLC plate. The plate was developed with a solvent system composed of hexane/ethyl ether/acetic acid (85:15:4). Color formation was carried out by iodine, and the radioactivity of the cholesterol portion was measured by a liquid scintillation counter.

The results were expressed in dpm of the resulting $^{14}$C-cholesterol present in 1 ml of the plasma. The inhibition of cholesterol biosynthesis was calculated by comparing the amounts of $^{14}$C-cholesterol biosynthesized in the test group and that in the control group. The results are shown in Table 3.

Table 3

Cholesterol biosynthesis inhibiting test in rats (n=5)

| Test drug | $^{14}$C-cholesterol, dpm (control group) | Inhibition of cholesterol biosynthesis |
|---|---|---|
| Compound of Example 2 | 471 (2196) | 79 % |
| Compound of Example 3 | 530 (2456) | 78 % |
| Compound of Example 10 | 402 (1456) | 72 % |
| Compound of Example 11 | 503 (2196) | 77 % |
| Compound of Example 12 | 340 (2098) | 84 % |
| Compound of Example 31 | 650 (2454) | 74 % |
| Compound of Example 51 | 83 (2104) | 96 % |
| Compound of Example 52 | 125 (2454) | 95 % |
| Compound of Example 54 | 94 (2394) | 96 % |
| Compound of Example 55 | 355 (2649) | 87 % |
| Compound of Example 56 | 316 (2394) | 87 % |
| Compound of Example 59 | 194 (1456) | 87 % |
| Compound of Example 67 | 115 (2394) | 95 % |

ACUTE TOXICITY TEST EXAMPLE

Each of test drugs (compounds 3, 9, 19, 20, 25, 51; free base) was dissolved in middle chain triglyceride (MCT), and orally administered to mice (ddy, male, body weight 28±2 g, two per group). The acute toxicity was evaluated from the mortality determined one week after administration.

The test drug had very low toxicity, and even in a high dose of 1000 mg/kg, no case of death was observed.

As can be seen from the results of the foregoing tests, the compounds of this invention strongly inhibit squalene epoxidase and thus the biosynthesis of cholesterol. Accordingly, they are effective for the treatment and prevention of various diseases induced by the increase of the biosynthesis of cholesterol, for example obesity, hyperlipemia and arteriosclerosis. Furthermore, the squalene epoxidase inhibiting activity of the compounds of this invention is not observed on fungi, and is specific for mammals. The compounds of this invention also have low toxicity. Accordingly, they are very useful as pharmaceuticals.

The compound of formula [I] provided by this invention is formulated into a form suitable for oral or parenteral administration, and can be used in the treatment and remedy of hypercholesterolemia, hyperlipemia and arteriosclerosis. In using the compounds of the invention clinically, they can be formulated together with pharmaceutically acceptable adjuvants suitable for dosage forms, and then administered. Various adjuvants usually used in the pharmaceutical field can be used. Examples of the adjuvants include gelatin, lactose, sucrose, titanium dioxide, starch, crystalline cellulose, hydroxypropylmethyl cellulose, carboxymethyl cellulose, corn starch, microcrystalline wax, white Vaseline, magnesium metasilicate aluminate, anhydrous calcium phosphate,

citric acid, trisodium citrate, hydroxypropyl cellulose, sorbitol, sorbitan fatty acid esters, polysorbate, sucrose fatty acid esters, polyoxyethylene hydrogenated castor oil, polyvinylpyrrolidone, magnesium stearate, light anhydrous silicic acid, talc, vegetable oil, benzyl alcohol, gum arabic, propylene glycol, polyalkylene glycols, cyclodextrin or hydroxypropyl cyclodextrin.

Formulations prepared as mixtures with these adjuvants include, for example, solid preparations such as tablets, capsules, granules, powders and suppositories and liquid preparations such as syrups, elixirs and injectable preparations. These preparations can be formed by ordinary methods known in the field of pharmaceutical preparation. The liquid preparations may be in the form of a solution or suspension in water or another suitable medium. As required, the injectable preparations may be dissolved in physiological saline or glucose solution, or a buffer or a preservative may be added.

These formulations may contain 1.0 to 100 % by weight, preferably 1.0 to 60 % by weight, of the compound of this invention based on the total drugs. They may also contain other therapeutically effective compounds.

When the compound of this invention is used as a hypolipemic agent, an antiarteriosclerotic agent or an hypocholesterolemic agent, its dosage and the number of administration differ depending upon the sex, age, body weight and the severity of symptom of a patient and the type and range of the intended therapeutic effect. Generally, in oral administration, it is administered preferably in a dose of 0.01 to 20 mg/kg once or in several divided portions. In parenteral administration, it is preferably administered in a dose of 0.001 to 2 mg/kg once or in several divided portions.

The following Examples and Referential Examples illustrate the present invention more specifically. It

should be understood that the present invention is not limited to these examples alone.

EXAMPLE 1

Production of (E)-N-(6,6-dimethyl-2-hepten-4-ynyl)-N-methyl-3-[3-(2-furyl)benzyloxy]benzylamine hydrochloride:-

190 mg of (E)-N-(6,6-dimethyl-2-hepten-4-ynyl)-N-methyl-3-hydroxybenzylamine was dissolved in 3 ml of anhydrous tetrahydrofuran, and 31 mg of 60 % oily sodium hydride was added with ice cooling and stirring. The solution was stirred for 10 minutes. To the resulting solution were added an ether solution of 3-(2-furyl)-benzyl chloride (prepared in advance by reacting 160 mg of 3-(2-furyl)benzyl alcohol and 73 microliters of thionyl chloride in anhydrous ethyl ether with ice cooling and stirring for 3 hours, and thereafter washing the resulting solution with a saturated aqueous solution of sodium chloride and a 5 % aqueous solution of sodium hydrogen carbonate) and 1 ml of dimethylformamide. The mixture was stirred overnight at room temperature. 30 ml of water and 30 ml of ethyl ether were added to the reaction solution, and the mixture was separated. The organic layer was separated, washed with a saturated aqueous solution of sodium chloride, and dried over anhydrous sodium sulfate. The solvent was then evaporated. The residue was purified by medium-pressure liquid chromatography [column: Lobar column, size B, Lichroprep Si 60F (E. Merck Co.; eluting solvent: hexane/ethyl acetate=10/1 → 8/1] to give 63 mg (yield 21 %) of a free base of the captioned compound as a colorless oil. The free base was treated with a solution of methanol and hydrogen chloride and recrystallized from a mixture of ethyl acetate/ethyl ether to give the captioned hydrochloride, m.p. 115-116 $^{o}$C.

IR $\nu_{max}^{KBr}$ $cm^{-1}$: 2968, 2488, 1605, 1497, 1461, 1266, 789.

NMR ($CDCl_3$) δ: 1.25(9H, s), 2.60(3H, s), 3.50-3.64 (2H, m), 4.00-4.13(2H, m), 5.20(2H, s), 5.78-5.84(1H, m), 6.18-6.32(1H, m), 6.47 (1H, dd, J=3.5Hz, 2.0Hz), 6.70(1H, d, J=3.5Hz), 7.05-7.11(2H, m), 7.30-7.47(5H, m), 7.62(1H, d, J=7.1Hz), 7.78(1H, s).

EXAMPLE 2

Production of (E)-N-(6,6-dimethyl-2-hepten-4-ynyl)-N-ethyl-3-[3-(1-pyrrolyl)benzyloxy]benzylamine:-

100 mg of (E)-N-(6,6-dimethyl-2-hepten-4-ynyl)-N-ethyl-3-hydroxybenzylamine was dissolved in 1 ml of anhydrous tetrahydrofuran, and 20 mg of 60 % oily sodium hydride was added to the solution with ice cooling and stirring, and the mixture was stirred for 10 minutes. To the resulting solution was added 1 ml of a dimethylformamide solution of 100 mg of 3-(1-pyrrolyl)benzylmethanesulfonate, and the mixture was stirred overnight at room temperature. The solution was extracted by adding 20 ml of water and 30 ml of ethyl ether. The organic layer was separated, washed with a saturated aqueous solution of sodium chloride, and dried over anhydrous sodium sulfate. The solvent was evaporated. The residue was purified by silica gel column chromatography [Wakogel C-200, 20 g, eluting solvent: hexane/ethyl acetate=10/1 → 5/1] to give 110 mg (yield 70 %) of the captioned compound as a colorless oil.

IR $\nu_{max}^{neat}$ $cm^{-1}$: 2968, 1596, 1506, 1488, 1455, 1341, 1263, 1071, 786, 726.

NMR ($CDCl_3$) δ: 0.98(3H, t, J=7.0Hz), 1.19(9H, s), 2.45(2H, q, J=7.0Hz), 3.03(2H, dd, J=6.4Hz, 1.6Hz), 3.49(2H, s), 5.06(2H, s), 5.59(1H, dt, J=15.9Hz, 1.6Hz), 6.01(1H, dt, J=15.9Hz, 6.4Hz), 6.30(2H, t, J=2.1Hz), 6.80(1H, ddd, J=8.3Hz, 2.6Hz, 0.8Hz), 6.88(1H, d, J=7.6Hz), 6.96-6.98(1H, m), 7.06(2H, t, J=2.1Hz), 7.17 (1H, t, J=7.8Hz), 7.25-7.32(2H, m), 7.39(1H, t, J=7.8Hz), 7.44-7.45(1H, m).

EXAMPLE 3

Production of (E)-N-ethyl-N-(6-methoxy-6-methyl-2-hepten-4-ynyl)-3-[3-(3-thienyl)benzyloxy]-benzylamine hydrochloride:-

57.5 mg of (E)-N-ethyl-N-(6-methoxy-6-methyl-2-hepten-4-ynyl)-3-hydroxybenzylamine was dissolved in 0.5 ml of anhydrous tetrahydrofuran, and under a nitrogen atmosphere, 8.1 mg of 60 % oily sodium hydride was added. The mixture was stirred at room temperature for 10 minutes, and to this solution was added 1 ml of a dimethylformamide solution of 53.7 mg of 3-(3-thienyl)-benzylmethanesulfonate. The mixture was stirred overnight at room temperature, and the solvent was evaporated under reduced pressure. The residue was purified by medium-pressure liquid chromatography [column: Lobar column, size A, Lichroprep Si 60 (E. Merck Co.); eluting solvent: hexane/ethyl acetate=6/1 → 1/1]] to give 81.6 mg (yield 89 %) of a free base of the captioned compound as a colorless oil. The free base was treated with a solution of hydrogen chloride and methanol and recrystallized from a mixture of ethyl acetate and ethyl ether to give the captioned hydrochloride, m.p. 153-155 $^{o}C$.

IR$\nu^{KBr}_{max}$ $cm^{-1}$: 3340, 2986, 2932, 1605, 1455, 1266, 1173, 1071, 777.

NMR ($CDCl_3$) $\delta$: 1.41(3H, t, J=7.2Hz), 1.47(6H, s), 2.90-3.20(2H, m), 3.34(3H, s), 3.40-3.80 (2H, m), 4.08(2H, br.s), 5.22(2H, s), 5.84 (1H, d, J=15.9Hz), 6.36(1H, dt, J=15.9Hz, 7.9Hz), 7.00-7.20(2H, m), 7.33(1H, t, J=7.9Hz), 7.40-7.70(7H, m), 7.73(1H, s).

EXAMPLE 4

Production of (E)-N-(6,6-dimethyl-2-hepten-4-ynyl)-N-methyl-3-[3-(5-isoxazolyl)benzyloxy]benzylamine:-

106 mg of 5-(3-methylphenyl)isoxazole, 119 mg of N-bromosuccinimide and 2 mg of benzoyl peroxide were dissolved in 10 ml of carbon tetrachloride, and the

solution was refluxed for 3 hours with stirring. After cooling, the precipitate was separated by filtration, and concentrated under reduced pressure to give 5-(3-bromomethylphenyl)isoxazole as a pale yellow oily product.

The resulting bromomethyl compound was dissolved in 5 ml of dimethylformamide. The solution was added to 10 ml of a tetrahydrofuran solution of phenolate prepared in advance from 171 mg of (E)-N-(6,6-dimethyl-2-hepten-4-ynyl)-N-methyl-3-hydroxybenzylamine and 30 mg of 60 % oily sodium hydride, and under ice cooling, the mixture was stirred for 1 hour. Water and ethyl ether were added to the reaction solution to dilute it. The organic layer was separated and then dried over anhydrous magnesium sulfate. The desiccant was separated by filtration, and the solvent was evaporated. The residue was purified by medium-pressure liquid chromatography [column: Lobar column, size A, Lichroprep Si 60 (E. Merck Co.); eluting solvent: hexane/ethyl acetate=10/1 → 5/1] and preparative thin-layer chromatography [thin layer plate: Kieselgel $60F_{254}$, Art. 5744 (E. Merck Co.); developing solvent: hexane/ethyl acetate=3/1] to give 19 mg (yield 11 %) of the captioned compound as a colorless oil.

IR $\nu_{max}^{neat}$ $cm^{-1}$: 2974, 2788, 1584, 1491, 1470, 1266, 786.

NMR ($CDCl_3$) $\delta$ : 1.24(9H, s), 2.19(3H, s), 3.04(2H, dd, J=6.5Hz, 1.4Hz), 3.47(2H, s), 5.13(2H, s), 5.65(1H, dt, J=15.8Hz, 1.4Hz), 6.08(2H, dt, J=15.8Hz, 6.5Hz), 6.55(1H, d, J=1.9Hz), 6.88 (1H, ddd, J=8.1Hz, 2.6Hz, 0.9Hz), 6.70-6.75 (1H, m), 7.00-7.03(1H, m), 7.24(1H, t, J=8.1Hz), 7.50-7.55(2H, m), 7.77(1H, dt, J=6.7Hz, 1.9Hz), 7.88-7.91(1H, m), 8.30(1H, d, J=1.9Hz).

EXAMPLE 5

Production of (E)-N-(6,6-dimethyl-2-hepten-4-ynyl)-N-methyl-3-[3-(1-imidazolyl)benzyloxy]benzylamine:-

90 mg of 1-(3-hydroxymethylphenyl)imidazole was dissolved in 10 ml of chloroform, and 100 microliters of thionyl chloride was added. The mixture was stirred at room temperature for 3 hours. The solvent was evaporated under reduced pressure. The residue was dissolved in a mixture of ethyl ether and water. The organic layer was separated, washed succesively with a 5 % aqueous solution of sodium hydrogen carbonate and then with water, and dried over anhydrous magnesium sulfate. The desiccant was separated by filtration and then ethyl ether was evaporated to give 1-(3-chloromethylphenyl)imidazole as a pale yellow oily product.

The resulting chloromethyl compound was dissolved in 1.5 ml of dimethylformamide, and a tetrahydrofuran solution (1.5 ml) of phenolate prepared from 140 mg of (E)-N-(6,6-dimethyl-2-hepten-4-ynyl)-N-methyl-3-hydroxybenzylamine and 22 mg of 60 % oily sodium hydride was added. The mixture was reacted as in Examples 1 to 4 to give 110 mg (yield 57 %) of the captioned compound as a colorless oil.

IR $\nu_{max}^{neat}$ $cm^{-1}$: 1599, 1506, 1491, 1455, 1311, 1263, 1056, 1026, 786.

NMR ($CDCl_3$) $\delta$: 1.24(9H, s), 2.18(3H, s), 3.36(2H, dd, J=6.5Hz, 1.4Hz), 3.47(2H, s), 5.13(2H, s), 5.65(1H, dt, J=15.8Hz, 1.4Hz), 6.07(1H, dt, J=15.8Hz, 6.5Hz), 6.87(1H, ddd, J=8.3Hz, 2.7Hz, 1.0Hz), 6.92(1H, d, J=8.3Hz), 6.99-7.10(1H, m), 7.22(1H, t, J=1.4Hz), 7.24(1H, t, J=7.7Hz), 7.31(1H, t, J=1.4Hz), 7.34-7.45(2H, m), 7.50(1H, t, J=7.7Hz), 7.51(1H, s), 7.88(1H, t, J=1.4Hz).

Compounds of Examples 6 to 44 below were obtained by performing the same reaction as in Examples 1 to 5 except that instead of the various starting compounds in Examples 1 to 5, the corresponding 3-hydroxybenzylamine derivatives and 3-heterocyclyl benzylhalogen derivatives or methanesulfonyl derivatives or starting materials therefor were used.

Example 6

(E)-N-(6,6-dimethyl-2-hepten-4-ynyl)-N-ethyl-3-[3-(3-furyl)benzyloxy]benzylamine:-

IR $\nu_{max}^{neat}$ $cm^{-1}$: 2968, 1458, 1263, 1161, 1059, 1038, 1020, 873, 777.

NMR ($CDCl_3$) $\delta$ : 1.03(3H, t, J=7.1Hz), 1.24(9H, s), 2.50(2H, q, J=7.1Hz), 3.09(2H, d, J=6.4Hz), 3.54(2H, s), 5.08(2H, s), 5.64(1H, dt, J=15.9Hz, 1.5Hz), 6.07(1H, dt, J=15.9Hz, 6.4Hz), 6.71(1H, dd, J=1.8Hz, 0.9Hz), 6.87 (1H, dd, J=7.8Hz, 2.0Hz), 6.92(1H, d, J=7.6Hz), 7.02(1H, br.s), 7.22(1H, t, J=7.8Hz), 7.34 (1H, dt, J=7.4Hz, 1.7Hz), 7.39(1H, t, J=7.1Hz), 7.45(1H, dt, J=7.4Hz, 1.7Hz), 7.48(1H, t, J=1.7Hz), 7.57(1H, br.s), 7.75(1H, t, J=1.4Hz).

Example 7

(E)-N-(6,6-dimethyl-2-hepten-4-ynyl)-N-methyl-3-[3-(2-thienyl)benzyloxy]benzylamine:-

IR $\nu_{max}^{neat}$ $cm^{-1}$: 2968, 1599, 1491, 1455, 1365, 1266, 1152, 1026, 786, 696.

NMR ($CDCl_3$) $\delta$ : 1.24(9H, s), 2.18(3H, s), 3.04 (2H, dd, J=6.6Hz, 1.5Hz), 3.47(2H, s), 5.10 (2H, s), 5.65(1H, dt, J=15.8Hz, 1.5Hz), 6.08 (1H, dt, J=15.8Hz, 6.6Hz), 6.86-6.93(2H, m), 7.00(1H, br.s), 7.23(1H, t, J=7.8Hz), 7.29 (1H, dd, J=5.1Hz, 1.1Hz), 7.33(1H, dd, J=3.6Hz, 1.1Hz), 7.36-7.39(1H, m), 7.40(1H, t, J=7.6Hz), 7.57(1H, dt, J=7.6Hz, 1.8Hz), 7.68-7.71(1H, m).

Example 8

(E)-N-(6,6-dimethyl-2-hepten-4-ynyl)-N-methyl-3-[3-(3-thienyl)benzyloxy]benzylamine:-

IR $\nu_{max}^{neat}$ $cm^{-1}$: 2968, 1599, 1491, 1455, 1365, 1263, 1026, 774.

NMR ($CDCl_3$) $\delta$ : 1.24(9H, s), 2.18(3H, s), 3.03 (2H, dd, J=6.6Hz, 1.5Hz), 3.47(2H, s), 5.10 (2H, s), 5.64(1H, dt, J=15.8Hz, 1.5Hz), 6.08

(1H, dt, J=15.8Hz, 6.6Hz), 6.86-6.92(2H, m), 7.00(1H, br.s), 7.23(1H, t, J=8.0Hz), 7.36 (1H, dt, J=7.6Hz, 1.6Hz), 7.39-7.40(2H, m), 7.41(1H, t, J=7.6Hz), 7.46-7.48(1H, m), 7.55 (1H, dt, J=7.6Hz, 1.7Hz), 7.67(1H, br.s).

Example 9

(E)-N-(6,6-dimethyl-2-hepten-4-ynyl)-N-ethyl-3-[3-(3-thienyl)benzyloxy]benzylamine hydrochloride:-

m.p. 168-169 °C.

IR $\nu_{max}^{KBr}$ $cm^{-1}$: 2974, 2500, 1602, 1461, 1266, 1173, 777, 759, 747.

NMR ($CDCl_3$) $\delta$ : 1.25(9H, s), 1.42(3H, t, J=7.3Hz), 2.95-3.06(2H, m), 3.50-3.67(2H, m), 4.08(2H, d, J=5.3Hz), 5.23(2H, s), 5.80(1H, d, J=15.7Hz), 6.22(1H, dt, J=15.7Hz, 7.5Hz), 7.06(1H, ddd, J=8.3Hz, 2.5Hz, 0.8Hz), 7.09(1H, d, J=8.3Hz), 7.33(1H, t, J=8.1Hz), 7.37-7.43(4H, m), 7.51 (1H, dd, J=2.8Hz, 1.5Hz), 7.53-7.58(2H, m), 7.73(1H, br.s).

Example 10

(E)-N-(6,6-dimethyl-2-hepten-4-ynyl)-N-propyl-3-[3-(3-thienyl)benzyloxy]benzylamine hydrochloride:-

m.p. 164-166 °C.

IR $\nu_{max}^{KBr}$ $cm^{-1}$: 2968, 1605, 1458, 1266, 777.

NMR ($CDCl_3$) $\delta$ : 0.92(3H, t, J=7.3Hz), 1.25(9H, s), 1.82-1.98(2H, m), 2.73-2.92(2H, m), 3.44-3.78 (2H, m), 4.09(2H, br.s), 5.23(2H, s), 5.78(1H, d, J=15.6Hz), 6.21(1H, dt, J=15.6Hz, 7.8Hz), 7.04-7.09(2H, m), 7.33(1H, t, J=8.1Hz), 7.35-7.43(4H, m), 7.50(1H, dd, J=2.5Hz, 1.5Hz), 7.53-7.57(2H, m), 7.74(1H, s).

Example 11

(E)-N-(6,6-dimethyl-2-hepten-4-ynyl)-N-methyl-3-[3-(1-pyrrolyl)benzyloxy]benzylamine:-

IR $\nu_{max}^{neat}$ $cm^{-1}$: 1596, 1506, 1488, 1458, 1341, 1266, 1029, 786, 726.

NMR ($CDCl_3$) $\delta$ : 1.24(9H, s), 2.18(3H, s), 3.03(2H, dd, J=6.6Hz, 1.5Hz), 3.47(2H, s), 5.11(2H, s), 5.64(1H, dt, J=15.8Hz, 1.5Hz), 6.07(1H, dt, J=15.8Hz, 6.6Hz), 6.35(2H, t, J=2.2Hz), 6.86 (1H, ddd, J=10.8Hz, 2.3Hz, 0.8Hz), 6.91(1H, d, J=7.8Hz), 6.98-6.99(1H, m), 7.10(2H, t, J=2.2Hz), 7.22(1H, t, J=7.8Hz), 7.25-7.50 (4H, m).

Example 12

(E)-N-(6,6-dimethyl-2-hepten-4-ynyl)-N-propyl-3-[3-(1-pyrrolyl)benzyloxy]benzylamine:-

IR $\nu_{max}^{neat}$ $cm^{-1}$: 2968, 1596, 1506, 1488, 1455, 1341, 1263, 1071, 723.

NMR ($CDCl_3$) $\delta$ : 0.85(3H, t, J=7.4Hz), 1.24(9H, s), 1.47(2H, sex., J=7.4Hz), 2.36(2H, t, J=7.4Hz), 3.06(2H, dd, J=6.4Hz, 1.6Hz), 3.53(2H, s), 5.11(2H, s), 5.63(1H, dt, J=15.9Hz, 1.6Hz), 6.05(1H, dt, J=15.9Hz, 6.4Hz), 6.35(2H, t, J=2.2Hz), 6.85(1H, ddd, J=8.2Hz, 2.6Hz, 0.9Hz), 6.92(1H, d, J=7.6Hz), 7.00-7.03(1H, m), 7.11 (2H, t, J=2.2Hz), 7.21(1H, t, J=7.8Hz), 7.28-7.37(2H, m), 7.44(1H, t, J=7.8Hz), 7.49 (1H, t, J=1.5Hz).

Example 13

(E)-N-(6,6-dimethyl-2-hepten-4-ynyl)-N-methyl-3-[3-(2-pyridyl)benzyloxy]benzylamine:-

IR $\nu_{max}^{neat}$ $cm^{-1}$: 2968, 1587, 1464, 1365, 1263, 1152, 1026, 768.

NMR ($CDCl_3$) $\delta$ : 1.24(9H, s), 2.18(3H, s), 3.03(1H, dd, J=6.5Hz, 1.5Hz), 3.47(2H, s), 5.15(2H, s), 5.64(1H, dt, J=15.9Hz, 1.5Hz), 6.08(1H, dt, J=15.9Hz, 6.5Hz), 6.86-6.92(2H, m), 7.00(1H, br.s), 7.22-7.27(3H, m), 7.49-7.51(2H, m), 7.75-7.77(2H, m), 7.93-7.96(1H, m), 8.08 (1H, br.s), 8.70(1H, dt, J=4.8Hz, 1.5Hz).

Example 14

(E)-N-(6,6-dimethyl-2-hepten-4-ynyl)-N-methyl-3-[3-(3-pyridyl)benzyloxy]benzylamine:-

IR $\nu_{max}^{neat}$ $cm^{-1}$: 2968, 1599, 1491, 1458, 1365, 1263, 1152, 1023, 783.

NMR ($CDCl_3$) $\delta$ : 1.24(9H, s), 2.18(3H, s), 3.03(2H, dd, J=6.5Hz, 1.5Hz), 3.47(2H, s), 5.62(2H, s), 5.64(1H, dt, J=15.9Hz, 1.5Hz), 6.07(1H, dt, J=15.9Hz, 6.6Hz), 6.87-6.93(2H, m), 7.01(1H, br.s), 7.23(1H, t, J=7.9Hz), 7.37(1H, ddd, J=6.8Hz, 5.0Hz, 0.9Hz), 7.48-7.57(3H, m), 7.67 (1H, br.s), 7.87-7.91(1H, m), 8.60(1H, dd, J=4.8Hz, 1.7Hz), 8.86(1H, dd, J=2.4Hz, 0.9Hz).

Example 15

(E)-N-(6,6-dimethyl-2-hepten-4-ynyl)-N-ethyl-3-[3-(3-pyridyl)benzyloxy]benzylamine:-

IR $\nu_{max}^{neat}$ $cm^{-1}$: 2968, 1596, 1458, 1263, 786, 711.

NMR ($CDCl_3$) $\delta$ : 1.02(3H, t, J=7.1Hz), 1.23(9H, s), 2.50(2H, q, J=7.1Hz), 3.09(2H, d, J=6.3Hz), 3.54(2H, s), 5.14(2H, s), 5.63(1H, dd, J=15.9Hz, 1.4Hz), 6.06(1H, dt, J=15.9Hz, 6.3Hz), 6.87 (1H, dd, J=8.0Hz, 2.7Hz), 6.92(1H, d, J=7.6Hz), 7.03(1H, br.s), 7.22(1H, t, J=7.8Hz), 7.36 (1H, ddd, J=7.6Hz, 4.9Hz, 1.2Hz), 7.48-7.57 (3H, m), 7.66(1H, d, J=1.2Hz), 7.87-7.91 (1H, m), 8.60(1H, dd, J=5.1Hz, 1.8Hz), 8.85 (1H, dd, J=2.7Hz, 1.2Hz).

Example 16

(E)-N-(6,6-dimethyl-2-hepten-4-ynyl)-N-methyl-3-[3-(4-pyridyl)benzyloxy]benzylamine:-

IR $\nu_{max}^{neat}$ $cm^{-1}$: 2972, 1596, 1490, 1458, 1364, 1266, 1152, 1026, 786.

NMR ($CDCl_3$) $\delta$ : 1.24(9H, s), 2.18(3H, s), 3.04(1H, dd, J=6.5Hz, 1.5Hz), 3.47(2H, s), 5.14(2H, s), 5.64(1H, dt, J=15.8Hz, 1.5Hz), 6.08(1H, dt,

J=15.9Hz, 1.5Hz), 6.87-6.93(2H, m), 7.00(1H, br.s), 7.24(1H, t, J=8.0Hz), 7.51-7.54(4H, m), 7.59-7.62(1H, m), 7.59-7.62(1H, m), 8.67(2H, dd, J=4.5Hz, 1.7Hz).

Example 17

(E)-N-(6,6-dimethyl-2-hepten-4-ynyl)-N-methyl-3-[3-(2-oxazolyl)benzyloxy]benzylamine:-

IR $\nu_{max}^{neat}$ $cm^{-1}$: 2974, 1590, 1491, 1458, 1365, 1266, 1152, 1026, 798, 729.

NMR ($CDCl_3$) $\delta$ : 1.24(9H, s), 2.18(3H, s), 3.30(2H, dd, J=6.6Hz, 1.5Hz), 3.46(2H, s), 5.12(2H, s), 5.63(1H, dd, J=15.8Hz, 1.5Hz), 6.08(1H, dt, J=15.8Hz, 6.6Hz), 6.85-6.92(2H, m), 6.99-7.15 (1H, m), 7.22(1H, t, J=7.7Hz), 7.24(1H, d, J=1.1Hz), 7.48(1H, t, J=7.7Hz), 7.54(1H, d, J=7.9Hz), 7.72(1H, d, J=1.1Hz), 8.01(1H, dt, J=7.9Hz, 1.2Hz), 8.13-8.16(1H, m).

Example 18

(E)-N-(6,6-dimethyl-2-hepten-4-ynyl)-N-methyl-3-[3-(5-oxazolyl)benzyloxy]benzylamine:-

IR $\nu_{max}^{neat}$ $cm^{-1}$: 2968, 1596, 1458, 1263, 1152, 1026, 954, 789, 693.

NMR ($CDCl_3$) $\delta$ : 1.24(9H, s), 2.19(3H, s), 3.04(2H, dd, J=6.5Hz, 1.4Hz), 3.47(2H, s), 5.11(2H, s), 5.65(1H, dt, J=15.9Hz, 1.4Hz), 6.08(1H, dt, J=15.9Hz, 6.5Hz), 6.86-6.93(2H, m), 7.00-7.02 (1H, m), 7.23(1H, t, J=7.9Hz), 7.38(1H, s), 7.42-7.43(1H, m), 7.45(1H, t, J=7.6Hz), 7.60-7.64(1H, m), 7.74-7.76(1H, m), 7.93 (1H. s).

Example 19

(E)-N-(6,6-dimethyl-2-hepten-4-ynyl)-N-ethyl-3-[3-(5-oxazolyl)benzyloxy]benzylamine:-

IR $\nu_{max}^{neat}$ $cm^{-1}$: 2968, 1491, 1458, 1263, 1107, 954, 789.

NMR ($CDCl_3$) δ : 1.03(3H, t, J=7.1Hz), 1.24(9H, s), 2.50(2H, q, J=7.1Hz), 3.09(2H, dd, J=6.4Hz, 1.5Hz), 3.54(2H, s), 5.11(2H, s), 5.64(1H, dt, J=15.8Hz, 1.5Hz), 6.07(1H, dt, J=15.8Hz, 6.4Hz), 6.84-6.89(1H, m), 6.93(1H, d, J=7.7Hz), 7.03(1H, br.s), 7.23(1H, t, J=7.7Hz), 7.39 (1H, s), 7.40-7.49(2H, m), 7.62(1H, dt, J=6.6Hz, 2.1Hz), 7.75(1H, br.s), 7.93(1H, s).

Treatment of the free base with hydrogen chloride-methanol in a customary manner gave the hydrochloride, m.p. 160 °C (dec.).

Example 20

(E)-N-(6,6-dimethyl-2-hepten-4-ynyl)-N-propyl-3-[3-(5-oxazolyl)benzyloxy]benzylamine:-

IR $\nu_{max}^{neat}$ $cm^{-1}$: 2968, 1596, 1491, 1455, 1263, 1107, 954, 789.

NMR ($CDCl_3$) δ : 0.85(3H, t, J=7.4Hz), 1.47(2H, sex, J=7.4Hz), 2.37(2H, t, J=7.4Hz), 3.07(2H, dd, J=6.4Hz, 1.5Hz), 3.53(2H, s), 3.53(2H, s), 5.10(2H, s), 5.63(1H, dt, J=15.9Hz, 1.5Hz), 6.06(1H, dt, J=15.9Hz, 6.4Hz), 6.84-6.89 (1H, m), 6.92(1H, d, J=7.8Hz), 7.02(1H, br.s), 7.22(1H, t, J=7.8Hz), 7.38(1H, s), 7.40-7.49(2H, m), 7.62(1H, dt, J=6.6Hz, 2.1Hz), 7.75(1H, br.s), 7.93(1H, s).

Example 21

(E)-N-(6,6-dimethyl-2-hepten-4-ynyl)-N-methyl-3-[3-(4-isoxazolyl)benzyloxy]benzylamine:-

IR $\nu_{max}^{neat}$ $cm^{-1}$: 2974, 1602, 1494, 1458, 1266, 756.

NMR ($CDCl_3$) δ : 1.24(9H, s), 2.19(3H, s), 3.04 (2H, dd, J=6.5Hz, 1.4Hz), 3.48(2H, s), 5.10 (2H, s), 5.65(1H, dt, J=15.9Hz, 1.4Hz), 6.08(2H, dt, J=15.9Hz, 6.5Hz), 6.85-6.95 (2H, m), 7.01(1H, br.s), 7.24(1H, t, J=8.0Hz), 7.38-7.49(3H, m), 7.57(1H, br.s), 8.58(1H, s), 8.70(1H, s).

Example 22

(E)-N-(6,6-dimethyl-2-hepten-4-ynyl)-N-methyl-3-[3-(2-thiazolyl)benzyloxy]benzylamine:-

IR $\nu_{max}^{neat}$ $cm^{-1}$: 2972, 1588, 1490, 1456, 1364, 1266, 1148, 1022, 788, 692.

NMR ($CDCl_3$) $\delta$ : 1.19(9H, s), 2.14(3H, s), 2.99 (2H, dd, J=6.6Hz, 1.4Hz), 3.42(2H, s), 5.08 (2H, s), 5.60(1H, dt, J=15.8Hz, 1.4Hz), 6.04 (1H, dt, J=15.8Hz, 6.6Hz), 6.83(1H, dd, J=8.1Hz, 1.5Hz), 6.87(1H, d, J=8.1Hz), 6.95 (1H, d, J=1.5Hz), 7.18(1H, t, J=7.8Hz), 7.30 (1H, d, J=3.3Hz), 7.42(1H, t, J=8.1Hz), 7.47 (1H, d, J=7.6Hz), 7.83(1H, d, J=3.3Hz), 7.87 (1H, d, J=7.3Hz), 8.01(1H, s).

Example 23

(E)-N-(6,6-dimethyl-2-hepten-4-ynyl)-N-methyl-3-[3-(4-isothiazolyl)benzyloxy]benzylamine:-

IR $\nu_{max}^{neat}$ $cm^{-1}$: 2968, 1590, 1491, 1458, 1365, 1263, 1152, 1026, 780.

NMR ($CDCl_3$) $\delta$ : 1.24(9H, s), 2.18(3H, s), 3.03 (2H, dd, J=6.5Hz, 1.5Hz), 3.47(2H, s), 5.12 (2H, s), 5.64(1H, dt, J=15.9Hz, 1.5Hz), 6.07 (1H, dt, J=15.9Hz, 6.5Hz), 6.85-6.94(2H, m), 7.01(1H, t, J=2.1Hz), 7.23(1H, t, J=7.9Hz), 7.40-7.50(2H, m), 7.55(1H, dt, J=6.9Hz, 1.9Hz), 8.73(1H, s), 8.79(1H, s).

Example 24

(E)-N-(6,6-dimethyl-2-hepten-4-ynyl)-N-methyl-3-[3-(5-isothiazolyl)benzyloxy]benzylamine:-

IR $\nu_{max}^{neat}$ $cm^{-1}$: 2968, 1590, 1491, 1458, 1419, 1368, 1266, 1152, 786, 756.

NMR ($CDCl_3$) $\delta$ : 1.24(9H, s), 2.19(3H, s), 3.04 (2H, dd, J=6.2Hz, 1.5Hz), 3.48(2H, s), 5.11 (2H, s), 5.65(1H, dt, J=15.6Hz, 1.5Hz), 6.08 (1H, dt, J=15.6Hz, 6.2Hz), 6.88(1H, ddd,

J=8.2Hz, 2.8Hz, 1.0Hz), 6.89-6.90(2H, m), 7.00-7.03(1H, m), 7.23(1H, t, J=7.8Hz), 7.43 (1H, d, J=2.1Hz), 7.45-7.50(2H, m), 7.54-7.59 (1H, m), 7.68-7.71(1H, m), 8.48(1H, d, J=2.1Hz).

Example 25

(E)-N-(6,6-dimethyl-2-hepten-4-ynyl)-N-ethyl-3-[3-(1-imidazolyl)benzyloxy]benzylamine:-

IR $\nu_{max}^{neat}$ $cm^{-1}$: 2968, 1506, 1491, 1458, 1308, 1263, 1056, 789.

NMR ($CDCl_3$) $\delta$: 1.02(3H, t, J=7.1Hz), 1.24(9H, s), 2.49(2H, q, J=7.1Hz), 3.08(2H, dd, J=6.4Hz, 1.5Hz), 3.54(2H, s), 5.13(2H, s), 5.64(1H, dt, J=15.9Hz, 1.5Hz), 6.06(1H, dt, J=15.9Hz, 6.4Hz), 6.85(1H, ddd, J=8.0Hz, 2.0Hz, 0.9Hz), 6.93(1H, d, J=7.5Hz), 7.00-7.05(1H, m), 7.21 (1H, t, J=1.4Hz), 7.23(1H, t, J=8.0Hz), 7.31 (1H, t, J=1.4Hz), 7.33-7.53(4H, m), 7.88(1H, t, J=1.4Hz).

Example 26

(E)-N-(6,6-dimethyl-2-hepten-4-ynyl)-N-propyl-3-[3-(1-imidazolyl)benzyloxy]benzylamine:-

IR $\nu_{max}^{neat}$ $cm^{-1}$: 2968, 1599, 1506, 1488, 1455, 1308, 1263, 1152, 1056, 786.

NMR ($CDCl_3$) $\delta$ : 0.85(3H, t, J=7.4Hz), 1.24(9H, s), 1.47(2H, sex, J=7.4Hz), 2.37(2H, t, J=7.4Hz), 3.06(2H, dd, J=6.3Hz, 1.5Hz), 3.53(2H, s), 5.13(2H, s), 5.63(1H, dt, J=15.6Hz, 1.5Hz), 6.05(1H, dt, J=15.6Hz, 6.3Hz), 6.84(1H, ddd, J=8.2Hz, 3.4Hz, 0.8Hz), 6.93(1H, d, J=7.6Hz), 7.01(1H, br.s), 7.21(1H, t, J=1.4Hz), 7.22 (1H, t, J=8.2Hz), 7.31(1H, t, J=1.4Hz), 7.33-7.53(4H, m), 7.88(1H, t, J=1.4Hz).

Example 27

(E)-N-(6,6-dimethyl-2-hepten-4-ynyl)-N-methyl-3-[3-(5-pyrimidinyl)benzyloxy]benzylamine:-

IR $\nu_{max}^{neat}$ $cm^{-1}$: 2974, 1584, 1455, 1419, 1266, 786, 756.

NMR ($CDCl_3$) $\delta$: 1.24(9H, s), 2.19(3H, s), 3.04 (2H, d, J=6.2Hz), 3.47(2H, s), 5.15(2H, s), 5.64(1H, d, J=15.9Hz), 6.07(1H, dt, J=15.9Hz, 6.2Hz), 6.82-6.93(2H, m), 7.02(1H, br.s), 7.23(1H, t, J=7.5Hz), 7.54-7.55(3H, m), 7.67 (1H, s), 8.96(2H, s), 9.22(1H, s).

Example 28

(E)-N-(6,6-dimethyl-2-hepten-4-ynyl)-N-methyl-3-[3-(1,2,4-triazol-1-yl)benzyloxy]benzylamine:-

IR $\nu_{max}^{neat}$ $cm^{-1}$: 2974, 1599, 1512, 1458, 1266, 1215, 1146, 759.

NMR ($CDCl_3$) $\delta$: 1.23(9H, s), 2.18(3H, s), 3.03 (2H, dd, J=6.6Hz, 1.5Hz), 3.46(2H, s), 5.14 (2H, s), 5.64(1H, dt, J=15.9Hz, 1.5Hz), 6.06 (1H, dt, J=15.9Hz, 6.6Hz), 6.85(1H, ddd, J=7.9Hz, 2.7Hz, 0.5Hz), 6.92(1H, d, J=7.9Hz), 6.98-7.05(1H, m), 7.23(1H, t, J=7.9Hz), 7.45-7.49(1H, m), 7.53(1H, t, J=7.8Hz), 7.64 (1H, dt, J=7.8Hz, 1.8Hz), 7.80(1H, br.s), 8.11(1H, s), 8.58(1H, s).

Example 29

(E)-N-ethyl-N-(6-methoxy-6-methyl-2-hepten-4-ynyl)-3-[3-(1-pyrrolyl)benzyloxy]benzylamine:-

IR $\nu_{max}^{neat}$ $cm^{-1}$: 1605, 1506, 1341, 1170, 1149, 1074, 960, 789, 723, 696.

NMR ($CDCl_3$) $\delta$: 1.09(3H, t, J=6.9Hz), 1.46(6H, s), 2.56(2H, q, J=6.9Hz), 3.15(2H, dd, J=6.5Hz, 1.5Hz), 3.35(3H, s), 3.61(2H, s), 5.12(2H, s), 5.73(1H, dt, J=15.9Hz, 1.5Hz), 6.20(1H, dt, J=15.9Hz, 6.5Hz), 6.37(2H, t, J=2.1Hz), 6.86 (1H, dd, J=7.8Hz, 1.6Hz), 6.93(1H, d, J=7.8Hz), 6.99-7.05(1H, m), 7.10(2H, t, J=2.1Hz), 7.23(1H, t, J=7.5Hz), 7.29-7.46(3H, m), 7.48-7.50(1H, m).

Example 30

(E)-N-(6,6-dimethyl-2-hepten-4-ynyl)-N-methyl-3-[3-(2,3-dihydro-4-thienyl)benzyloxy]benzylamine:-

IR $\nu_{max}^{neat}$ $cm^{-1}$: 2968, 2788, 1584, 1491, 1455, 1365, 1263, 1152, 1023, 783.

NMR ($CDCl_3$) $\delta$: 1.24(9H, s), 2.18(3H, s), 3.03 (2H, dd, J=6.6Hz, 1.4Hz), 3.11-3.19(2H, m), 3.35-3.43(2H, m), 3.46(2H, s), 5.04(2H, s), 5.64(1H, dt, J=15.9Hz, 1.4Hz), 6.08(1H, dt, J=15.9Hz, 6.6Hz), 6.59(1H, t, J=2.1Hz), 6.83-6.93(2H, m), 6.97-7.00(1H, m), 7.19-7.33 (4H, m), 7.39-7.42(1H, m).

Example 31

(E)-N-(6,6-dimethyl-2-hepten-4-ynyl)-N-ethyl-3-[3-(2,3-dihydro-4-thienyl)benzyloxy]benzylamine:-

IR $\nu_{max}^{neat}$ $cm^{-1}$: 2968, 2926, 1587, 1491, 1455, 1365, 1263, 1149, 777.

NMR ($CDCl_3$) $\delta$: 1.03(3H, t, J=7.1Hz), 1.24(9H, s), 2.49(2H, q, J=7.1Hz), 3.08(2H, dd, J=6.4Hz, 1.5Hz), 3.15(2H, dt, J=8.4Hz, 1.8Hz), 3.38 (2H, t, J=8.4Hz), 3.53(2H, s), 5.04(2H, s), 5.64(1H, dt, J=15.8Hz, 1.5Hz), 6.07(1H, dt, J=15.8Hz, 6.4Hz), 6.59(1H, t, J=1.8Hz), 6.82-6.87(1H, m), 6.89-6.94(1H, m), 6.98-7.02 (1H, m), 7.21(1H, t, J=7.8Hz), 7.23-7.35 (3H, m), 7.39-7.42(1H, m).

Example 32

(E)-N-ethyl-N-(6-methoxy-6-methyl-2-hepten-4-ynyl)-3-[3-(2,3-dihydro-4-thienyl)benzyloxy]benzylamine:-

IR $\nu_{max}^{neat}$ $cm^{-1}$: 1452, 1380, 1365, 1254, 1173, 1149, 1077, 819, 777, 693.

NMR ($CDCl_3$) $\delta$: 1.04(3H, t, J=7.0Hz), 1.46(6H, s), 2.51(2H, q, J=7.0Hz), 3.09-3.18(4H, m), 3.35(3H, s), 3.35-3.42(2H, m), 3.54(2H, s),

5.04(2H, s), 5.68(1H, dt, J=15.7Hz, 1.8Hz), 6.16(1H, dt, J=15.7Hz, 6.6Hz), 6.59(1H, t, J=1.6Hz), 6.83-6.89(1H, m), 6.90-6.95(1H, m), 6.98-7.04(1H, m), 7.21(1H, t, J=7.5Hz), 7.25-7.35(3H, m), 7.39-7.40(1H, m).

Example 33

(E)-N-(6,6-dimethyl-2-hepten-4-ynyl)-N-ethyl-3-[3-(2,5-dihydro-3-thienyl)benzyloxy]benzylamine:-

IR $\nu_{max}^{neat}$ $cm^{-1}$: 2968, 2920, 1587, 1491, 1455, 1365, 1263, 1152, 777.

NMR ($CDCl_3$)$\delta$ : 1.02(3H, t, J=7.1Hz), 1.24(9H, s), 2.49(2H, q, J=7.1Hz), 3.08(2H, dd, J=6.4Hz, 1.5Hz), 3.53(2H, s), 3.91-3.96(2H, m), 4.11-4.15(2H, m), 5.06(2H, s), 5.64(1H, dt, J=15.8Hz, 1.5Hz), 6.05(1H, dt, J=15.8Hz, 6.4Hz), 6.26(1H, quint, J=2.0Hz), 6.82-6.87 (1H, m), 6.89-6.94(1H, m), 6.99-7.01(1H, m), 7.21(1H, t, J=8.0Hz), 7.34-7.37(3H, m), 7.47-7.49(1H, m).

Example 34

(E)-N-(6,6-dimethyl-2-hepten-4-ynyl)-N-methyl-3-[3-(1-pyrrolidinyl)benzyloxy]benzylamine:-

IR $\nu_{max}^{neat}$ $cm^{-1}$: 2968, 1608, 1584, 1506, 1491, 1458, 1368, 1266, 1152, 768.

NMR ($CDCl_3$)$\delta$ : 1.24(9H, s), 1.97-2.02(4H, m), 2.18(3H, s), 3.03(2H, dd, J=6.6Hz, 1.5Hz), 3.29-3.31(4H, m), 3.46(2H, s), 5.01(2H, s), 5.64(1H, dt, J=15.9Hz, 1.5Hz), 6.08(1H, dt, J=15.9Hz, 6.5Hz), 6.51(1H, dd, J=8.3Hz, 2.4Hz), 6.62-6.64(1H, m), 6.72(1H, d, J=7.4Hz), 6.85-6.90(2H, m), 6.98(1H, t, J=2.1Hz), 7.20-7.22(2H, m).

Example 35

(E)-N-(6,6-dimethyl-2-hepten-4-ynyl)-N-ethyl-3-[5-(3-thienyl)-2-thienylmethyloxy]benzylamine:-

IR $\nu_{max}^{neat}$ $cm^{-1}$: 2968, 2926, 1596, 1446, 1377, 1260, 1017, 852, 801, 771.

NMR ($CDCl_3$) $\delta$ : 1.04(3H, t, J=7.0Hz), 1.24(9H, s), 2.50(2H, q, J=7.0Hz), 3.10(1H, dd, J=6.3Hz, 1.4Hz), 3.54(2H, s), 5.18(2H, s), 5.64(1H, dt, J=15.9Hz, 1.4Hz), 6.08(1H, dt, J=15.9Hz, 6.3Hz), 6.86(1H, dd, J=8.0Hz, 2.7Hz), 6.93 (1H, d, J=7.6Hz), 7.00-7.04(2H, m), 7.07(1H, d, J=3.6Hz), 7.22(1H, t, J=8.0Hz), 7.29(1H, dd, J=5.2Hz, 1.2Hz), 7.33(1H, dd, J=5.2Hz, 4.5Hz), 7.36(1H, dd, J=4.5Hz, 1.2Hz).

Example 36

(E)-N-(6,6-dimethyl-2-hepten-4-ynyl)-N-methyl-3-[2-(5-oxazolyl)-4-pyridylmethyloxy]benzylamine:-

IR $\nu_{max}^{neat}$ $cm^{-1}$: 2968, 1620, 1491, 1455, 1365, 1266, 1113, 957, 831, 762.

NMR ($CDCl_3$) $\delta$ : 1.24(9H, s), 2.19(3H, s), 3.05 (2H, dd, J=6.5Hz, 1.5Hz), 3.48(2H, s), 5.14 (2H, s), 5.65(1H, dt, J=15.9Hz, 1.5Hz), 6.09 (1H, dt, J=15.9Hz, 6.5Hz), 6.86(1H, ddd, J=7.9Hz, 2.7Hz, 0.9Hz), 6.94(1H, d, J=7.9Hz), 7.00-7.01(1H, m), 7.25(1H, t, J=7.9Hz), 7.32 (1H, dt, J=5.0Hz, 0.8Hz), 7.73(1H, s), 7.76-7.77(1H, m), 7.99(1H, s), 8.64(1H, dd, J=5.0Hz, 0.8Hz).

Example 37

(E)-N-(6,6-dimethyl-2-hepten-4-ynyl)-N-methyl-3-[4-(5-oxazolyl)-2-pyridylmethyloxy]benzylamine:-

IR $\nu_{max}^{neat}$ $cm^{-1}$: 2968, 1617, 1584, 1494, 1455, 1263, 1155, 1113, 957.

NMR ($CDCl_3$) $\delta$ : 1.24(9H, s), 2.19(3H, s), 3.04 (2H, d, J=6.5Hz), 3.48(2H, s), 5.24(2H, s), 5.64(1H, dt, J=15.9Hz, 1.7Hz), 6.08(1H, dt, J=15.9Hz, 6.5Hz), 6.88-6.95(2H, m), 7.04-7.05 (1H, m), 7.19-7.27(1H, m), 7.46(1H, dd,

J=5.4Hz, 1.5Hz), 7.59(1H, s), 7.79(1H, m), 8.00(1H, s), 8.65(1H, d, J=6.2Hz).

Example 38

(E)-N-(6,6-dimethyl-2-hepten-4-ynyl)-N-methyl-3-[5-(5-oxazolyl)furfuryloxy]benzylamine:-

IR $\nu_{max}^{neat}$ $cm^{-1}$: 2968, 2926, 1458, 1263, 1107, 1020, 963, 789.

NMR ($CDCl_3$) $\delta$ : 1.24(9H, s), 2.19(3H, s), 3.04 (2H, dd, J=6.5Hz, 1.5Hz), 3.47(2H, s), 5.05 (2H, s), 5.65(1H, dt, J=15.9Hz, 1.5Hz), 6.09 (1H, dt, J=15.9Hz, 6.5Hz), 6.53(1H, d, J=3.4Hz), 6.64(1H, d, J=3.4Hz), 6.87(1H, ddd, J=8.1Hz, 2.7Hz, 0.8Hz), 6.93(1H, d, J=8.1Hz), 7.00(1H, m), 7.24(1H, t, J=8.1Hz), 7.30 (1H, s), 7.86(1H, s).

Example 39

(E)-N-(6,6-dimethyl-2-hepten-4-ynyl)-N-methyl-3-[3-(5-oxazolyl)-5-furylmethyloxy]benzylamine:-

IR $\nu_{max}^{neat}$ $cm^{-1}$: 2968, 1584, 1461, 1269, 1152, 1098, 1041, 1032, 891, 831.

NMR ($CDCl_3$) $\delta$ : 1.24(9H, s), 2.20(3H, s), 3.05 (2H, dd, J=6.5Hz, 1.4Hz), 3.48(2H, s), 4.97 (2H, s), 5.66(1H, dt, J=15.8Hz, 1.4Hz), 6.09 (1H, 15.8Hz, 6.5Hz), 6.75(1H, d, J=0.8Hz), 6.82-6.88(1H, m), 6.92(1H, d, J=7.8Hz), 6.98(1H, br.s), 7.23(1H, t, J=7.8Hz), 7.28 (1H, s), 7.54(1H, d, J=0.8Hz), 7.86(1H, s).

Example 40

(E)-N-(6,6-dimethyl-2-hepten-4-ynyl)-N-methyl-3-[2-(5-oxazolyl)-4-thiazolylmethyloxy]benzylamine:-

IR $\nu_{max}^{neat}$ $cm^{-1}$: 2968, 1596, 1491, 1455, 1263, 1158, 1104, 1059, 1017, 966, 894.

NMR ($CDCl_3$) $\delta$: 1.19(9H, s), 2.14(3H, s), 3.00 (2H, dd, J=6.6Hz, 1.4Hz), 3.43(2H, s), 5.22 (2H, d, J=0.96Hz), 5.60(1H, dt, J=15.8Hz,

1.4Hz), 6.03(1H, dt, J=15.8Hz, 6.6Hz), 6.83 (1H, ddd, J=7.6Hz, 2.6Hz, 0.75Hz), 6.89 (1H, d, J=7.6Hz), 6.97(1H, m), 7.19(1H, t, J=7.6Hz), 7.37(1H, t, J=0.96Hz), 7.63(1H, s), 7.92(1H, s).

Example 41

(E)-N-(6,6-dimethyl-2-hepten-4-ynyl)-N-methyl-3-[6-(5-oxazolyl)-2-pyridylmethyloxy]benzylamine:-

IR $\nu^{neat}_{max}$ $cm^{-1}$: 2968, 1596, 1452, 1365, 1260, 1158, 1107, 804, 786.

NMR ($CDCl_3$) δ: 1.24(9H, s), 2.18(3H, s), 3.03 (2H, dd, J=6.5Hz, 1.4Hz), 3.46(2H, s), 5.25 (2H, s), 5.64(1H, dt, J=15.8Hz, 1.4Hz), 6.07 (1H, dt, J=15.8Hz, 6.5Hz), 6.86-6.91(1H, m), 6.91-6.95(1H, m), 7.01-7.04(1H, m), 7.23(1H, t, J=8.0Hz), 7.51(1H, dd, J=7.8Hz, 0.9Hz), 7.59(1H, dd, J=7.8Hz, 0.9Hz), 7.72(1H, s), 7.80(1H, t, J=7.8Hz), 7.99(1H, s).

Example 42

(E)-N-(6,6-dimethyl-2-hepten-4-ynyl)-N-methyl-3-[5-(5-oxazolyl)-3-pyridylmethyloxy]benzylamine:-

IR $\nu^{neat}_{max}$ $cm^{-1}$: 2968, 1590, 1491, 1455, 1266, 1152, 1107, 1026, 963, 759.

NMR ($CDCl_3$) δ: 1.24(9H, s), 2.19(3H, s), 3.05 (2H, dd, J=6.6Hz, 1.4Hz), 3.48(2H, s), 5.13 (2H, s), 5.65(1H, dt, J=15.9Hz, 1.4Hz), 6.09 (1H, dt, J=15.9Hz, 6.6Hz), 6.88(1H, dd, J=7.8Hz, 2.6Hz), 6.94(1H, d, J=7.8Hz), 7.01-7.04(1H, m), 7.25(1H, t, J=7.8Hz), 7.49 (1H, s), 8.00(1H, s), 8.05(1H, t, J=2.0Hz), 8.65(1H, d, J=2.0Hz), 8.90(1H, d, J=2.0Hz).

Example 43

(E)-N-(6,6-dimethyl-2-hepten-4-ynyl)-N-methyl-3-[2-methyl-3-(1-pyrrolyl)benzyloxy]benzylamine hydrochloride:-

m.p. 137-139 °C.

IR $\nu_{max}^{neat}$ $cm^{-1}$: 2968, 2500, 1599, 1497, 1458, 1332, 1263, 1038, 723.

NMR ($CDCl_3$) $\delta$ : 1.25(9H, s), 2.18(3H, s), 2.65 (3H, s), 3.48-3.61(1H, m), 3.65-3.78(1H, m), 3.95-4.08(1H, m), 4.16-4.29(1H, m), 5.21 (2H, s), 5.85(1H, d, J=15.6Hz), 6.20-6.36 (1H, m), 6.32(2H, t, J=2.1Hz), 6.79(2H, t, J=2.1Hz), 7.04-7.11(1H, m), 7.24-7.30(2H, m), 7.35(1H, t, J=7.9Hz), 7.51(1H, t, J=4.5Hz), 7.63(1H, br.s).

<u>Example 44</u>

(E)-N-(6,6-dimethyl-2-hepten-4-ynyl)-N-methyl-3-[6-methyl-3-(1-pyrrolyl)benzyloxy]benzylamine:-

IR $\nu_{max}^{neat}$ $cm^{-1}$: 2968, 1590, 1518, 1488, 1455, 1341, 1266, 1026, 723.

NMR ($CDCl_3$) $\delta$ : 1.24(9H, s), 2.19(3H, s), 2.39 (3H, s), 3.04(2H, dd, J=6.5Hz, 1.5Hz), 3.48 (2H, s), 5.06(2H, s), 5.65(1H, dt, J=15.9Hz, 1.5Hz), 6.08(1H, dt, J=15.9Hz, 6.5Hz), 6.33 (2H, t, J=2.2Hz), 6.86-6.95(2H, m), 6.99-7.02 (1H, m), 7.07(2H, t, J=2.2Hz), 7.21-7.27 (3H, m), 7.50(1H, br.s).

EXAMPLE 45

Production of (E)-N-(6,6-dimethyl-2-hepten-4-ynyl)-N-methyl-3-[3-(4-thiazolyl)benzyloxy]benzylamine:-

600 mg of 2-chloro-4-(3-tolyl)thiazole was dissolved in a mixture of 8 ml of carbon tetrachloride and 2 ml of 1,2-dichloroethane. N-bromosuccinimide (511 mg) and 3 mg of benzoyl peroxide were added, and the mixture refluxed with stirring for 3 hours. After cooling, the precipitate was separated from the reaction mixture by filtration. It was washed with an aqueous solution of sodium hydrogen carbonate, and the solvent was evaporated under reduced pressure to give 4-(3-bromomethylphenyl)-2-chlorothiazole as a pale yellow oil.

The resulting bromomethyl compound was dissolved in 2 ml of dimethylformamide, and the solution was added to 8 ml of a tetrahydrofuran solution of phenolate prepared in advance from 388 mg of (E)-N-(6,6-dimethyl-2-hepten-4-ynyl)-N-methyl-3-hydroxybenzylamine and 68 mg of 60 % oily sodium hydride. The mixture was stirred for 1 hour under ice cooling and then for 2 hours at room temperature. Water and ethyl ether were added to the reaction solution. The organic layer was separated and dried over anhydrous sodium sulfate. The desiccant was separated by filtration, and the solvent was evaporated under reduced pressure. The residue was purified by silica gel column chromatography [Wakogel C-200, 80 g, eluting solvent: hexane/ethyl acetate=6/1 → 4/1] to give 396 mg (yield 57 %) of (E)-N-(6,6-dimethyl-2-hepten-4-ynyl)-N-methyl-3-[3-(2-chloro-4-thiazolyl)benzyloxy]-benzylamine as a pale yellow oil.

The resulting ether compound (92 mg) was dissolved in 0.8 ml of acetic acid. The solution was heated to 57 to 60 °C with stirring, and 40 mg of zinc powder was added at a time. The mixture was stirred for 30 minutes, and the reaction mixture was poured into ice water. Sodium carbonate was added to adjust the pH to 9.0. The solution was extracted with ethyl acetate. The extract was dried over anhydrous sodium sulfate, and the solvent was evaporated. The residue was purified by medium-pressure liquid chromatography [column: Lobar column, size B, Lichroprep Si 60 (E. Merck Co.); eluting solvent: hexane/ethyl acetate=6/1 → 3/1] to give 52 mg (yield 60 %) of the captioned compound as a colorless oil.

IR $\nu_{max}^{neat}$ $cm^{-1}$: 2968, 1587, 1491, 1455, 1365, 1266, 1026, 789.

NMR ($CDCl_3$) $\delta$: 1.24(9H, s), 2.18(3H, s), 3.03 (2H, dd, J=6.5Hz, 1.5Hz), 3.47(2H, s), 5.13 (2H, s), 5.64(1H, dt, J=15.8Hz, 1.5Hz),

6.08(1H, dt, J=15.8Hz, 6.5Hz), 6.86-6.92 (2H, m), 7.00(1H, br.s), 7.22(1H, t, J=7.8Hz), 7.44-7.45(1H, m), 7.57(1H, d, J=2.0Hz), 7.64 (1H, t, J=7.7Hz), 7.87-7.91(1H, m), 8.02(1H, br.s), 8.84(1H, d, J=2.0Hz).

Compounds of Examples 46 to 48 were obtained by using 5-(3-bromomethylphenyl)-2-bromothiazole obtained from 5-(3-tolyl)thiazole [see J. Org. Chem., 51, 3375 (1986); Org. React., 6, 381; and Ann., 628 (1981)] instead of 4-(3-bromomethylphenyl)-2-chlorothiazole, condensing it with the corresponding 3-hydroxybenzylamine derivative, and performing the same dehalogenation reaction as in Example 45.

Example 46

(E)-N-(6,6-dimethyl-2-hepten-4-ynyl)-N-methyl-3-[3-(5-thiazolyl)benzyloxy]benzylamine:-

IR $\nu_{max}^{neat}$ $cm^{-1}$: 2974, 1590, 1458, 1266, 873, 789, 693.

NMR ($CDCl_3$) $\delta$: 1.24(9H, s), 2.19(3H, s), 3.04 (2H, dd, J=6.6Hz, 1.5Hz), 3.47(2H, s), 5.10 (2H, s), 5.64(1H, dt, J=15.8Hz, 1.5Hz), 6.08(1H, dt, J=15.8Hz, 6.6Hz), 6.88(1H, dd, J=8.1Hz, 1.8Hz), 6.92(1H, d, J=7.8Hz), 7.00 (1H, t, J=1.8Hz), 7.23(1H, t, J=7.8Hz), 7.42-7.44(2H, m), 7.53-7.56(1H, m), 7.65-7.66 (1H, m), 8.10(1H, d, J=0.6Hz), 8.76(1H, d, J=0.6Hz).

Example 47

(E)-N-(6,6-dimethyl-2-hepten-4-ynyl)-N-ethyl-3-[3-(5-thiazolyl)benzyloxy]benzylamine:-

IR $\nu_{max}^{neat}$ $cm^{-1}$: 2972, 1588, 1490, 1456, 1364, 1264, 1152, 1046, 874, 788.

NMR ($CDCl_3$) $\delta$: 0.96(3H, t, J=7.0Hz), 1.19(9H, s), 2.46(2H, q, J=7.0Hz), 3.04(2H, d, J=6.0Hz), 3.50(2H, s), 5.06(2H, s), 5.60(1H, d, J=15.8Hz), 6.02(1H, dt, J=15.8Hz, 6.0Hz),

6.82(1H, dd, J=8.1Hz, 2.1Hz), 6.89(1H, d, J=8.0Hz), 6.98(1H, br.s), 7.18(1H, t, J=8.0Hz), 7.38-7.41(2H, m), 7.48-7.52(1H, m), 7.62 (1H, s), 8.05(1H, d, J=0.6Hz), 8.71(1H, d, J=0.6Hz).

Example 48

(E)-N-(6,6-dimethyl-2-hepten-4-ynyl)-N-propyl-3-[3-(5-thiazolyl)benzyloxy]benzylamine:-

IR $\nu_{max}^{neat}$ $cm^{-1}$: 2968, 1490, 1458, 1364, 1264, 1152, 872, 788, 694.

NMR ($CDCl_3$) $\delta$ : 0.85(3H, t, J=7.4Hz), 1.24(9H, s), 1.46-1.51(2H, m), 2.37(2H, t, J=7.1Hz), 3.07 (2H, dd, J=6.4Hz, 1.3Hz), 3.53(2H, s), 5.10 (2H, s), 5.63(1H, dd, J=15.9Hz, 1.3Hz), 6.04 (1H, dt, J=15.9Hz, 6.4Hz), 6.86(1H, dd, J=7.8Hz, 1.8Hz), 6.92(1H, d, J=7.4Hz), 7.20 (1H, s), 7.22(1H, t, J=7.8Hz), 7.43-7.44 (2H, m), 7.53-7.55(1H, m), 7.66(1H, br.s), 8.10(1H, s), 8.76(1H, s).

EXAMLE 49

Production of (E)-N-(6,6-dimethyl-2-hepten-4-ynyl)-N-methyl-3-[3-(3-pyrrolyl)benzyloxy]benzylamine:-

Methyl isocyanoacetate (280 microliters) and 400 microliters of 1,8-diazabicyclo[5.4.0]undeca-7-ene (DBU) were added to 20 ml of tetrahydrofuran, and with stirring at 45 to 50 $^{o}C$, a tetrahydrofuran solution (5 ml) of 500 mg of (E)-N-(6,6-dimethyl-2-hepten-4-ynyl)-N-methyl-3-(3-formylbenzyloxy)benzylamine was added, and the mixture was stirred at the above temperature for 5 hours. The reaction mixture was allowed to cool and neutralized with acetic acid. The solvent was evaporated under reduced pressure and ethyl acetate and water was added to the residue to extract it. The organic layer was separated, dried over anhydrous magnesium sulfate. The desiccant was separated by filtration, and the solvent was evaporated. Then, the

residue was purified by silica gel column chromatography [Wakogel C-200, 30 g, eluting solvent: hexane/ethyl acetate:5/1] to give 140 mg (yield 20 %) of (E)-N-(6,6-dimethyl-2-hepten-4-ynyl)-N-methyl-3-[3-(2,4-dimethoxycarbonyl-3-pyrrolyl)benzyloxy]benzylamine as a pale yellow oil.

The resulting pyrrolyl compound (32 mg) was added to a mixture of 2 g of potassium hydroxide and 6 ml of water. The mixture was heated under reflux for 6 hours with stirring. After cooling, ethyl ether was added to the reaction mixture to extract it. The extract was dried over anhydrous magnesium sulfate, and the solvent was evaporated. The residue was purified by silica gel column chromatography [Wakogel C-200, 30 g; eluting solvent: hexane/ethyl acetate=10/1 → 2/1] to give 12.4 mg (yield 50 %) of the captioned compound as a pale yellow oil.

IR $\nu_{max}^{neat}$ $cm^{-1}$: 3440, 2972, 2928, 2872, 1610, 1490, 1454, 1364, 1266, 1034, 778.

NMR ($CDCl_3$) $\delta$: 1.24(9H, s), 2.18(3H, s), 3.03 (2H, dd, J=6.6Hz, 1.5Hz), 3.47(2H, s), 5.08 (2H, s), 5.64(1H, dt, J=15.8Hz, 1.5Hz), 6.09 (1H, dt, J=15.8Hz, 6.6Hz), 6.54-6.57(1H, m), 6.82-6.92(3H, m), 6.99-7.01(1H, m), 7.10-7.13 (1H, m), 7.22(1H, t, J=7.6Hz), 7.23-7.28 (1H, m), 7.35(1H, t, J=7.4Hz), 7.49(1H, dt, J=7.4Hz, 1.7Hz), 7.59-7.61(1H, m), 8.20-8.40 (1H, br).

EXAMPLE 50

Production of (E)-N-(6,6-dimethyl-2-hepten-4-ynyl)-N-methyl-3-[3-(1,3,4-oxadiazol-2-yl)benzyloxy]-benzylamine:-

173 mg of (E)-N-(6,6-dimethyl-2-hepten-4-ynyl)-N-methyl-3-(3-methoxycarbonylbenzyloxy)benzylamine was dissolved in 3 ml of ethanol, and 100 mg of 98 % hydrazine hydrate was added. The mixture was heated under

reflux for 3 hours. After the reaction mixture was evaporated under reduced pressure, water and ethyl acetate were added to the residue to extract it. The organic layer was separated and dried over anhydrous sodium sulfate. The desiccant was separated by filtration and the solvent was evaporated. The residual carbohydrazide was added to 4 ml of trimethyl orthoformate and the mixture was heated under reflux for 8 hours. After evaporating the excess trimethyl orthoformate, the residue was extracted with a mixture of ethyl acetate and water. The organic layer was separated and dried over anhydrous sodium sulfate. The desiccant was separated by filtration and the solvent was evaporated under reduced pressure. The residue was purified by silica gel column chromatography [Wakogel C-200, 30 g; eluting solvent: hexane/ethyl acetate=2/1] and medium-pressure liquid chromatography [column: Lobar column, size A, Lichroprep Si 60 (E. Merck Co.); eluting solvent: hexane/ethyl acetate=3/1 → 2/1) to give 18 mg (yield 10 %) of the captioned compound as a colorless oil.

IR $\nu_{max}^{neat}$ $cm^{-1}$: 2974, 1590, 1491, 1455, 1368, 1266, 1152, 960, 726.

NMR ($CDCl_3$) $\delta$: 1.24(9H, s), 2.19(3H, s), 3.04 (1H, dd, J=6.5Hz, 1.5Hz), 3.47(2H, s), 5.15 (2H, s), 5.65(1H, dt, J=15.8Hz, 1.5Hz), 6.08 (1H, dt, J=15.8Hz, 6.5Hz), 6.85-6.90(1H, m), 6.90-6.94(1H, m), 7.00(1H, br.s), 7.24(1H, t, J=7.8Hz), 7.55(1H, t, J=7.6Hz), 7.63-7.67 (1H, m), 8.05(1H, dt, J=6.3Hz, 1.5Hz), 8.18 (1H, br.s), 8.49(1H, s).

EXAMPLE 51

Production of (E), (E)-N-(6,6-dimethyl-2-hepten-4-ynyl)-N-methyl-3-[2-[3-(3-thienyl)phenyl]-ethenyl]benzylamine hydrochloride:-

95 mg of (E)-3-[2-[3-(3-thienyl)phenyl]-ethenyl]benzyl chloride was dissolved in 2 ml of

dimethylformamide. To the solution were added 58 mg of (E)-N-methyl-6,6-dimethyl-2-hepten-4-ynylamine hydrochloride and 126 mg of potassium carbonate. The mixture was stirred overnight at room temperature. The reaction mixture was evaporated under reduced pressure, and ethyl ether and water were added to the residue. The organic layer was separated and dried over anhydrous sodium sulfate. The desiccant was separated by filtration, and the solvent was evaporated. The residue was purified by silica gel column chromatography [Wakogel C-200, 15 g; eluting solvent: hexane → hexane/ethyl acetate=10/1] to give 88 mg (yield 67 %) of a free base of the captioned compound as a colorless oil.

The free base obtained as above was treated with a hydrogen chloride-methanol solution and recrystallized from a mixture of chloroform and hexane to give the captioned hydrochloride, m. p. 132-133 °C.

IR $\nu_{max}^{KBr}$ $cm^{-1}$: 3430, 2968, 2482, 1464, 966, 777, 699.

NMR ($CDCl_3$) $\delta$ : 1.25(9H, s), 2.61(3H, s), 3.56-3.61 (2H, m), 4.05-4.10(2H, m), 5.84(1H, d, J=15.9Hz), 6.27(1H, dt, J=15.9Hz, 7.3Hz), 7.12-7.29(2H, m), 7.37-7.53(8H, m), 7.58 (1H, dt, J=7.4Hz, 1.9Hz), 7.74(1H, br.s), 7.79(1H, br.s).

Compounds of Examples 52 to 66 were obtained by the same method as in Example 51 except using the corresponding benzyl chloride, bromide or methanesulfonate derivatives and 2-hepten-4-ynylamine hydrochlorides instead of the starting compound (E)-3-[2-[3-(3-thienyl)-phenyl]ethenyl]benzyl chloride and (E)-N-methyl-6,6-dimethyl-2-hepten-4-ynylamine hydrochloride.

Example 52

(E),(E)-N-(6,6-dimethyl-2-hepten-4-ynyl)-N-ethyl-3-[2-[3-(3-thienyl)phenyl]ethenyl]benzylamine hydrochloride:-

m.p. 174-176 °C.

IR $\nu_{max}^{KBr}$ $cm^{-1}$: 3436, 2968, 966, 777, 699.

NMR ($CDCl_3$) $\delta$ : 1.25(9H, s), 1.40-1.46(3H, m), 2.98-3.01(2H, m), 3.57-3.62(2H, m), 4.07-4.09 (2H, m), 5.83(1H, d, J=15.6Hz), 6.25(1H, dt, J=15.6Hz, 7.3Hz), 7.12-7.25(2H, m), 7.30-7.53 (9H, m), 7.56(1H, d, J=7.5Hz), 7.74-7.75(1H, m), 7.86(1H, br.s).

Example 53

(E),(E)-N-(6,6-dimethyl-2-hepten-4-ynyl)-N-propyl-3-[2-[3-(3-thienyl)phenyl]ethenyl]benzylamine:-

IR $\nu_{max}^{neat}$ $cm^{-1}$: 2968, 2806, 1605, 963, 774, 696.

NMR ($CDCl_3$) $\delta$ : 0.89(3H, t, J=7.2Hz), 1.24(9H, s), 1.47-1.57(2H, m), 2.41(2H, t, J=7.2Hz), 3.11 (2H, d, J=6.4Hz), 3.58(2H, s), 5.66(1H, d, J=15.8Hz), 6.11(1H, dt, J=15.8Hz, 6.4Hz), 7.16(2H, s), 7.22-7.26(1H, m), 7.30(1H, t, J=7.2Hz), 7.36-7.51(8H, m), 7.73-7.74(1H, m).

Example 54

(E),(E)-N-ethyl-N-(6-methoxy-6-methyl-2-hepten-4-ynyl)-3-[2-[3-(3-thienyl)phenyl]ethenyl]benzylamine:-

IR $\nu_{max}^{neat}$ $cm^{-1}$: 2980, 2932, 2818, 1605, 1251, 1170, 1149, 1074, 774, 699.

NMR ($CDCl_3$) $\delta$ : 1.08(3H, t, J=7.1Hz), 1.46(6H, s), 2.55(2H, q, J=7.1Hz), 3.15(2H, dd, J=6.4Hz, 1.2Hz), 3.36(3H, s), 3.59(2H, s), 5.71(1H, dt, J=15.9Hz, 1.2Hz), 6.20(1H, dt, J=15.9Hz, 6.4Hz), 7.16(2H, s), 7.20-7.25(2H, m), 7.31 (1H, t, J=7.5Hz), 7.35-7.55(7H, m), 7.74(1H, t, J=2.3Hz).

Example 55

(E),(E)-N-(6,6-dimethyl-2-hepten-4-ynyl)-N-methyl-3-[2-[3-(1-pyrrolyl)phenyl]ethenyl]benzylamine:-

IR $\nu_{max}^{neat}$ $cm^{-1}$: 2968, 1605, 1506, 1341, 1071, 963, 756, 723, 696.

NMR ($CDCl_3$) δ : 1.24(9H, s), 2.22(3H, s), 3.08(2H, dd, J=6.6Hz, 1.5Hz), 3.51(2H, s), 5.67(1H, dt, J=15.9Hz, 1.5Hz), 6.12(1H, dt, J=15.9Hz, 6.6Hz), 6.37(2H, t, J=2.1Hz), 7.13(2H, t, J=2.1Hz), 7.15(2H, s), 7.20-7.34(3H, m), 7.37-7.44(3H, m), 7.49-7.54(2H, m).

Example 56

(E),(E)-N-(6,6-dimethyl-2-hepten-4-ynyl)-N-ethyl-3-[2-[3-(1-pyrrolyl)phenyl]ethenyl]benzylamine:-

IR $\nu_{max}^{neat}$ $cm^{-1}$: 2968, 2926, 1608, 1587, 1506, 1341, 1071, 963, 723.

NMR ($CDCl_3$) δ : 1.07(3H, t, J=6.8Hz), 1.24(9H, s), 2.54(2H, q, J=6.8Hz), 3.14(2H, dd, J=6.5Hz, 1.5Hz), 3.60(2H, s), 5.67(1H, dt, J=15.9Hz, 1.5Hz), 6.10(1H, dt, J=15.9Hz, 6.5Hz), 6.37 (2H, t, J=2.2Hz), 7.13(2H, t, J=2.2Hz), 7.12-7.16(2H, m), 7.25-7.34(3H, m), 7.38-7.44 (3H, m), 7.50(1H, s), 7.52-7.54(1H, m).

Example 57

(E),(E)-N-ethyl-N-(6-methoxy-6-methyl-2-hepten-4-ynyl)-3-[2-[3-(1-pyrrolyl)phenyl]ethenyl]-benzylamine:-

IR $\nu_{max}^{neat}$ $cm^{-1}$: 1605, 1506, 1341, 1170, 1149, 1074, 960, 789, 723, 696.

NMR ($CDCl_3$) δ: 1.09(3H, t, J=6.9Hz), 1.46(6H, s), 2.56(2H, q, J=6.9Hz), 3.15(2H, dd, J=6.5Hz, 1.5Hz), 3.35(3H, s), 3.61(2H, s), 5.73(1H, dt, J=15.9Hz, 1.5Hz), 6.20(1H, dt, J=15.9Hz, 6.5Hz), 6.37(2H, t, J=2.7Hz), 7.12-7.16 (4H, m), 7.25-7.35(3H, m), 7.38-7.45(3H, m), 7.50-7.54(2H, m).

Example 58

(E),(E)-N-(6,6-dimethyl-2-hepten-4-ynyl)-N-ethyl-3-[2-[3-(3-pyridyl)phenyl]ethenyl]benzylamine:-

IR $\nu_{max}^{neat}$ $cm^{-1}$: 3016, 2974, 1605, 1473, 1365, 1266, 1215, 963, 759, 711.

NMR ($CDCl_3$) δ : 1.07(3H, t, J=7.1Hz), 1.24(9H, s), 2.55(2H, q, J=7.1Hz), 3.13(1H, dd, J=6.7Hz, 1.5Hz), 3.60(2H, s), 5.70(1H, dt, J=15.9Hz, 1.5Hz), 6.10(1H, dt, J=15.9Hz, 6.7Hz), 7.19 (2H, s), 7.23-7.59(8H, m), 7.71(1H, br.s), 7.89-7.94(1H, m), 8.61(1H, dd, J=4.8Hz, 1.6Hz), 8.89(1H, d, J=1.6Hz).

Example 59

(E),(E)-N-(6,6-dimethyl-2-hepten-4-ynyl)-N-ethyl-3-[2-[3-(5-oxazolyl)phenyl]ethenyl]benzylamine:-

IR $\nu_{max}^{neat}$ $cm^{-1}$: 2968, 2926, 1263, 1107, 960, 798, 756, 696.

NMR ($CDCl_3$) δ: 1.07(3H, t, J=7.0Hz), 1.24(9H, s), 2.51-2.58(2H, m), 3.12-3.14(2H, m), 3.58-3.62 (2H, m), 5.68(1H, d, J=15.7Hz), 6.10(1H, dt, J=15.7Hz, 6.1Hz), 7.15-7.57(10H, m), 7.81 (1H, t, J=1.5Hz), 7.94(1H, s).

Example 60

(E),(E)-N-ethyl-N-(6-methoxy-6-methyl-2-hepten-4-ynyl)-3-[2-[3-(5-oxazolyl)phenyl]ethenyl]-benzylamine:-

IR $\nu_{max}^{neat}$ $cm^{-1}$: 3128, 2984, 2936, 2820, 1604, 1582, 1506, 968.

NMR ($CDCl_3$) δ : 1.08(3H, t, J=7.0Hz), 1.46(6H, s), 2.56(2H, q, J=7.0Hz), 3.15(2H, d, J=6.3Hz), 3.36(3H, s), 3.60(2H, s), 5.71(1H, dt, J=15.8Hz, 1.5Hz), 6.21(1H, dt, J=15.8Hz, 6.3Hz), 7.13(1H, d, J=16.1Hz), 7.14(1H, d, J=16.1Hz), 7.32(1H, t, J=7.5Hz), 7.41(1H, s), 7.42-7.45(2H, m), 7.49-7.50(2H, m), 7.51-7.52 (1H, m), 7.55(1H, dt, J=7.5Hz, 1.6Hz), 7.81 (1H, t, J=1.6Hz), 7.94(1H, s).

Example 61

(E),(E)-N-(6,6-dimethyl-2-hepten-4-ynyl)-N-propyl-3-[2-[3-(1-pyrrolyl)phenyl]ethenyl]benzylamine:-

IR $\nu_{max}^{neat}$ $cm^{-1}$: 2968, 1608, 1506, 1341, 1071, 960, 723, 696.

NMR ($CDCl_3$) $\delta$ : 0.89(3H, t, J=7.3Hz), 1.24(9H, s), 1.48-1.60(2H, m), 2.42(2H, t, J=7.3Hz), 3.12 (2H, d, J=6.2Hz), 3.58(2H, s), 5.66(1H, d, J=15.8Hz), 6.10(1H, dt, J=15.8Hz, 6.2Hz), 6.37(2H, t, J=2.2Hz), 7.14(2H, t, J=2.2Hz), 7.13-7.15(2H, m), 7.22-7.34(3H, m), 7.38-7.42 (3H, m), 7.48-7.52(1H, m), 7.52-7.54(1H, m).

Example 62

(E),(E)-N-(6,6-dimethyl-2-hepten-4-ynyl)-N-ethyl-3-[2-[3-(1-imidazolyl)phenyl]ethenyl]benzylamine:-

IR $\nu_{max}^{neat}$ $cm^{-1}$: 2972, 2864, 1608, 1588, 1504, 1308, 1060, 962.

NMR ($CDCl_3$) $\delta$: 1.10(3H, t, J=7.2Hz), 1.25(9H, s), 2.57(2H, q, J=7.2Hz), 3.18(2H, dd, J=6.3Hz, 1.5Hz), 3.61(2H, s), 5.64(1H, dt, J=15.9Hz, 1.5Hz), 6.06(1H, dt, J=15.9Hz, 6.3Hz), 7.14 (1H, d, J=16.0Hz), 7.19(1H, d, J=16.0Hz), 7.21-7.58(9H, m), 7.67-7.74(1H, m), 7.91 (1H, br.s).

Example 63

(E),(E)-N-(6,6-dimethyl-2-hepten-4-ynyl)-N-methyl-2-[2-[3-(3-thienyl)phenyl]ethenyl]-4-pyridyl-methylamine:-

IR $\nu_{max}^{neat}$ $cm^{-1}$: 1602, 1479, 1458, 1365, 1263, 1203, 975, 852, 774.

NMR ($CDCl_3$) $\delta$ : 1.24(9H, s), 2.34(3H, s), 3.10(2H, dd, J=7.0Hz, 1.6Hz), 3.52(2H, s), 5.68(1H, dt, J=15.8Hz, 1.6Hz), 6.10(1H, dt, J=15.8Hz, 7.0Hz), 7.14(1H, d, J=5.6Hz), 7.23(1H, d, J=16.4Hz), 7.41-7.48(4H, m), 7.49-7.55(3H, m), 7.68(1H, d, J=16.4Hz), 7.81(1H, t, J=1.8Hz), 8.54(1H, d, J=4.7Hz).

Example 64

(E)-N-(6,6-dimethyl-2-hepten-4-ynyl)-N-methyl-3-[2-[3-(3-thienyl)phenyl]ethyl]benzylamine:-

IR $\nu_{max}^{neat}$ $cm^{-1}$: 2968, 1458, 1365, 963, 774, 699.

NMR ($CDCl_3$) $\delta$ : 1.24(9H, s), 2.16(3H, s), 2.95 (4H, s), 3.02(2H, dd, J=6.6Hz, 1.5Hz), 3.45 (2H, s), 5.63(1H, dt, J=15.9Hz, 1.5Hz), 6.08 (1H, dt, J=15.9Hz, 6.6Hz), 7.07-7.25(5H, m), 7.27-7.52(6H, m).

Example 65

(E)-N-(6,6-dimethyl-2-hepten-4-ynyl)-N-ethyl-3-[2-[3-(1-pyrrolyl)phenyl]ethyl]benzylamine:-

IR $\nu_{max}^{neat}$ $cm^{-1}$: 2968, 2928, 2864, 2796, 1610, 1594, 1506, 726.

NMR ($CDCl_3$) $\delta$ : 1.03(3H, t, J=7.1Hz), 1.24(9H, s), 2.48(2H, q, J=7.1Hz), 2.95(4H, s), 3.06(2H, dd, J=6.2Hz, 2.3Hz), 3.53(2H, s), 5.62(1H, dt, J=15.8Hz, 2.3Hz), 6.06(1H, dt, J=15.8Hz, 6.2Hz), 6.33(2H, t, J=2.2Hz), 7.04(2H, t, J=2.2Hz), 7.04-7.08(2H, m), 7.13-7.17(3H, m), 7.19-7.23(2H, m), 7.31(1H, t, J=7.8Hz).

Example 66

(E)-N-(6,6-dimethyl-2-hepten-4-ynyl)-N-ethyl-3-[2-[3-(3-pyridyl)phenyl]ethyl]benzylamine:-

IR $\nu_{max}^{neat}$ $cm^{-1}$: 2968, 2932, 2866, 1473, 1458, 1365, 789, 705.

NMR ($CDCl_3$) $\delta$ : 1.02(3H, t, J=7.3Hz), 1.24(9H, s), 2.50(2H, q, J=7.3Hz), 2.96-2.99(4H, m), 3.01 (1H, d, J=6.4Hz), 3.54(1H, s), 5.63(1H, d, J=16.2Hz), 6.08(1H, dd, J=16.2Hz, 6.4Hz), 7.06-7.11(1H, m), 7.15-7.27(4H, m), 7.32-7.42 (4H, m), 7.83(1H, dt, J=8.4Hz, 1.8Hz), 8.60 (1H, br.s), 8.80(1H, br.s).

EXAMPLE 67

Production of (E),(E)-N-(6,6-dimethyl-2-hepten-4-ynyl)-N-ethyl-5-[2-[3-(3-thienyl)phenyl]ethenyl]-furfurylamine:-

18 mg of (E)-N-(6,6-dimethyl-2-hepten-4-ynyl)-N-ethyl-5-formylfurfurylamine and 19 mg of dimethyl-3-(3-thienyl)benzylphosphonate [synthesized by condensation between 3-(3-thienyl)benzyl bromide and trimethyl phosphite] were dissolved in dimethylformamide, and 2.6 mg of 60 % oily sodium hydride was added. The mixture was stirred overnight at room temperature. The reaction mixture was concentrated under reduced pressure. The residue was purified by preparative thin-layer chromatography [thin layer plate: Kieselgel $60F_{254}$, Art. 5715 (E. Merck Co.); developing solvent: hexane/ethyl acetate=3/1] to give 15 mg (yield 55 %) of the captioned compound as a pale yellow oil.

IR $\nu_{max}^{neat}$ $cm^{-1}$: 2968, 1602, 1458, 1365, 1266, 1020, 960, 774.

NMR ($CDCl_3$) $\delta$ : 1.16(3H, t, J=7.0Hz), 1.24(9H, s), 2.58-2.68(2H, m), 3.25(2H, d, J=6.8Hz), 3.77 (2H, s), 5.73(1H, d, J=15.9Hz), 6.13(1H, dt, J=15.9Hz, 6.8Hz), 6.25-6.35(2H, m), 6.90(1H, d, J=16.4Hz), 7.04(1H, d, J=16.4Hz), 7.32-7.52 (6H, m), 7.67(1H, br.s).

Compounds of Examples 68 and 69 were obtained by performing the same reaction as in Example 67 except that the corresponding formyl derivatives were used instead of the starting (E)-N-(6,6-dimethyl-2-hepten-4-ynyl)-N-ethyl-5-formylfurfurylamine.

<u>Example 68</u>

(E),(E)-N-(6,6-dimethyl-2-hepten-4-ynyl)-N-methyl-4-[2-[3-(3-thienyl)phenyl]ethenyl]-2-pyridylmethylamine:-

IR $\nu_{max}^{neat}$ $cm^{-1}$: 2968, 2868, 1602, 1450, 1266, 966, 777.

NMR ($CDCl_3$) δ : 1.24(9H, s), 2.35(3H, s), 3.20-3.27 (2H, m), 3.74-3.79(2H, m), 5.72(1H, d, J=15.7Hz), 6.18(1H, dt, J=15.7Hz, 6.6Hz), 7.19(1H, d, J=16.4Hz), 7.19-7.63(9H, m), 7.76 (1H, m), 8.52(1H, d, J=5.3Hz).

Example 69

(E),(E)-N-(6,6-dimethyl-2-hepten-4-ynyl)-N-ethyl-3-[2-[3-(3-thienyl)phenyl]ethenyl]-5-isoxazolyl-methylamine:-

IR $\nu_{max}^{neat}$ $cm^{-1}$: 2968, 2926, 1440, 1365, 963, 852, 774.

NMR ($CDCl_3$) δ : 0.88(3H, t, J=7.1Hz), 1.25(9H, s), 2.53-2.68(2H, m), 3.17-3.28(2H, m), 3.81 (1H, s), 5.71(1H, d, J=15.9Hz), 6.08(1H, dt, J=15.9Hz, 6.5Hz), 6.40-6.48(1H, m), 7.15-7.25 (2H, m), 7.38-7.53(5H, m), 7.56(1H, dt, J=7.1Hz, 2.1Hz), 7.73(1H, s).

EXAMPLE 70

Production of (E)-N-(6,6-dimethyl-2-octen-4-ynyl)]-N-ethyl-3-[3-(3-thienyl)benzyloxy]benzylamine:-

50 mg of N-ethyl-3-[3-(3-thienyl)benzyloxy]-benzylamine hydrochloride was dissolved in 1.5 ml of dimethylformamide, and 30 mg of 1-bromo-6,6-dimethyl-2-octen-4-yne (a mixture of the E-form and the Z-form in a ratio of about 4:1) and 65 mg of sodium carbonate were added. The mixture was stirred overnight at room temperature. The reaction mixture was diluted with water and then extracted with ethyl ether. The extract was washed with a saturated aqueous solution of sodium chloride and dried over anhydrous sodium sulfate. The desiccant was separated by filtration, and then the solvent was evaporated. The residue was purified by medium-pressure liquid chromatography [column: Lobar column, size A, Lichroprep Si 60 (E. Merck Co.); eluting solvent: hexane/ethyl acetate=7/1] to give 39 mg (yield 63 %) of the captioned compound as a colorless oil.

IR $\nu_{max}^{neat}$ $cm^{-1}$: 2968, 2926, 2800, 1584, 1491, 1458, 1260, 774.

NMR ($CDCl_3$) $\delta$ : 0.97(3H, t, J=7.3Hz), 1.00-1.10 (3H, m), 1.18(6H, s), 1.44(2H, q, J=7.3Hz), 2.45-2.60(2H, m), 3.05-3.15(2H, m), 3.50-3.60 (2H, m), 5.11(2H, s), 5.65(1H, d, J=15.9Hz), 6.07(1H, dt, J=15.9Hz, 6.4Hz), 6.85-6.90 (1H, m), 6.90-6.95(1H, m), 7.01-7.06(1H, m), 7.22(1H, t, J=8.0Hz), 7.35-7.45(4H, m), 7.47 (1H, dd, J=2.3Hz, 1.8Hz), 7.55(1H, dt, J=7.0Hz, 1.8Hz), 7.66-7.69(1H, m).

EXAMPLE 71

Production of (E)-N-(6,6-dimethyl-2-hepten-4-ynyl)-N-methyl-4-hydroxy-3-[3-(3-thienyl)benzyloxy]-benzylamine hydrochloride:-

A dimethylformamide solution (1 ml) of 32 mg of 3-(3-thienyl)benzyl bromide was added to a tetrahydrofuran solution (1.5 ml) of phenolate prepared from 40 mg of (E)-N-(6,6-dimethyl-2-hepten-4-ynyl)-N-methyl-3-hydroxy-4-methoxymethyloxybenzylamine and 5 mg of 60 % oily sodium hydride. The mixture was stirred at room temperature for 2 hours. Ethyl ether was added to the reaction mixture, and the insoluble inorganic salts were separated by filtration. The filtrate was concentrated under reduced pressure. The residue was dissolved in a mixture of 1 ml of 10 % hydrogen chloride-methanol and 1 ml of tetrahydrofuran, and the solution was left to stand at room temperature for 2 hours. The reaction mixture was concentrated under reduced pressure, and a 5 % aqueous solution of sodium hydrogen carbonate and ethyl ether were added to the residue to extract it. The organic layer was separated and dried over anhydrous magnesium sulfate. The desiccant was separated by filtration and the solvent was evaporated. The residue was purified by silica gel column chromatography [Wakogel C-200, 5 g; eluting solvent: hexane/ethyl acetate=3/1] to

give 31 mg (yield 56 %) of a free base of the captioned compound as a colorless oil. The free base was treated with a hydrogen chloride-methanol solution and recrystallized from a mixture of ethyl ether and isopropyl ether to give the captioned hydrochloride, m.p. 88-90 °C.

IR $\nu_{max}^{KBr}$ $cm^{-1}$: 2968, 1521, 1464, 1446, 1281, 777.

NMR ($CDCl_3$) $\delta$ : 1.25(3H, s), 3.39-3.54(1H, m), 3.59-3.74(1H, m), 3.83-4.00(1H, m), 4.01-4.20 (1H, m), 5.31(2H, s), 5.81(1H, d, J=15.6Hz), 5.91(1H, br.s), 6.21(1H, dt, J=15.6Hz, 7.7Hz), 6.80(1H, d, J=7.8Hz), 6.91(1H, d, J=7.8Hz), 7.38-7.48(4H, m), 7.50-7.55(1H, m), 7.56-7.62(1H, m), 7.75(1H, s), 7.82(1H, s).

Compounds of Examples 72 and 73 were obtained by performing the same reaction as in Example 71 except that instead of the starting compounds (E)-N-(6,6-dimethyl-2-hepten-4-ynyl)-N-methyl-3-hydroxy-4-methoxymethyloxybenzylamine and/or 3-(3-thienyl)benzyl bromide, the corresponding methoxymethyloxybenzylamine derivatives and/or 3-(5-oxazolyl)benzylmethanesulfonate were used.

Example 72

(E)-N-(6,6-dimethyl-2-hepten-4-ynyl)-N-ethyl-2-hydroxy-3-[3-(5-oxazolyl)benzyloxy]benzylamine:-

IR $\nu_{max}^{neat}$ $cm^{-1}$: 2968, 1476, 1365, 1245, 1107, 1035, 954, 789, 750, 693.

NMR ($CDCl_3$) $\delta$ : 1.13(3H, t, J=7.0Hz) 1.23(9H, s), 2.63(2H, q, J=7.0Hz), 3.20(2H, d, J=7.0Hz), 3.78(2H, s), 5.18(2H, s), 5.62(1H, d, J=15.7Hz), 6.08(1H, dt, J=15.7Hz, 7.0Hz), 6.59-6.64(1H, m), 6.67(1H, t, J=8.0Hz), 6.84 (1H, dd, J=8.0Hz, 1.8Hz), 7.37(1H, s), 7.38-7.50(2H, m), 7.60(1H, dt, J=7.0Hz, 1.8Hz), 7.77(1H, s), 7.91(1H, s).

Example 73

(E)-N-(6,6-dimethyl-2-hepten-4-ynyl)-N-methyl-4-hydroxy-3-[3-(5-oxazolyl)benzyloxy]benzylamine:-

IR $\nu_{max}^{neat}$ $cm^{-1}$: 2968, 1518, 1461, 1365, 1275, 1200, 1119, 795, 759.

NMR ($CDCl_3$) $\delta$: 1.24(9H, s), 2.19(3H, s), 2.96-3.12 (2H, m), 3.44(2H, s), 5.16(2H, s), 5.65(1H, d, J=16.1Hz), 6.08(1H, dt, J=16.1Hz, 6.7Hz), 6.80(1H, dt, J=8.0Hz, 1.7Hz), 6.89(1H, d, J=8.0Hz), 7.02(1H, br.s), 7.39(1H, s), 7.39-7.42(1H, m), 7.48(1H, t, J=7.6Hz), 7.65 (1H, dt, J=7.6Hz, 1.6Hz), 7.73(1H, s), 7.93 (1H, s).

EXAMPLE 74

Production of (E)-N-(6,6-dimethyl-2-hepten-4-ynyl)-N-ethyl-3-[3-(3-thienyl)benzylamino]benzylamine:-

90 mg of (E)-N-(6,6-dimethyl-2-hepten-4-ynyl)-N-ethyl-3-aminobenzylamine and 63 mg of 3-(3-thienyl)-benzaldehyde were dissolved in 2.5 ml of anhydrous methanol, and the solution was stirred overnight at room temperature in the presence of molecular sieves 3A. The molecular sieves were separated by filtration from the reaction mixture. Then, 12.5 mg of sodium borohydride was added, and the mixture was further stirred for 30 minutes at room temperature. The solvent was evaporated under reduced pressure. The residue was dissolved in a mixture of methylene chloride and water. The organic layer was separated, and dried over anhydrous magnesium sulfate. The desiccant was separated by filtration, and the solvent was evaporated. The residue was purified by silica gel column chromatography [Wakogel C-200, 5 g; eluting solvent: hexane/ethyl acetate=10/1 → 5/1] to give 80 mg (yield 55 %) of the captioned compound as a pale yellow oil.

IR $\nu_{max}^{neat}$ $cm^{-1}$: 2968, 2926, 1608, 1491, 774.

NMR ($CDCl_3$) $\delta$: 1.01(3H, t, J=7.1Hz), 1.24(9H, s), 2.49(2H, q, J=7.1Hz), 3.08(2H, dd, J=6.4Hz, 1.5Hz), 3.48(2H, s), 4.37(2H, s), 5.63(1H, dt, J=15.9Hz, 1.5Hz), 6.06(1H, dt, J=15.9Hz,

6.4Hz), 6.51-6.56(1H, m), 6.66-6.69(1H, m), 7.11(1H, t, J=8.0Hz), 7.31(1H, dt, J=7.8Hz, 1.7Hz), 7.35-7.40(3H, m), 7.45(1H, t, J=2.2Hz), 7.51(1H, dt, J=7.8Hz, 1.7Hz), 7.61(1H, br.s).

Compounds of Examples 75 to 80 were obtained by performing the same reaction as in Example 74 except that instead of the starting compound (E)-N-(6,6-dimethyl-2-hepten-4-ynyl)-N-ethyl-3-aminobenzylamine and/or 3-(3-thienyl)benzaldehyde, the corresponding 3-aminobenzylamine derivatives and/or 3-substituted benzaldehyde derivatives were used.

Example 75

(E)-N-(6,6-dimethyl-2-hepten-4-ynyl)-N-methyl-3-[3-(3-thienyl)benzylamino]benzylamine:-

IR $\nu_{max}^{neat}$ $cm^{-1}$: 2968, 1608, 1491, 1458, 1365, 774.

NMR ($CDCl_3$) $\delta$ : 1.24(9H, s), 2.18(3H, s), 3.02(2H, dd, J=6.5Hz, 1.4Hz), 3.42(2H, s), 4.37(2H, s), 5.63(1H, dt, J=15.8Hz, 1.4Hz), 6.07(1H, dt, J=15.8Hz, 6.5Hz), 6.52-6.56(1H, m), 6.65-6.68 (2H, m), 7.11(1H, t, J=7.9Hz), 7.30(1H, dt, J=7.5Hz, 1.4Hz), 7.35-7.40(3H, m), 7.45(1H, t, J=2.1Hz), 7.51(1H, dt, J=7.5Hz, 1.7Hz), 7.61 (1H, br.s).

Example 76

(E)-N-(6,6-dimethyl-2-hepten-4-ynyl)-N-ethyl-3-[3-(1-pyrrolyl)benzylamino]benzylamine:-

IR $\nu_{max}^{neat}$ $cm^{-1}$: 2968, 1608, 1506, 1338, 1071, 783, 726.

NMR ($CDCl_3$) $\delta$ : 1.00(3H, t, J=7.1Hz), 1.24(9H, s), 2.47(2H, q, J=7.1Hz), 3.07(2H, dd, J=6.5Hz, 1.5Hz), 3.48(2H, s), 4.39(2H, s), 5.62(1H, dt, J=15.8Hz, 1.5Hz), 6.05(1H, dt, J=15.8Hz, 6.5Hz), 6.33(2H, t, J=2.2Hz), 6.49-6.53(1H, m), 6.66-6.69(2H, m), 7.08(2H, t, J=2.2Hz), 7.10 (1H, t, J=8.1Hz), 7.24-7.32(2H, m), 7.36-7.42 (2H, m).

Example 77

(E)-N-(6,6-dimethyl-2-hepten-4-ynyl)-N-methyl-3-[3-(5-oxazolyl)benzylamino]benzylamine:-

IR $\nu_{max}^{neat}$ $cm^{-1}$: 2974, 1611, 1506, 1494, 1458, 1107, 954, 696.

NMR ($CDCl_3$) $\delta$: 1.20(9H, s), 2.14(3H, s), 3.05(2H, dd, J=6.6Hz, 1.5Hz), 3.42(2H, s), 4.38(2H, s), 5.59(1H, dt, J=15.9Hz, 1.5Hz), 6.03(1H, dt, J=15.9Hz, 6.6Hz), 6.52(1H, dt, J=8.0Hz, 1.0Hz), 6.67-6.69(2H, m), 7.11(1H, t, J=8.0Hz), 7.35-7.42(3H, m), 7.57(1H, dt, J=7.4Hz, 1.6Hz), 7.68(1H, br.s), 7.91(1H, s).

Example 78

(E)-N-(6,6-dimethyl-2-hepten-4-ynyl)-N-ethyl-3-[3-(5-oxazolyl)benzylamino]benzylamine:-

IR $\nu_{max}^{neat}$ $cm^{-1}$: 2968, 2926, 1611, 1506, 1494, 1476, 1266, 1107, 954, 789, 759, 696.

NMR ($CDCl_3$) $\delta$: 1.00(3H, t, J=7.1Hz), 1.24(9H, s), 2.48(2H, q, J=7.1Hz), 3.07(2H, dd, J=6.4Hz, 1.5Hz), 3.48(2H, s), 4.38(2H, s), 5.62(1H, dt, J=15.9Hz, 1.5Hz), 6.05(1H, dt, J=15.9Hz, 6.4Hz), 6.50-6.54(1H, m), 6.67-6.69(2H, m), 7.11(1H, t, J=7.9Hz), 7.36(1H, s), 7.37-7.44(2H, m), 7.57 (1H, dt, J=7.2Hz, 1.7Hz), 7.68(1H, br.s), 7.91 (1H, s).

Example 79

(E)-N-ethyl-N-(6-methoxy-6-methyl-2-hepten-4-ynyl)-3-[3-(3-thienyl)benzylamino]benzylamine:-

IR $\nu_{max}^{CHCl_3}$ $cm^{-1}$: 3412, 3100, 2980, 2932, 2818, 1608, 1491, 1473, 1365.

NMR ($CDCl_3$) $\delta$: 1.04(3H, t, J=7.0Hz), 1.46(6H, s), 2.50(2H, q, J=7.0Hz), 3.13(2H, d, J=6.0Hz), 3.52(2H, s), 4.37(2H, s), 5.67(1H, d, J=16.2Hz), 6.16(1H, dt, J=16.2Hz, 6.0Hz), 6.52-6.57(1H, m), 6.66-6.72(2H, m), 7.12(1H, t, J=7.8Hz), 7.29-7.40(4H, m), 7.45(1H, t, J=2.2Hz), 7.51 (1H, dt, J=7.5Hz, 2.1Hz), 7.61(1H, br.s).

Example 80

(E)-N-(6,6-dimethyl-2-hepten-4-ynyl)-N-propyl-2-[3-(3-thienyl)benzylamino]-4-pyridylmethylamine:-

IR $\nu_{max}^{neat}$ $cm^{-1}$: 2968, 1611, 1569, 1515, 1464, 1368, 1269, 774.

NMR ($CDCl_3$) $\delta$ : 0.81(3H, t, J=7.3Hz), 1.24(9H, s), 1.40(2H, sex, J=7.3Hz), 2.32(2H, t, J=7.3Hz), 3.03(2H, dd, J=6.4Hz, 1.4Hz), 3.42(3H, s), 4.53(2H, s), 5.59(1H, dt, J=15.9Hz, 1.4Hz), 5.99(1H, dt, J=15.9Hz, 6.4Hz), 6.48(1H, d, J=1.6Hz), 6.58(1H, dd, J=5.4Hz, 1.6Hz), 7.27-7.31(1H, m), 7.34-7.40(3H, m), 7.45 (1H, t, J=1.8Hz), 7.50(1H, dt, J=7.6Hz, 1.7Hz), 7.59(1H, br.s), 7.96(1H, dt, J=7.6Hz, 1.7Hz).

EXAMPLE 81

Production of (E)-N-(6,6-dimethyl-2-hepten-4-ynyl)-N-ethyl-3-[N'-methyl-3-(3-thienyl)benzylamino]-benzylamine:-

100 mg of the (E)-N-(6,6-dimethyl-2-hepten-4-ynyl)-N-ethyl-3-[3-(3-thienyl)benzylamino]benzylamine obtained in Example 74 was dissolved in 3 ml of acetonitrile. 0.1 ml of a 35 % aqueous solution of formaldehyde and 22.7 mg of sodium cyanoborohydride were added, and the mixture was stirred at room temperature for 30 minutes. The reaction mixture was evaporated under reduced pressure. The residue was dissolved in a mixture of ethyl acetate and water. The organic layer was separated, and dried over anhydrous magnesium sulfate. The desiccant was separated by filtration, and the solvent was evaporated. The residue was purified by silica gel column chromatography [Wakogel C-200, 5 g; eluting solvent: hexane/ethyl acetate=10/1 → 6/1] to give 55 mg (yield 53 %) of the captioned compound as a pale yellow crystalline solid, m.p. 51-52 °C.

IR $\nu_{max}^{neat}$ $cm^{-1}$: 2974, 2926, 1605, 1500, 1368, 1218, 762.

NMR ($CDCl_3$) δ: 0.99(3H, t, J=7.0Hz), 1.23(9H, s), 2.47(2H, q, J=7.0Hz), 3.02(3H, s), 3.06(1H, dd, J=6.3Hz, 1.4Hz), 3.51(2H, s), 4.56(2H, s), 5.61(1H, dt, J=15.8Hz, 1.4Hz), 6.05(1H, dt, J=15.8Hz, 6.3Hz), 6.63-6.71(2H, m), 6.78-6.79 (1H, m), 7.12-7.19(2H, m), 7.31-7.38(3H, m), 7.41(1H, dd, J=2.7Hz, 1.5Hz), 7.46-7.49(2H, m).

EXAMPLE 82

Production of (E),(E)-N-(6,6-dimethyl-2-hepten-4-ynyl)-N-ethyl-5-[2-[3-(3-thienyl)phenyl]-ethenyl]-(1,3,4-oxadiazol-2-yl)methylamine:-

57 mg of (E)-N-(6,6-dimethyl-2-hepten-4-ynyl)-N-ethylglycylhydrazide [synthesized by condensing (E)-N-ethyl-6,6-dimethyl-2-hepten-4-ynylamine and methyl bromoacetate in the presence of sodium hydrogen carbonate, and then reacting the product with hydrazine] and 20 mg of sodium hydrogen carbonate were added to 1 ml of dioxane. To the mixture was added a dioxane solution (1 ml) of 3-(3-thienyl)cinnamoyl chloride which had been prepared from 50 mg of 3-(3-thienyl)cinnamic acid [synthesized by condensing 3-(3-thienyl)benzaldehyde and malonic acid under heat in the presence of piperidine/pyridine] and 0.3 ml of of thionyl chloride. The mixture was stirred at room temperature for 30 minutes. The inorganic salts were separated by filtration, and the solvent evaporated under reduced pressure. The residue was dissolved in 0.8 ml of phosphorus oxychloride, and the solution stirred at 65 °C for 16 hours. The reaction mixture was poured into ice water, and sodium hydrogen carbonate was added to neutralize it. The solution was then extracted with ethyl acetate. The extract was dried over anhydrous magnesium sulfate, and the solvent was evaporated. The residue was purified by silica gel column chromatography [Wakogel C-200, 10 g; eluting solvent: hexane/ethyl acetate=3/1] to give 37 mg (yield 36 %) of the captioned compound as a colorless oil.

IR $\nu_{max}^{neat}$ $cm^{-1}$: 2972, 1648, 1364, 1266, 964, 854, 778.

NMR ($CDCl_3$) $\delta$: 1.13(3H, t, J=7.1Hz), 1.24(9H, s), 2.65(2H, q, J=7.1Hz), 3.26(2H, dd, J=6.8Hz, 1.5Hz), 3.95(2H, s), 5.73(1H, dt, J=15.9Hz, 1.5Hz), 6.07(1H, dt, J=15.9Hz, 6.8Hz), 7.09 (1H, d, J=16.3Hz), 7.39-7.50(4H, m), 7.51 (1H, dd, J=3.1Hz, 1.7Hz), 7.60(1H, d, J=16.3Hz), 7.62(1H, dt, J=7.4Hz, 1.8Hz), 7.76(1H, t, J=1.8Hz).

EXAMPLE 83

Production of (E)-N-(6,6-dimethyl-2-hepten-4-ynyl)-N-ethyl-3-[3-(1-pyrrolyl)benzoylamino]benzylamine:-

83 mg of (E)-N-(6,6-dimethyl-2-hepten-4-ynyl)-N-ethyl-3-aminobenzylamine and 100 mg of 3-(1-pyrrolyl)-benzoic acid were dissolved in a mixture of 1 ml of methylene chloride and 2 ml of tetrahydrofuran, and 92 mg of N,N'-dicyclohexylcarbodiimide (DCC) was added. The mixture was stirred at room temperature for 3 hours. The reaction mixture was evaporated under reduced pressure, and the residue was dissolved in methylene chloride. The insoluble material was separated by filtration, and washed successively with a 5 % aqueous solution of sodium hydrogen carbonate, 5 % hydrochloric acid and a saturated aqueous solution of sodium chloride. It was dried over anhydrous sodium sulfate, and the solvent was evaporated. The residue was purified by silica gel column chromatography [Wakogel C-200, 20 g; eluting solvent: hexane/-ethyl acetate=10/1 → 3/1] to give 98 mg (yield 61 %) of the captioned compound as a pale yellow oil.

IR $\nu_{max}^{neat}$ $cm^{-1}$: 2968, 1653, 1593, 1554, 1503, 1443, 1341, 723.

NMR ($CDCl_3$) $\delta$: 1.05(3H, t, J=7.1Hz), 1.23(9H, s), 2.52(2H, q, J=7.1Hz), 3.10(2H, dd, J=6.7Hz, 1.6Hz), 3.56(2H, s), 5.65(1H, dt, J=15.8Hz, 1.6Hz), 6.08(1H, dt, J=15.8Hz, 6.7Hz), 6.37

(2H, t, J=2.5Hz), 7.11-7.15(3H, m), 7.31(1H, t, J=7.8Hz), 7.48-7.58(3H, m), 7.62-7.70 (2H, m), 7.91-7.94(2H, m).

EXAMPLE 84

Production of (E)-N-(6,6-dimethyl-2-hepten-4-ynyl)-N-methyl-3-[3-(1,3,4-triazol-1-yl)benzyloxy]-benzylamine:-

430 mg of ethyl 3-(1,3,4-triazol-1-yl)benzoate [synthesized substantially in accordance with the method described in J. Med. Chem., 5, 383 (1962)] was dissolved in a mixture of 20 ml of tetrahydrofuran and 20 ml of dioxane, and 100 mg of lithium aluminium hydride was added. The mixture was stirred at room temperature for 2 hours. The reaction mixture was concentrated under reduced pressure, and the residue was distributed between ethyl acetate and water. The organic layer was separated and dried over anhydrous sodium sulfate. The desiccant was separated by filtration, and the solvent evaporated. The residue was purified by silica gel column chromatography [Wakogel C-200, 20 g; eluting solvent: chloroform/methanol=20/1] to give 240 mg (yield 69 %) of 1-(3-hydroxymethylphenyl)-1,3,4-triazole.

220 mg of the resulting alcohol compound was dissolved in 20 ml of chloroform, and 2 ml of thionyl chloride was added. The mixture was stirred at room temperature for 2 hours. The reaction mixture was concentrated under reduced pressure and distributed between chloroform and water. The organic layer was separated, and washed with a 5 % aqueous solution of sodium hydrogen carbonate and a saturated aqueous solution of sodium chloride. After drying over anhydrous magnesium sulfate, the solvent was evaporated under reduced pressure to give 143 mg (yield 59 %) of 1-(3-chloromethylphenyl)-1,3,4-triazole.

38 mg of the resulting chloromethyl compound was dissolved in 2 ml of dimethylformamide, and the

solution was added to a tetrahydrofuran solution (2 ml) of phenolate prepared in advance from 60 mg of (E)-N-(6,6-dimethyl-2-hepten-4-ynyl)-N-methyl-3-hydroxybenzylamine and 14 mg of 60 % oily sodium hydride. The mixture was stirred at room temperature for 5 hours. Ethyl ether and water were added to the reaction mixture to separate it. The separated organic layer was collected, and dried over anhydrous magnesium sulfate. The solvent was evaporated under reduced pressure. The residue was purified by medium-pressure liquid chromatography [column: Lobar column, size A, Lichroprep Si 60 (E. Merck Co.]; eluting solvent: hexane/ethyl acetate=1/1 → 1/5] to give 48 mg (yield 59 %) of the captioned compound as a pale yellow oil.

IR $\nu_{max}^{neat}$ $cm^{-1}$: 2968, 1599, 1518, 1491, 1452, 1368, 1269, 1152, 1092, 1032, 786, 762.

NMR ($CDCl_3$) $\delta$: 1.24(9H, s), 2.19(3H, s), 3.03(2H, dd, J=6.6Hz, 1.5Hz), 3.48(2H, s), 5.15(2H, s), 5.64(1H, dt, J=15.3Hz, 1.5Hz), 6.05(1H, dt, J=15.3Hz, 6.6Hz), 6.87(1H, ddd, J=7.8Hz, 2.7Hz, 1.2Hz), 6.93(1H, d, J=7.8Hz), 6.99-7.02 (1H, m), 7.24(1H, t, J=7.8Hz), 7.35(1H, dt, J=6.9Hz, 2.4Hz), 7.50-7.59(3H, m), 8.50(2H, s).

EXAMPLE 85

Production of (E)-N-(6,6-dimethyl-2-hepten-4-ynyl)-N-ethyl-3-[3-(3-thienyl)benzylthio]benzylamine:-

10 mg of 3-[3-(3-thienyl)benzylthio]benzaldehyde was dissolved in 1 ml of ethanol, and 1.8 mg of sodium borohydride was added. The mixture was stirred at room temperature for 30 minutes. The solvent was evaporated under reduced pressure, and ethyl ether and water were added to the residue to extract it. The organic layer was separated, and dried over anhydrous magnesium sulfate. The desiccant was separated by filtration, and the solvent was evaporated. The residue was dissolved in

1 ml of ethyl acetate, and 4.4 mg of methanesulfonyl chloride and 5.9 mg of triethylamine were added. The mixture was stirred at room temperature for 10 minutes. The precipitated triethylamine hydrochloride was separated by filtration, and the solvent was evaporated. The residue was dissolved in 1 ml of dimethylformamide, and (E)-N-ethyl-6,6-dimethyl-2-hepten-4-ynylamine hydrochloride and 10 mg of potassium carbonate were added. The mixture was stirred overnight at room temperature. The reaction mixture was concentrated under reduced pressure, and the residue was dissolved in ethyl ether. The insoluble material was separated by filtration, and the solvent was evaporated under reduced pressure. The residue was purified by preparative thin-layer chromatography [thin-layer plate: Kieselgel $60F_{254}$, Art. 5744 (E. Merck Co.); developing solvent: hexane/ethyl acetate=5/1] to give 7.5 mg (yield 51 %) of the captioned compound as a colorless oil.

IR $\nu_{max}^{neat}$ $cm^{-1}$: 1478, 1363, 1265, 844, 777.

NMR ($CDCl_3$) $\delta$: 1.00(3H, t, J=7.1Hz), 1.24(9H, s), 2.44(2H, q, J=7.1Hz), 3.03(2H, d, J=6.5Hz), 3.49(2H, s), 4.15(2H, s), 5.62(1H, d, J=15.9Hz), 6.04(1H, dt, J=15.9Hz, 6.5Hz), 7.13-7.42(8H, m), 7.45(1H, t, J=1.7Hz), 7.46-7.51(2H, m).

EXAMPLE 86

Production of (E),(E)-N-(6,6-dimethyl-2-hepten-4-ynyl)-N-ethyl-3-[2-[3-(5-thiazolyl)phenyl]-ethenyl]benzylamine:-

116 mg of methyl (E)-3-[2-[3-(5-thiazolyl)-phenyl]ethenyl]benzoate [synthesized by condensing methyl (E)-3-[2-(3-bromophenyl)ethenyl]benzoate obtained by the condensation of dimethyl 3-methoxycarbonylbenzylphosphonate with 3-bromobenzaldehyde, with 5-trimethylstannylthiazole: see "Synthesis" 757 (1986)] was dissolved in 3 ml of tetrahydrofuran, and under ice cooling

14 mg of lithium aluminium hydride was added. The mixture was stirred for 30 minutes. The reaction mixture was poured into water, and ethyl ether was added to extract it. The extract was dried over anhydrous magnesium sulfate. The desiccant was separated by filtration, and the solvent was evaporated. The residue was dissolved in a mixture of 3 ml of ethyl acetate and 3 ml of methylene chloride, and 31 microliters of methanesulfonyl chloride and 70 microliters of triethylamine were added. The mixture was stirred at room temperature for 30 minutes. The triethylamine hydrochloride was separated by filtration, and the solvent evaporated under reduced pressure. The residue was dissolved in 3 ml of dimethylformamide, and 81 mg of (E)-N-ethyl-6,6-dimethyl-2-hepten-4-ynylamine hydrochloride and 42 mg of sodium carbonate were added. The mixture was stirred at room temperature for 3 hours. The reaction mixture was concentrated under reduced pressure, and ethyl ether and water were added to the residue to extract it. The organic layer separated was collected and dried over anhydrous magnesium sulfate. The desiccant was separated by filtration, and the solvent evaporated. The residue was purified by medium-pressure liquid chromatography [column: Lobar column, size A, Lichroprep Si 60 (E. Merck Co.); eluting solvent: hexane/ethyl acetate=8/1 → 6/1] to give 63 mg (yield 36 %) of the captioned compound as a cololess oil.

IR $\nu_{max}^{neat}$ $cm^{-1}$: 2968. 2872, 1605, 1458, 1392, 1365, 1266, 963, 876, 795.

NMR ($CDCl_3$) $\delta$: 1.07(3H, t, J=7.1Hz), 1.24(9H, s), 2.54(2H, q, J=7.1Hz), 3.11(1H, d, J=6.7Hz), 3.59(2H, s), 5.67(1H, d, J=15.9Hz), 6.11(1H, dt, J=15.9Hz, 6.7Hz), 7.15(2H, s), 7.29-7.57 (7H, m), 7.69(1H, dd, J=3.6Hz, 1.7Hz), 8.12 (1H, s), 8.77(1H, s).

EXAMPLE 87

Production of (E)-N-(6,6-dimethyl-2-hepten-4-ynyl)-N-methyl-3-[3-(3-tetrahydrothienyl)benzyloxy]-benzylamine:-

25 mg of 3-(3-tetrahydrothienyl)benzyl alcohol was dissolved in 5 ml of ethyl ether, and 15 microliters of methanesulfonyl chloride and 30 microliters of triethylamine were added. Under ice cooling, the mixture was stirred for 1 hour. The triethylamine hydrochloride that precipitated was separated by filtration, and the solvent evaporated. The residue was dissolved in 1 ml of dimethylformamide, and the solution was added to 10 ml of a dimethylformamide solution of phenolate prepared in advance from 100 mg of (E)-N-(6,6-dimethyl-2-hepten-4-ynyl)-N-methyl-3-hydroxybenzylamine and 30 mg of 60 % oily sodium hydride. The mixture was stirred at room temperature for 3 hours. Water and ethyl ether were added to the reaction mixture to dilute it. The organic layer was separated and dried over magnesium sulfate. The desiccant was separated by filtration, and the solvent evaporated. The residue was purified by medium-pressure liquid chromatography [column: Lobar column, size A, Lichroprep Si 60 (E. Merck Co.); eluting solvent: hexane/ethyl acetate=20/1 $\rightarrow$ 10/1] to give 42 mg (yield 75 %) of the captioned compound as a colorless oil.

IR $\nu_{max}^{neat}$ $cm^{-1}$: 1446, 1365, 1272, 1152, 1026, 885, 786, 693.

NMR ($CDCl_3$) $\delta$: 1.24(9H, s), 2.07(1H, ddt, J=12.2Hz, 10.6Hz, 8.5Hz), 2.19(3H, s), 2.42(1H, dq, J=12.2Hz, 4.6Hz), 2.88-3.01(3H, m), 3.04(2H, dd, J=6.6Hz, 1.5Hz), 3.17(1H, dd, J=10.2Hz, 6.7Hz), 3.29-3.41(1H, m), 3.47(2H, s), 5.04 (2H, s), 5.64(1H, dt, J=15.8Hz, 1.5Hz), 6.07 (1H, dt, J=15.8Hz, 6.6Hz), 6.86(1H, ddd, J=8.2Hz, 2.6Hz, 1.4Hz), 6.91(1H, d, J=7.5Hz), 6.99(1H, br.s), 7.19-7.35(4H, m), 7.37(1H, br.s).

Compounds of Examples 88 and 89 were obtained by performing the same reaction as in Example 87 except that instead of the starting 3-(3-tetrahydrothienyl)-benzyl alcohol and (E)-N-(6,6-dimethyl-2-hepten-4-ynyl)-N-methyl-3-hydroxybenzylamine, the corresponding benzyl alcohol derivativee and (E)-N-(6,6-dimethyl-2-hepten-4-ynyl)-N-ethyl-3-hydroxybenzylamine were used.

Example 88

(E)-N-(6,6-dimethyl-2-hepten-4-ynyl)-N-ethyl-3-[3-(3,4-dihydro-2*H*-thiopyran-5-yl)benzyloxy]benzylamine:-

IR $\nu_{max}^{neat}$ $cm^{-1}$: 2968, 2926, 2866, 1599, 1491, 1455, 1263, 777.

NMR ($CDCl_3$) $\delta$: 1.03(3H, t, J=7.1Hz), 1.24(9H, s), 2.14-2.23(1H, m), 2.50(2H, q, J=7.1Hz), 2.46-2.58(2H, m), 2.88-2.93(2H, m), 3.09(2H, d, J=6.5Hz), 3.54(2H, s), 5.04(2H, s), 5.64 (1H, dt, J=15.9Hz, 1.5Hz), 6.06(1H, dt, J=15.9Hz, 6.5Hz), 6.45(1H, s), 6.85(1H, dd, J=8.4Hz, 2.0Hz), 6.92(1H, d, J=7.2Hz), 7.00 (1H, br.s), 7.19-7.36(4H, m), 7.39(1H, br.s).

Example 89

(E)-N-(6,6-dimethyl-2-hepten-4-ynyl)-N-ethyl-3-[3-(5,6-dihydro-2*H*-thiopyran-3-yl)benzyloxy]benzylamine:-

IR $\nu_{max}^{neat}$ $cm^{-1}$: 3034, 2968, 2926, 1740, 1602, 1491, 1458, 1263.

NMR ($CDCl_3$) $\delta$: 1.04(3H, t, J=7.1Hz), 1.24(9H, s), 2.45-2.59(4H, m), 2.77(2H, t, J=5.7Hz), 3.09 (2H, d, J=6.5Hz), 3.50(2H, dd, J=3.9Hz, 2.1Hz), 3.55(2H, br.s), 5.05(2H, s), 5.65 (1H, d, J=15.9Hz), 6.07(1H, dt, J=15.9Hz, 6.5Hz), 6.11-6.17(1H, m), 6.83-6.89(1H, m), 6.92(1H, d, J=7.5Hz), 7.01(1H, br.s), 7.19-7.36(4H, m), 7.40(1H, br.s).

EXAMPLE 90

Production of a powder containing the compound of Example 19 as an active ingredient:-

25 parts of the compound (hydrochloride) of Example 19 was dissolved in a mixture of 500 parts of ethanol and 500 parts of chloroform, and 75 parts of polyvinyl pyrrolidone K-30 was added. The mixture was evaporated to dryness under reduced pressure by a conventional method. The residual solid was pulverized to a fine powder, and uniformly mixed with 250 parts of lactose, 145 parts of corn starch and 5 parts of magnesium stearate to form a powder containing 25 mg of the active ingredient per 500 mg.

EXAMPLE 91

Production of capsules containing the compound of Example 19 as an active ingredient:-

25 parts of the compound (hydrochloride) of Example 19 was dissolved in a mixture of 500 parts of ethanol and 500 parts of chloroform, and 72.5 parts of polyvinyl pyrrolidone K-30 and 2.5 parts of Tween 60 were added. The mixture was evaporated to dryness under reduced pressure by a conventional method. The residual solid was pulverized to a fine powder, and uniformly mixed with 50 parts of lactose, 45 parts of corn starch and 5 parts of magnesium stearate. The powder was filled in hard gelatin capsules in an amount of 200 mg per capsule to give capsules containing 25 mg of the active ingredient per capsule.

EXAMPLE 92

Production of capsules containing the compound of Example 19 as an active ingredient:-

25 parts of the compound (hydrochloride) of Example 19 was suspended in 1000 parts of water, and 150 parts of beta-cyclodextrin was added. The mixture was stirred at room temperature for 12 hours. 1000 parts of water was further added, and the mixture was stirred for

an additional 3 hours at room temperature. The mixture was lyophilized by a conventional method, and the resulting cotton-like solid was lightly pulverized. The particles were filled in hard gelatin capsules in an amount of 70 mg per capsule to form capsules containing 10 mg of the active ingredient per capsule.

The following Referential Examples illustrate the general synthesizing methods of the starting compounds used in the foregoing Examples.

REFERENTIAL EXAMPLE 1

Production of (E)-N-(6,6-dimethyl-2-hepten-4-ynyl)-N-methyl-3-hydroxybenzylamine:-

10.0 g of 3-hydroxybenzaldehyde and 9.55 g of a 40 % methanol solution of methylamine were mixed, and then the solvent was evaporated. The resulting Schiff base was dissolved in 50 ml of ethanol, and with stirring under ice cooling, 10.0 g of sodium borohydride was added. The mixture was stirred overnight at room temperature. The solvent was evaporated under reduced pressure, and ethyl acetate and a saturated aqueous solution of sodium chloride were added to extract it. The organic layer was separated, and dried over anhydrous sodium sulfate. The solvent was evaporated, and the residue was purified by silica gel column chromatography [Wakogel C-100, 100 g; eluting solvent: methylene chloride/methanol=10/1 → 5/1] to give 8.88 g (yield 79 %) of N-methyl-3-hydroxybenzylamine as pale yellow crystals, m.p. 138-140 °C.

The resulting N-methylamine compound (8.88 g) and 18.0 g of potassium carbonate were added to 30 ml of dimethylformamide. With stirring at room temperature, a dimethylformamide solution (10 ml) of 13.0 g of 1-bromo-6,6-dimethyl-2-hepten-4-yne (a mixture of the E-form and the Z-form in a ratio of about 3:1) was added. The mixture was stirred overnight at room temperature. After the reaction, the solvent was evaporated. The

residue was extracted with a mixture of ethyl acetate and a saturated aqueous solution of sodium chloride. The organic layer was separated and dried over anhydrous magnesium sulfate. The desiccant was separated by filtration, and the solvent was evaporated. The residue was purified by silica gel column chromatography [Wakogel C-200, 300 g; eluting solvent: hexane/ethyl acetate=10/1] to give 7.94 g (total yield 39 %) of the captioned compound as pale yellow crystals, m.p. 76-77 °C.

The 3-hydroxybenzylamine derivatives used in Examples 1 to 48, such as (E)-N-(6,6-dimethyl-2-hepten-4-ynyl)-N-ethyl-3-hydroxybenzylamine, (E)-N-(6,6-dimethyl-2-hepten-4-ynyl)-N-propyl-3-hydroxybenzylamine or (E)-N-ethyl-N-(6-methoxy-6-methyl-2-hepten-4-ynyl)-3-hydroxybenzylamine, were obtained by performing the same reaction as in Referential Example 1 except that in place of the starting methylamine-methanol solution, a methanol solution of ethylamine or propylamine was used and as required, 1-bromo-6-methoxy-6-methyl-2-hepten-4-yne was used instead of 1-bromo-6,6-dimethyl-2-hepten-4-yne was used.

REFERENTIAL EXAMPLE 2

Production of 3-(2-furyl)benzyl chloride:-

Ethyl 3-(2-furyl)benzoate [J. Chem. Soc., (B), 1971, 2305] was dissolved in 5 ml of anhydrous ethyl ether. With stirring under ice cooling, 23 mg of lithium aluminium hydride was added. The mixture was stirred for 40 minutes. After the reaction, water and ethyl ether were added to extract the reaction mixture. The extract was worked up in a customary manner to give 170 mg (yield 83 %) of 3-(2-furyl)benzyl alcohol.

REFERENTIAL EXAMPLE 3

Production of 3-(1-pyrrolyl)benzylmethanesulfonate:-

1.6 g of ethyl m-aminobenzoate was dissolved in 10 ml of glacial acetic acid, and 1.3 g of 2,5-dimethoxy-

tetrahydrofuran was added. The mixture was heated under reflux for 2 hours. The solvent was evaporated, and the residue was dissolved in ethyl acetate and water. The organic layer was separated and worked up in a customary manner, and finally recrystallized from hexane to give 1.7 g (yield 82 %) of purified ethyl 3-(1-pyrrolyl)-benzoate as colorless needles, m.p. 64-65 °C.

1.1 g of the resulting pyrrolyl compound was dissolved in 30 ml of ethyl ether, and with stirring under ice cooling, 0.2 g of lithium aluminium hydride was added. The mixture was stirred for 1 hour. Water and ethyl ether were added to the reaction mixture to extract it. The extract was worked up in a customary manner and then recrystallized from a mixture of ethyl acetate and hexane to give 0.80 g (yield 91 %) of 3-(1-pyrrolyl)-benzyl alcohol as colorless needles, m.p. 66-68 °C.

170 mg of the resulting alcohol compound was dissolved in 10 ml of methylene chloride, and 120 mg of methanesulfonyl chloride and 150 mg of triethylamine were added. The mixture was stirred under ice cooling for 1 hour. The reaction mixture was washed with water, and dried over anhydrous magnesium sulfate. Evaporation of the solvent gave 230 mg (yield 92 %) of the captioned compound as a pale yellow oil.

REFERENTIAL EXAMPLE 4

Production of 3-(3-thienyl)benzylmethane-sulfonate:-

790 mg of magnesium was suspended in 1 ml of anhydrous tetrahydrofuran, and a minute amount of 1,2-dibromoethane was added. After determining the occurrence of bubbling, a tetrahydrofuran solution (12 ml) of 5 g of 3-bromobenzaldehyde dimethyl acetal was added dropwise at room temperature over 1.5 hours with stirring. The mixture was stirred at 45 to 55 °C for 30 minutes, and then 80 mg of bis(diphenylphosphino)ethane nickel (II) chloride and a tetrahydrofuran solution

(6 ml) of 3-bromothiophene were added. The mixture was stirred at room temperature for 3 hours. A saturated aqueous solution of ammonium chloride was added to the reaction mixture, and the insoluble material was separated by filtration. Ethyl acetate was added to extract the reaction mixture. The extract was treated in a customary manner and purified by silica gel column chromatography [Wakogel C-100, 70 g; eluting solvent: hexane/ethyl acetate=50/1 $\rightarrow$ 10/1] to give 1.35 g (yield 27 %) of 3-(3-thienyl)benzaldehyde dimethyl acetate, m.p. 45-46 $^{o}$C.

The resulting thienyl compound (1.35 g) was dissolved in a mixture of 4 ml of 1N hydrochloric acid and 8 ml of tetrahydrofuran. The solution was stirred at room temperature for 1 hour, and the solvent was evaporated under reduced pressure. The residue was extracted with ethyl acetate and water. The extract was worked up in a customary manner to give 1.05 g (yield 96 %) of 3-(3-thienyl)benzaldehyde, m.p. 44-45 $^{o}$C.

1.05 g of the resulting aldehyde compound was dissolved in 15 ml of ethanol, and 250 mg of sodium borohydride was added. The mixture was stirred at room temperature for 30 minutes. The reaction mixture was concentrated under reduced pressure, and the residue was extracted with ethyl acetate and water. The extract was worked up in a customary manner and purified by silica gel column chromatography [Wakogel C-100, 20 g; eluting solvent: hexane/ethyl acetate=5/1] to give 960 mg (yield 86 %) of 3-(3-thienyl)benzyl alcohol, m.p. 89-90 $^{o}$C.

300 mg of the resulting alcohol compound was dissolved in 7 ml of ethyl acetate, and to its solution, with stirring under ice cooling, 320 mg of triethylamine and an ethyl acetate solution (1 ml) of 270 mg of methanesulfonyl chloride were added. The mixture was stirred at room temperature for 30 minutes. The precipitated salt was separated by filtration, and the

reaction mixture was washed with a saturated aqueous solution of sodium hydrogen carbonate, and dried over anhydrous magnesium sulfate. The solvent was evaporated under reduced pressure to give the captioned compound as a pale yellow powder.

When the starting 3-bromothiophene is replaced by the corresponding bromine-substituted heterocyclic derivative in Referential Example 4, a 3-(3-furyl)benzyl derivative, a 3-(2-thienyl)benzyl derivative, a 3-(2-pyridyl)benzyl derivative, a 3-(3-pyridyl)benzyl derivative and a 3-(4-pyridyl)benzyl derivative are obtained.

REFERENTIAL EXAMPLE 5

Production of 5-(3-methylphenyl)isoxazole:-

1.56 g of 3-ethynyltoluene was dissolved in 20 ml of ethyl ether, and with stirring at -70 $^{o}$C, 8.5 ml of 1.5 M n-butyllithium-hexane solution and 1.2 ml of ethyl formate were added. The mixture was stirred at the same temperature for 20 minutes. The reaction mixture was poured into ice water, and the organic layer was separated. The solvent was evaporated to give 900 mg (yield 47 %) of 3-(3-methylphenyl)-2-propynaldehyde as a colorless oil.

130 mg of the resulting aldehyde was dissolved in 20 ml of ethanol, and 5 ml of an aqueous solution of 70 mg of hydroxylamine hydrochloride was added. The solvent was evaporated under reduced pressure. The residue was purified by medium-pressure liquid chromatography [column: Lobar column, size A, Lichroprep Si 60 (E. Merck Co.); eluting solvent: hexane/ethyl acetate=-10/1] to give 122 mg (yield 85 %) of 3-(3-methylphenyl)-2-propynaldehyde oxime as a pale yellow oil.

122 mg of the resulting oxime compound was dissolved in 20 ml of ethanol, and one drop of a 1N aqueous solution of sodium hydroxide was added. The solution was left to stand for 3 minutes, and three drops of 1N HCl was added to terminate the reaction. The

solvent was evaporated under reduced pressure. The residue was extracted with a mixture of water and ethyl ether. The organic layer was separated, and the solvent was evaporated to give 106 mg (yield 87 %) of the captioned compound as a pale yellow oil.

REFERENTIAL EXAMPLE 6

Production of 1-(3-hydroxymethylphenyl)imidazole:-

230 mg of 1-(3-ethoxycarbonylphenyl)imidazole [see J. Am. Chem. Soc., 79, 4922 (1957)] was dissolved in 10 ml of ethyl ether, and 50 mg of lithium aluminium hydride was added. The mixture was stirred at room temperature for 2 hours. The reaction mixture was poured into ice water. The organic layer was separated and dried over anhydrous magnesium sulfate. The desiccant was separated by filtration. The solvent was evaporated and then the residue was purified by silica gel column chromatography [Wakogel C-200, 20 g; eluting solvent: chloroform/methanol=20/1] to give 160 mg (yield 92 %) of the captioned compound as a colorless oil.

REFERENTIAL EXAMPLE 7

Production of 3-(2-oxazolyl)benzyl bromide:-

200 mg of 2-(3-methylphenyl)oxazole [synthesized substantially in accordance with the method described in Ang. Chem., 75, 165 (1963)] was dissolved in 5 ml of carbon tetrachloride, and 231 mg of N-bromosuccinimide and a catalytic amount of benzoyl peroxide were added. The mixture was refluxed for 2 hours with stirring. After the reaction, the insoluble material was separated by filtration, and the solvent was evaporated under reduced pressure to give the captioned compound.

By a similar method, 3-(2-thiazolyl)benzyl bromide can be synthesized.

REFERENTIAL EXAMPLE 8

Production of 3-(5-oxazolyl)benzyl alcohol:-

400 mg of 3-(hydroxymethyl)benzaldehyde

(produced by reducing isophthalaldehyde with an equimolar sodium borohydride), 574 mg of p-toluenesulfonylmethyl isocyanide and 406 mg of potassium carbonate were added to 10 ml of methanol. With stirring, the mixture was heated under reflux for 1 hour. The reaction mixture was concentrated under reduced pressure, and the residue was extracted with ethyl acetate and water. The extract was worked up in a customary manner, and purified by silica gel column chromatography [Wakogel C-200, 50 g; eluting solvent: hexane/ethyl acetate=1/1] to give 375 mg (yield 73 %) of the captioned compound as a white powder.

When the above alcohol compound is mesylated by the same method as in Referential Example 3, 3-(5-oxazolyl)benzylmethanesulfonate is obtained as a colorless oil.

REFERENTIAL EXAMPLE 9

Production of 3-(4-isoxazolyl)benzyl bromide:-

185 mg of 4-(3-methylphenyl)isoxazole [see J. Heterocyclic Chem., 11, 51 (1974); and J. Chem. Soc., Perkin II, 1121 (1977)] was dissolved in 8 ml of carbon tetrachloride, and 206 mg of N-bromosuccinimide and a catalytic amount of benzoyl peroxide were added. The mixture was refluxed with stirring for 3 hours. The insoluble material was separated by filtration and the solvent was evaporated. The residue was purified by silica gel column chromatography [Wakogel C-200, 30 g; eluting solvent: hexane/ethyl acetate=10/1] to give 210 mg (yield 76 %) of the captioned compound as a colorless powder

When the same reaction as in Referential Example 9 is carried out except using 4-(3-methylphenyl)isothiazole [see J. prakt. Chem., 318, 507 (1976)], 5-(3-methylphenyl)isothiazole [see J. prakt. Chem., 318, 507 (1976)], 5-(3-methylphenyl)pyrimidine [see J. Heterocyclic Chem., 11, 55 (1974); Heterocycles, 19, 1080 (1982)], or 1-(3-methylphenyl)-1,2,4-triazole

[see J. Org. Chem., 21, 1037 (1956), and Japanese Laid-Open Patent Publication No. 4173/1976] instead of the starting 4-(3-methylphenyl)isoxazole, 3-(4-isothiazolyl)-benzyl bromide, 3-(5-isothiazolyl)benzyl bromide, 3-(5-pyrimidyl)benzyl bromide or 3-(1,2,4-triazol-1-yl)-benzylbromide are obtained.

REFERENTIAL EXAMPLE 10

Production of 3-(2,3-dihydro-4-thienyl)benzyl alcohol and 3-(2,5-dihydro-3-thienyl)benzyl alcohol:-

A tetrahydrofuran solution (10 ml) of tetrahydrothiophen-3-one was added dropwise with stirring under ice cooling to a tetrahydrofuran solution (15 ml) of a Grignard reagent prepared from 2.31 g of 3-bromobenzaldehyde dimethyl acetal and 0.36 g of metallic magnesium. After the addition, the mixture was stirred at room temperature for 1 hour. The reaction mixture was poured into ice water, and ethyl ether was added to extract the product. The extract was worked up in a customary manner to give 1.20 g (yield 47 %) of a crude product of 3-(3-hydroxytetrahydro-3-thienyl)benzaldehyde dimethyl acetal.

1.20 g of the resulting alcohol compound was dissolved in a mixture of 20 ml of tetrahydrofuran and 3 ml of 10 % HCl. After standing for 2 hours at room temperature, ethyl ether and water were added to the solution to dilute it. The organic layer was separated, and the solvent was evaporated under reduced pressure. The residue was dissolved in 20 ml of methylene chloride, and 1.0 ml of methanesulfonyl chloride and 2.0 ml of triethylamine were added. The mixture was stirred under ice cooling for 30 minutes. The reaction mixture was poured into ice water, and the organic layer was separated and the solvent was evaporated to dryness under reduced pressure. The residue was dissolved in 10 ml of ethanol, and 0.2 g of sodium borohydride was added. The solution was stirred at room temperature for 1 hour. The

reaction mixture was concentrated under reduced pressure, and ethyl ether and water were added to the residue to extract it. The extract was worked up in a customary manner, and the product was purified by medium-pressure liquid chromatography [Lobar column, size B, Lichroprep Si 60 (E. Merck & Co.); eluting solvent: hexane → hexane/ethyl acetate 10/1] to give 0.45 g (yield 50 %) of 3-(2,3-dihydro-4-thienyl)benzyl alcohol and 0.20 g (yield 22 %) of 3-(2,5-dihydro-3-thienyl)benzyl alcohol.

REFERENTIAL EXAMPLE 11

Production of 3-(1-pyrrolidinyl)benzyl alcohol:-

0.25 g of N-(3-ethoxycarbonylphenyl)succinimide [synthesized by heat condensing ethyl m-aminobenzoate with succinic anhydride in acetic acid] was dissolved in 5 ml of anhydrous tetrahydrofuran, and with stirring under ice cooling, 0.17 g of lithium aluminium hydride was added. The mixture was maintained at room temperature for 30 minutes, and then heated under reflux for 4 hours. After the reaction, ethyl acetate and water were added. The organic layer was separated, and then worked up in a customary manner and purified by medium-pressure liquid chromatography [Lobar column, size B, Lichroprep Si 60 (E. Merck & Co.); eluting solvent: hexane/ethyl acetate=10/1] to give 84 mg (yield 47 %) of the captioned compound as a colorless oil.

When the resulting alcohol compound is mesylated by the same method as in Referential Example 4, 3-(1-pyrrolidinyl)benzyl methanesulfonate is obtained as a colorless oil.

REFERENTIAL EXAMPLE 12

Production of 5-(3-thienyl)thienylmethyl alcohol:-

5 g of 3-bromothiophene was dissolved in 35 ml of anhydrous ethyl ether, and with stirring under cooling at -70 to -65 $^{o}C$, 19 ml of a 15 % n-butyllithium-hexane

solution and 10.5 g of tributyltin chloride were added. The mixture was stirred at the above temperature for 1 hour and then at room temperature for 1 hour. The reaction mixture was washed with a saturated aqueous solution of sodium hydrogen carbonate, and then the solvent was evaporated. The residue was purified by distillation under reduced pressure to give 8.5 g (yield 74 %) of tributyl(3-thienyl)tin, b.p. 150-158 °C/2 mmHg.

1.19 g of the above tin compound and 0.56 g of 5-bromothiophene-2-carbaldehyde were dissolved in 3 ml of toluene, and 20 mg of tetrakis(triphenylphosphine)-palladium was added. The mixture was heated with stirring under reflux for 7 hours. The reaction mixture was washed with a 10 % aqueous solution of potassium fluoride and a 5 % aqueous solution of potassium carbonate. The solvent was evaporated, and the residue was purified by silica gel column chromatography [Wakogel C-200, 50 g; eluting solvent: hexane/ethyl acetate=10/1] to give 0.36 g (yield 63 %) of 5-(3-thienyl)thiophene-2-carbaldehyde.

144 mg of the resulting aldehyde compound was reduced with 15.6 mg of lithium aluminium hydride in 4 ml of tetrahydrofuran, and then worked up in a customary manner to give 134 mg (yield 92 %) of the captioned compound as a colorless oil.

REFERENTIAL EXAMPLE 13

Production of 2-(5-oxazolyl)-4-pyridinemethanol:-

1.07 g of dimethyl pyridine-2,4-dicarboxylate was dissolved in 20 ml of toluene, and with stirring under cooling at -80 to -70 °C, 6.04 ml of a 1M toluene solution of diisobutylaluminium hydride was added dropwise over 2.5 hours, and the mixture was stirred at this temperature for 1 hour. The reaction mixture was poured into ice water, and ethyl ether was added. The organic layer was separated, worked up in a customary manner, and

then purified by medium-pressure liquid chromatography [Lobar column, size B, Lichroprep Si 60 (E. Merck Co.); eluting solvent: hexane/ethyl acetate=4/1 → 3/1] to give 0.27 g (yield 30 %) of methyl 2-formylisonicotinate as a colorless crystalline powder.

203 mg of the resulting formyl compound was dissolved in 8 ml of methanol, and 240 mg of p-toluenesulfonylmethyl isocyanide and 170 mg of potassium carbonate were added. The mixture was heated under reflux for 10 minutes. The reaction mixture was evaporated to dryness under reduced pressure. The residue was extracted with methylene chloride and water. The organic layer was worked up in a customary manner to give 224 mg (yield 91 %) of methyl 2-(5-oxazolyl)isonicotinate as a pale yellow powder.

102 mg of the resulting oxazolyl compound was dissolved in 2 ml of anhydrous tetrahydrofuran, and with stirring under ice cooling, 14 mg of lithium aluminium hydride was added. The mixture was stirred at this temperature for 30 minutes. The reaction mixture was poured into ice water, and methylene chloride was added to extract it. The extract was worked up in a customary manner and purified by silica gel column chromatography [Wakogel C-200, 5.5 g; eluting solvent: methylene chloride/methanol=50/1 → 20/1] to give 49.5 mg (yield 56 %) of the captioned compound as a pale yellow crystalline powder.

REFERENTIAL EXAMPLE 14

Production of 5-(5-oxazolyl)-3-pyridinemethanol:-

1.67 g of dimethyl pyridine-3,5-dicarboxylate was dissolved in 30 ml of anhydrous tetrahydrofuran, and with stirring under ice cooling, 162 mg of lithium aluminium hydride was added. The mixture was stirred at this temperature for 30 minutes. The reaction mixture was poured into ice water, and ethyl ether was added

to extract it. The extract was worked up in a customary manner to give crude methyl 5-hydroxymethylnicotinate. The crude product was dissolved in 20 ml of methylene chloride, and 2.2 g of pyridinium chlorochromate was added. The mixture was stirred overnight at room temperature. The reaction mixture was poured into ice water. The organic layer was separated, worked up in a customary manner, and purified by silica gel column chromatography [Wakogel C-200, 80 g; eluting solvent: chloroform/methanol=20/1] to give 0.37 g (yield 26 %) of methyl 5-formylnicotinate, m.p. 96-97 °C.

110 mg of the resulting formyl compound was used as a starting material, and subjected to oxazolylation and reduction as in Referential Example 13 to give 77 mg (yield 64 %) of the captioned compound as a colorless oil.

When the same reaction as in Referential Example 14 is carried out using dimethyl pyridine-2,6-dicarboxylate instead of the starting dimethyl pyridine-3,5-dicarboxylate, 6-(5-oxazolyl)-2-pyridinemethanol is obtained.

REFERENTIAL EXAMPLE 15

Production of 5-(5-hydroxymethyl-2-furyl)-oxazole:-

300 mg of 5-formylfurfuryl alcohol [see Japanese Laid-Open Patent Publication No. 154758/1979] was dissolved in 10 ml of methanol, and 502 mg of p-toluenesulfonylmethyl isocyanide and 329 mg of potassium carbonate were added. The mixture was heated under reflux for 1 hour. The reaction mixture was concentrated under reduced pressure, and the residue was dissolved in a mixture of water and ethyl acetate. The organic layer was separated, and dried over anhydrous magnesium sulfate. The desiccant was separated by filtration, and the solvent was evaporated under reduced pressure. The residue was purified by silica gel column chromatography

[Wakogel C-200, 45 g; eluting solvent: hexane/ethyl acetate=3/2 → 1/1] to give 260 mg (yield 66 %) of the captioned compound as a pale yellow crystalline powder.

REFERENTIAL EXAMPLE 16

Production of 4-hydroxymethyl-2-(5-oxazolyl)-thiazole:-

1.5 g of diethyl thiazole-2,4-dicarboxylate was suspended in 15 ml of ethanol, and at -10 °C, 0.16 g of sodium borohydride and 3 ml of an ethanol solution of 0.58 g of calcium chloride were added. The mixture was stirred at the above temperature for 2 hours. Acetone was added to decompose the excess of the reducing agent, and the solvent was evaporated under reduced pressure. Dilute sulfuric acid was added to the residue, and insoluble calcium sulfate was separated by filtration. The filtrate was adjusted to pH 10 with an aqueous solution of potassium carbonate, and then extracted with chloroform. The chloroform layer was dried over anhydrous magnesium sulfate and evaporated to dryness under reduced pressure. The residue was treated with isopropyl ether to give 0.78 g (yield 64 %) of ethyl 2-hydroxymethylthiazole-4-carboxylate as a colorless crystalline powder.

0.78 g of the resulting hydroxymethyl compound was dissolved in 40 ml of chloroform, and 25 g of active manganese dioxide was added. The mixture was stirred at room temperature for 5 days. The precipitate was separated by filtration, and the filtrate was evaporated to dryness under reduced pressure to give 0.67 g (yield 87 %) of ethyl 2-formylthiazole-4-carboxylate as colorless needles.

60 mg of ethyl 2-(5-oxazolyl)thiazxole-4-carboxylate prepared by reacting 80 mg of the resulting formyl compound, 91 mg of p-toluenesulfonylmethyl isocyanide and 60 mg of potassium carbonate as in Referential Example 13 was dissolved in 3 ml of ethanol. To the solution were added 6.6 mg of sodium borohydride and

24 mg of calcium chloride. The mixture was stirred at room temperature for 1 hour. The reaction mixture was concentrated under reduced pressure, and 10 % sulfuric acid was added to the residue. The resulting precipitate was separated by filtration, and potassium carbonate was added to the filtrate to adjust its pH to 10. Chloroform was added to extract the product. The extract was dried over anhydrous magnesium sulfate, and the solvent was evaporated. The residue was purified by preparative thin-layer chromatography [thin-layer plate: Kieselgel $60F_{254}$, Art. 5744 (E. Merck Co.); developing solvent: hexane/ethyl acetate=1/4] to give 20 mg (yield 25 %) of the captioned compound as a white crystalline powder.

## REFERENTIAL EXAMPLE 17

Production of 5-(5-hydroxymethyl-3-furyl)-oxazole:-

408 mg of dimethyl furan-3,5-dicarboxylate [see J. Chem. Soc., Perkin I, 1130 (1973)] was dissolved in 4 ml of anhydrous tetrahydrofuran, and 59 mg of lithium aluminium hydride was added. The mixture was stirred overnight at room temperature. The reaction mixture was poured into water, and ethyl acetate was added to extract it. The extract was worked up in a customary manner and purified by silica gel column chromatography [Wakogel C-200, 20 g; eluting solvent: hexane/ethyl acetate=2/1] to give 44 mg (yield 13 %) of methyl 5-hydroxymethyl-furan-3-carboxylate as a colorless oil.

44 mg of the resulting alcohol compound was dissolved in 2 ml of chloroform, and 60 mg of pyridinium chlorochromate was added. The mixture was stirred overnight at room temperature. The reaction mixture was poured into ice water, and the organic layer was separated, worked up in a customary manner, and purified by silica gel column chromatography [Wakogel C-200, 2 g; eluting solvent: hexane/ethyl acetate=3/1] to give 28 mg

of a purified formyl compound. It was dissolved in 2 ml of methanol, and 35 mg of p-toluenesulfonylmethyliso-cyanide and 25 mg of potassium carbonate were added. The mixture was refluxed for 30 minutes, and worked up in a customary manner to give 30 mg of methyl 5-(5-oxazolyl)-furan-3-carboxylate. The resulting compound was dissolved in 2ml of tetrahydrofuran, and with ice cooling, 6 mg of lithium aluminium hydride was added. The mixture was stirred at the above temperature for 30 minutes. The reaction mixture was poured into ice water, and ethyl ether was added to extract it. The extract was worked up in a customary manner to give 22 mg (yield 48 %) of the captioned compound as a pale yellow oil.

REFERENTIAL EXAMPLE 18

Production of 6-methyl-3-(1-pyrrolyl)benzyl alcohol:-

158 mg of methyl 3-amino-6-methylbenzoate was dissolved in 3 ml of acetic acid, and 139 mg of 2,5-dimethoxytetrahydrofuran was added. The mixture was heated under reflux for 1 hour. The reaction mixture was evaporated to dryness under reduced pressure. The residue was purified by silica gel column chromatography [Wakogel C-200, 10 g; eluting solvent: hexane/ethyl acetate=10/1] to give 185 mg (yield 87 %) of methyl 3-(1-pyrrolyl)-6-methylbenzoate, m.p. 56-57 $^{o}$C.

180 mg of the resulting pyrrolyl compound was dissolved in 2 ml of ethyl ether, and 24 mg of lithium aluminium hydride was added. The mixture was stirred at room temperature for 30 minutes. Water and ethyl ether were added to the reaction mixture. The organic layer was separated and dried over anhydrous magnesium sulfate. The desiccant was separated by filtration, and the solvent was evaporated to give 155 mg (yield quantitative) of the captioned compound as a colorless crystalline powder, m.p. 70-71 $^{o}$C.

When the same reaction as in Referential

Example 18 is carried out using methyl 3-amino-2-methylbenzoate instead of the starting methyl 3-amino-6-methylbenzoate, 2-methyl-3-(1-pyrrolyl)benzyl alcohol is obtained.

REFERENTIAL EXAMPLE 19

Production of (E)-N-(6,6-dimethyl-2-hepten-4-ynyl)-N-methyl-3-(3-formylbenzyloxy)benzylamine:-

A dimethylformamide solution (1.5 ml) of 90 mg of 3-chloromethylbenzaldehyde was added to a phenolate solution prepared from 150 mg of (E)-N-(6,6-dimethyl-2-hepten-4-ynyl)-N-methyl-3-hydroxybenzylamine, 23.2 mg of 60 % oily sodium hydride and 1 ml of tetrahydrofuran. The mixture was stirred overnight at room temperature. The solvent was evaporated under reduced pressure. The residue was extracted with ethyl acetate-water, worked up in a customary manner, and purified by silica gel column chromatography [Wakogel C-200, 15 g; eluting solvent: hexane/ethyl acetate=10/1 → 5/1] to give 85 mg (yield 39 %) of the captioned compound as a colorless oil.

When the same reaction as in Referential Example 19 is carried out using methyl 3-bromomethylbenzoate instead of the starting 3-chloromethylbenzaldehyde, (E)-N-(6,6-dimethyl-2-hepten-4-ynyl)-N-methyl-3-(3-methoxycarbonylbenzyloxy)benzylamine is obtained.

REFERENTIAL EXAMPLE 20

Production of (E)-3-[2-[3-(3-thienyl)phenyl]ethenyl]benzyl chloride:-

2.40 g of 3-(3-thienyl)benzyl chloride was dissolved in 40 ml of toluene, and 3.62 g of triphenylphosphine was added. The mixture was heated under reflux for 50 hours. The reaction mixture was allowed to cool, and the precipitated crystals were collected by filtration to give 3.60 g (yield 66 %) of 3-(3-thienyl)benzyltriphenylphosphonium chloride.

1.21 g of the resulting phosphonium salt was dissolved in 45 ml of ethanol, and 0.35 g of 3-formyl-

benzyl alcohol and 0.27 g of sodium ethoxide were added. The mixture was stirred at room temperature for 2 hours. The solvent was evaporated under reduced pressure. The residue was distributed between ethyl acetate and water, worked up in a customary manner, and purified by silica gel column chromatography [Wakogel C-300, 100 g; eluting solvent: hexane/ethyl acetate=10/1 → 5/1] to give 0.34 g (yield 46 %) of an E-form of 3-[2-[3-(3-thienyl)phenyl]-ethenyl]benzyl alcohol and 0.34 g of its Z-form.

0.20 g of the resulting (E)-form alcohol was dissolved in 6 ml of chloroform, and 0.15 ml of thionyl chloride and one drop of dimethylformamide were added. The mixture was stirred at room temperature for 1 hour. The reaction mixture was concentrated under reduced pressure. The residue was distributed between ethyl ether and water, worked up in a customary manner, and purified by silica gel column chromatography [Wakogel C-100, 20 g; eluting solvent: hexane/ethyl acetate=20/1] to give 0.19 g (yield 87 %) of the captioned compound as a colorless crystalline powder, m.p. 79-81 $^{o}$C.

When the same reaction as in Referential Example 20 is carried out except that instead of the starting 3-(3-thienyl)benzyl chloride, the corresponding 3-substituted benzyl chloride or bromide is used, (E)-3-[2-[3-(5-oxazolyl)phenyl]ethenyl]benzyl chloride, (E)-3-[2-[3-(5-thiazolyl)phenyl]ethenyl]benzyl chloride and (E)-3-[2-[3-(1-imidazolyl)phenyl]ethenyl]benzyl chloride are obtained.

REFERENTIAL EXAMPLE 21

Production of (E)-3-[2-[3-(1-pyrrolyl)phenyl]-ethenyl]benzyl alcohol:-

560 mg of 3-(dimethoxymethyl)benzyltriphenyl-phosphonium bromide [synthesized by brominating 3-hydroxymethylbenzaldehyde with phosphorus tribromide, and then acetalizing the product in anhydrous methanol in the presence of p-toluenesulfonic acid, followed by reacting

it with triphenylphosphine] and 180 mg of 3-(1-pyrrolyl)-benzaldehyde [synthesized by oxidizing 3-(1-pyrrolyl)-benzyl alcohol with pyridinium chlorochromate in chloroform] were dissolved in 20 ml of methanol. 110 mg of sodium methoxide was added, and the mixture was stirred at room temperature for 3 hours. The reaction mixture was concentrated under reduced pressure, and ethyl acetate and water were added to the residue to extract it. The organic layer was worked up in a customary manner, and purified by medium-pressure liquid chromatography [Lobar column, size B, Lichroprep Si 60 (E. Merck Co.); eluting solvent: hexane/ethyl acetate=10/1] to give 135 mg (yield 42 %) of an E-form of 3-[2-[3-(1-pyrrolyl)-phenyl]ethenyl]benzaldehyde dimethyl acetal and 127 mg of its Z-form.

135 mg of the resulting (E)-form acetal was dissolved in a mixture of 5 ml of tetrahydrofuran and 5 ml of 2N HCl. The solution was stirred at room temperature for 3 hours. The solvent was then evaporated under reduced pressure. The residue was worked up in a customary manner. The resulting formyl compound was dissolved in 10 ml of ethanol, and 40 mg of sodium borohydride was added. The mixture was stirred at room temperature for 30 minutes. The reaction mixture was concentrated under reduced pressure. The residue was distributed between ethyl acetate and water, worked up in a customary manner, and purified by silica gel column chromatography [Wakogel C-100, 20 g; eluting solvent: hexane/ethyl acetate=5/1] to give 78 mg (yield 67 %) of the captioned compound as a colorless crystalline powder, m.p. 108-110 $^{o}$C.

When the same reaction as in Referential Example 21 is carried out using 3-(3-pyridyl)benzaldehyde instead of the starting 3-(1-pyrrolyl)benzaldehyde, (E)-3-[2-[3-(3-pyridyl)phenyl]ethenyl]benzyl alcohol is obtained.

REFERENTIAL EXAMPLE 22

Production of 3-[2-[3-(3-thienyl)phenyl]ethyl]-benzyl alcohol:-

81 mg of (Z)-3-[2-[3-(3-thienyl)phenyl]-ethenyl]benzyl alcohol was dissolved in 3 ml of ethanol, and in the presence of 15 mg of 10 % palladium-carbon, catalytically reduced at ordinary temperature and atmospheric pressure for 15 hours. The catalyst was separated by filtration, and the solvent was evaporated to give the captioned compound as a colorless oil in a quantitative yield.

When the same reduction as in Referential Example 22 is carried out using (Z)-3-[2-[3-(1-pyrrolyl)-phenyl]ethenyl]benzyl alcohol or (Z)-3-[2-[3-(3-pyridyl)-phenyl]ethenyl]benzyl alcohol instead of the starting (Z)-3-[2-[3-(3-thienyl)phenyl]ethenyl]benzyl alcohol, 3-[2-[3-(1-pyrrolyl)phenyl]ethyl]benzyl alcohol and 3-[2-[3-(3-pyridyl)phenyl]ethyl]benzyl alcohol are obtained.

REFERENTIAL EXAMPLE 23

Production of (E)-N-(6,6-dimethyl-2-hepten-4-ynyl)-N-ethyl-5-formylfurfurylamine:-

100 mg of 5-hydroxymethylfurfural was dissolved in 2 ml of anhydrous ethyl ether, and with stirring under ice cooling, an ethyl ether solution (1 ml) of 30 microliters of phosphorus tribromide was added. The mixture was stirred at the above temperature for 10 minutes. The reaction mixture was washed with a saturated aqueous solution of sodium hydrogen carbonate. Then, 183 mg of (E)-N-ethyl-6,6-dimethyl-2-hepten-4-ynylamine hydrochloride, 136 mg of potassium carbonate and 3 ml of dimethylformamide were added, and the mixture was stirred overnight at room temperature. The reaction mixture was concentrated under reduced pressure. The residue was distributed between ethyl ether and water, worked up in a customary manner, and purified by silica gel column

chromatography to give 132 mg (yield 53 %) of the captioned compound as a pale yellow oil.

REFERENTIAL EXAMPLE 24

Production of (E)-N-(6,6-dimethyl-2-hepten-4-ynyl)-N-methyl-4-formyl-2-pyridylmethylamine:-

160 mg of (E)-N-(6,6-dimethyl-2-hepten-4-ynyl)-N-methyl-4-ethoxycarbonyl-2-pyridylmethylamine [synthesized by tosylating 4-ethoxycarbonyl-2-pyridinemethanol with p-toluenesulfonyl chloride and triethylamine, and thereafter condensing the tosylated product with (E)-N-methyl-6,6-dimethyl-2-hepten-4-ynylamine] was dissolved in 2 ml of toluene, and with stirring under cooling at -75 to -70 °C, 0.56 ml of a 1M toluene solution of diisobutyl aluminium hydride was added. The mixture was stirred at the above temperature for 40 minutes. The reaction mixture was poured into ice water, and ethyl ether was added. The organic layer was separated, worked up in a customary manner, and purified by medium-pressure liquid chromatography [Lobar column, size A, Lichroprep Si 60 (E. Merck Co.); eluting solvent: hexane/ethyl acetate=4/1] to give 15 mg (yield 11 %) of the captioned compound as a pale yellow oil.

The same reaction as in Referential Example 24 is carried out except that instead of the starting (E)-N-(6,6-dimethyl-2-hepten-4-ynyl)-N-methyl-4-ethoxycarbonyl-2-pyridylmethylamine, (E)-N-(6,6-dimethyl-2-hepten-4-ynyl)-N-ethyl-3-ethoxycarbonyl-5-isoxazolylmethylamine [synthesized by brominating ethyl 5-methylisoxazole-3-carboxylate with N-bromosuccinimide and condensing the brominated product with (E)-N-ethyl-6,6-dimethyl-2-hepten-4-ynylamine] is used, (E)-N-(6,6-dimethyl-2-hepten-4-ynyl)-N-ethyl-3-formyl-5-isoxazolylamine is obtained.

REFERENTIAL EXAMPLE 25

Production of (E)-N-(6,6-dimethyl-2-hepten-4-ynyl)-N-methyl-3-hydroxy-4-methoxymethyloxybenzylamine:-

3 g of 3,4-dihydroxybenzaldehyde and 0.87 g of 60 % oily sodium hydride were suspended in 13 ml of tetrahydrofuran, and a dimethylformamide solution (10 ml) of 3.3 ml of methoxymethyl chloride was added. The mixture was stirred at room temperature for 1 hour. The reaction mixture was concentrated under reduced pressure. The residue was distributed between ethyl acetate and water, the organic layer was worked up in a customary manner, and then purified by silica gel column chromatography [Wakogel C-200, 150 g; eluting solvent: hexane/ethyl acetate=4/1] to give 1.78 g (yield 45 %) of 3-hydroxy-4-methoxymethyloxybenzaldehyde.

360 mg of the resulting methoxymethyloxy compound was dissolved in 6 ml of a 40 % methanol solution of methylamine, and 151 mg of sodium borohydride was added. The mixture was stirred for 1 hour at room temperature. The reaction mixture was concentrated under reduced pressure. The residue was distributed between ethyl acetate and water. The organic layer was separated, and the solvent was evaporated. The residue was dissolved in 10 ml of dimethylformamide, and 361 mg of 1-bromo-6,6-dimethyl-2-hepten-4-yne (a 3:1 mixture of the E-form and Z-form) and 276 mg of potassium carbonate were added. The mixture was stirred overnight at room temperature. The reaction mixture was concentrated under reduced pressure. The residue was distributed between ethyl ether and water. The organic layer was separated, worked up in a customary manner, and purified by silica gel column chromatography [Wakogel C-200, 30 g; eluting solvent: hexane/ethyl acetate=3/1] to give 140 mg (yield 22 %) of the captioned compound as a pale yellow oil.

When 2,3-dihydroxybenzaldehyde is used instead of the starting 3,4-dihydroxybenzaldehyde, and selectively alkylated with methoxymethyl chloride and triethylamine in chloroform to synthesize 3-hydroxy-2-methoxymethyloxybenzaldehyde and thereafter the same

reaction as in Referential Example 25 is carried out, (E)-N-(6,6-dimethyl-2-hepten-4-ynyl)-N-methyl-3-hydroxy-2-methoxymethyloxybenzylamine is obtained.

REFERENTIAL EXAMPLE 26

Production of (E)-N-(6,6-dimethyl-2-hepten-4-ynyl)-N-ethyl-3-aminobenzylamine:-

6.0 g of N-(3-bromomethylphenyl)phthalimide [synthesized by brominating N-(m-tolyl)phthalimide with N-bromosuccinimide in carbon tetrachloride] was dissolved in 100 ml of dimethylformamide, and 3.82 g of (E)-N-ethyl-6,6-dimethyl-2-hepten-4-ynylamine hydrochloride and 7.87 g of potassium carbonate were added. The mixture was stirred overnight at room temperature. The reaction mixture was concentrated under reduced pressure. The residue was extracted with ethyl acetate-water and the organic layer was separated, and the solvent was evaporated, and then the residue was washed with a small amount of ethyl ether to give 5.5 g (yield 72 %) of (E)-N-(6,6-dimethyl-2-hepten-4-ynyl)-N-ethyl-3-phthalimidobenzylamine, m.p. 96-98 $^{o}C$.

150 mg of the resulting benzylamine compound was dissolved in 5 ml of ethanol, and 23 mg of hydrazine was added. The mixture was stirred at room temperature for 30 minutes. The precipitate was separated by filtration, and the solvent was evaporated. The residue was distributed between methylene chloride and water. The organic layer was worked up in a customary manner and purified by silica gel column chromatography [Wakogel C-100, 5 g; eluting solvent: hexane/ethyl acetate=10/1 → 3/1] to give 95 mg (yield 95 %) of the captioned compound as a pale yellow crystalline powder, m.p. 65-66 $^{o}C$.

REFERENTIAL EXAMPLE 27

Production of 3-[3-(3-thienyl)benzylthio]benzaldehyde:-

0.3 g of sulfur was added to a tetrahydrofuran

solution (20 ml) of a Grignard reagent prepared from 1.0 g of 3-bromobenzaldehyde dimethyl acetal and 0.4 g of magnesium, and the mixture was stirred at room temperature for 2 hours. 0.3 g of lithium aluminium hydride was added and the mixture was stirred at 40 °C for 3 hours. The reaction mixture was poured into ice water, and hydrochloric acid was added to acidify it. Ethyl ether was added to extract it. The extract was worked up in a customary manner, and the solvent was evaporated to give 0.36 g (yield 60 %) of 3-mercaptobenzaldehyde.

60 mg of the resulting mercapto compound was dissolved in 5 ml of dimethylformamide, and 50 mg of 3-(3-thienyl)benzylbromide and 30 mg of 60 % oily sodium hydride were added. The mixture was stirred at room temperature for 3 hours. The reaction mixture was poured into ice water, worked up in a customary manner, and purified by medium-pressure liquid chromatography [column: Lobar column, size A, Lichroprep Si 60 (E. Merck Co.); eluting solvent: hexane/ethyl acetate=30/1 → 10/1] to give 2.3 mg (yield 4 %) of the captioned compound as a colorless oil.

REFERENTIAL EXAMPLE 28

Production of 3-(3-tetrahydrothienyl)benzyl alcohol:-

30 mg of the 3-(2,5-dihydro-3-thienyl)benzyl alcohol obtained in Referential Example 10 was dissolved in 250 ml of ethanol, and in the presence of 50 mg of 10 % palladium-carbon, catalytically reduced for 8 hours under a hydrogen pressure of 3.5 $kg/cm^2$. The catalyst was separated by filtration, and the solvent was evaporated under reduced pressure to give 25 mg (yield 82 %) of the captioned compound as a colorless oil.

REFERENTIAL EXAMPLE 29

Production of 3-(3,4-dihydro-2*H*-thiopyran-5-yl)benzyl alcohol and 3-(5,6-dihydro-2*H*-thiopyran-3-yl)benzyl alcohol:-

534 mg of 3-(tetrahydro-3-hydroxy-3-thiopyranyl)benzaldehyde dimethyl acetal obtained by performing the same reaction as in Referential Example 10 using 3-bromobenzaldehyde dimethyl acetal and tetrahydrothiopyran-3-one as starting materials was dissolved in 5 ml of ethyl acetate, and with stirring under ice cooling, 187 microliters of methanesulfonyl chloride and 553 microliters of triethylamine were added. The mixture was stirred for 30 minutes. The triethylamine hydrochloride was separated by filtration and the solvent was evaporated. The residue was dissolved in 20 ml of benzene, and 373 mg of 90 % potassium tert-butoxide was added, and the mixture was stirred at room temperature for 15 hours. Ethyl ether and water were added to the reaction mixture, and the mixture was worked up in a customary manner. The resulting 3-dihydrothiopyranylbenzaldehyde dimethyl acetal was dissolved in a mixture of 3 ml of 1N HCl and 6 ml of tetrahydrofuran. The solution was stirred at room temperature for 3 hours. The reaction mixture was concentrated under reduced pressure, and the residue was distributed between ethyl ether and water, and worked up in a customary manner. The resulting formyl compound was dissolved in 20 ml of tetrahydrofuran, and 380 mg of lithium aluminium hydride was added. The mixture was stirred for one hour under ice cooling. The reaction mixture was poured into ice water, worked up in a customary manner and purified by silica gel column chromatography [Wakogel C-200, 20 g; eluting solvent: hexane/ethyl acetate=2/1] to give 7 mg (yield 2 %) of the captioned 3-(3,4-dihydro-2*H*-thiopyran-5-yl)benzyl alcohol and 4 mg (yield 1 %) of 3-(5,6-dihydro-2*H*-thiopyran-3-yl)benzyl alcohol.

## Utilizability in Industry

The compounds of this invention inhibit biosynthesis of cholesterol by inhibiting mammalian squalene epoxidase, and lower the blood cholesterol

level. Accordingly, they can be expected to be effective as therapeutic and prophylactic agents for diseases induced by excessive cholesterol, for example, obesity, hyperlipemia, arterioscrelosis and heart and brain diseases incidental to them.

**SCOPE OF CLAIM FOR PATENT**

(1) A substituted allylamine derivative represented by the following general formula

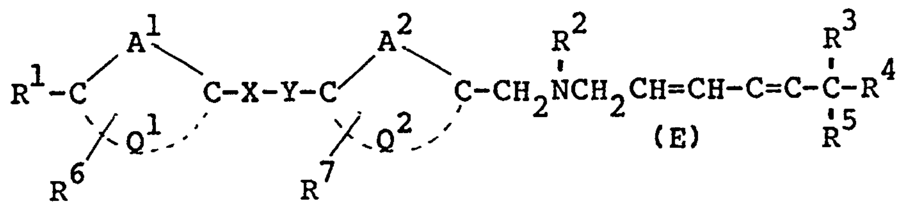

[I]

wherein

$A^1$ and $A^2$ are identical or different and each represents a methine group or a nitrogen, oxygen or sulfur atom;

$Q^1$ and $Q^2$ are identical or different and each represents a group which may contain 1 or 2 hetero atoms selected from the class consisting of nitrogen, oxygen and sulfur atoms and which forms a 5- or 6-membered aromatic ring together with the adjoining carbon atoms and $A^1$ or $A^2$;

X and Y are identical or different, and each represents an oxygen or sulfur atom, a carbonyl group, a group of the formula $-CHR^a-$ in which $R^a$ represents a hydrogen atom or a lower alkyl group, or a group of the formula $-NR^b-$ in which $R^b$ represents a hydrogen atom or a lower alkyl group, or when taken together, X and Y form a vinylene or ethynylene group;

$R^1$ represents a 5- or 6-membered heterocyclic group containing 1 to 4 hetero atoms selected from the class consisting of nitrogen, oxygen and sulfur atoms;

$R^2$ represents a lower alkyl group, an allyl group, a propargyl group or a cyclopropyl group;

$R^3$ and $R^4$ are identical or different and each represents a lower alkyl group, or they represent groups which when taken together, form a cycloalkane together with the adjoining carbon atom;

$R^5$ represents a hydrogen atom, a lower alkyl group or a lower alkoxy group; and

$R^6$ and $R^7$ are identical or different and each represents a hydrogen atom, a halogen atom, a hydroxyl group, a cyano group, a lower alkyl group, or a lower alkoxy group;

with the proviso that when one of X and Y represents an oxygen atom, a sulfur atom or the group $-NR^b-$ (where $R^b$ is as defined), the other represents a carbonyl group or the group $-CHR^a-$ (where $R^a$ is as defined);

and its nontoxic salt.

(2) The substituted allylamine derivative and its nontoxic salt set forth in claim 1 in which $R^1$ is a pyrrolyl, furyl, thienyl, oxazolyl, isoxazolyl, thiazolyl, isothiazolyl, imidazolyl, pyrazolyl, oxadiazolyl, thiadiazolyl, triazolyl, tetrazolyl, furazanyl, pyridyl, pyridazinyl, pyrimidinyl, pyrazinyl, triazinyl, dihydrothienyl, tetrahydrothienyl, pyrrolinyl, pyrrolidinyl, oxazolinyl, oxazolidinyl, isoxazolinyl, isoxazolidinyl, thiazolinyl, thiazolidinyl, isothiazolinyl, isothiazolidinyl, 1,2-dithiolanyl, 1,3-dithiolanyl, 1,2-dithiolyl, 1,3-dithiolyl, dihydrothiopyranyl, tetrahydrothiopyranyl, 1,4-dithianyl, 1,4-dithiinyl, 1,4-oxathiinyl or thiomorpholinyl group; the 5- or 6-membered aromatic rings represented by the formulae

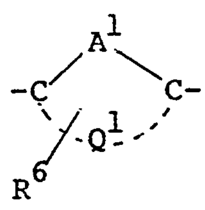

or

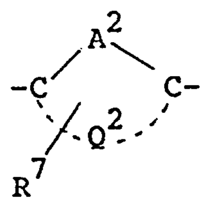

are identical or different, and each represents a benzene, pyrrole, furan, thiophene, oxazole, isoxazole, thiazole, isothiazole, imidazole, 1,3,4-oxadiazole, 1,3,4-thiadiazole, pyridine, pyridazine, pyrimidine, pyrazine or triazine ring.

(3) The substituted allylamine derivative and its nontoxic salt set forth in claim 2 in which the aromatic ring represented by the formula

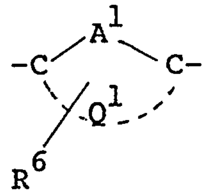

is a benzene or thiophene ring.

(4) The substituted allylamine derivative and its nontoxic salt set forth in claim 1 in which $R^1$ is a thienyl, pyrrolyl, oxazolyl, isoxazolyl, thiazolyl, isothiazolyl, imidazolyl, pyridyl or dihydrothienyl; the aromatic ring represented by the formula

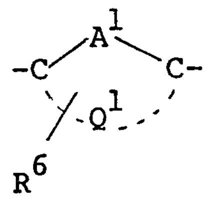

is a benzene or thiophene ring; and the 5- or 6-membered aromatic ring represented by the following formula

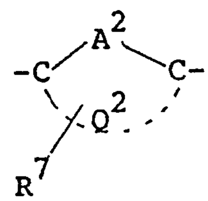

is a benzene, pyrrole, furan, thiophene, oxazole, isoxazole, thiazole, isothiazole, imidazole, 1,3,4-oxadiazole, 1,3,4-thiadiazole, pyridine, pyridazine, pyrimidine, pyrazine or triazine ring.

(5) The allylamine derivative and its nontoxic salt set forth in claim 4 in which $R^1$ is a 3-thienyl, 1-pyrrolyl, 5-oxazolyl, 4-isoxazolyl, 5-isoxazolyl, 4-thiazolyl, 5-thiazolyl, 3-isothiazolyl, 4-isothiazolyl, 5-isothiazolyl, 3-pyridyl, 2,3-dihydro-4-thienyl or 2,5-dihydro-3-thienyl group.

(6) The substituted allylamine derivative and its nontoxic salt set forth in claim 4 or 5 in which the 5- or 6-membered aromatic ring represented by the formula

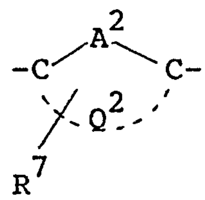

is a benzene, furan, thiophene, oxazole, isoxazole, thiazole, isothiazole, 1,3,4-oxadiazole, 1,3,4-thiadiazole, pyridine, pyridazine, pyrimidine or pyrazine ring.

(7) The substituted allylamine derivative and its nontoxic salt set forth in claims 4, 5 or 6 in which X is a methylene group, and Y is a methylene group, an oxygen atom, a sulfur atom or an imino group, or X and Y taken together represent an (E)-vinylene group.

(8) The substituted allylamine derivative and its nontoxic salt set forth in claim 7 in which $R^2$ is a methyl, ethyl or propyl group, and $R^3$ and $R^4$ are methyl groups, and $R^5$ is a methyl, ethyl or methoxy group.

(9) A process for producing the substituted allylamine derivative of general formula [I] set forth in claim 1 and its nontoxic salt, which comprises

reacting a substituted amine derivative represented by the general formula

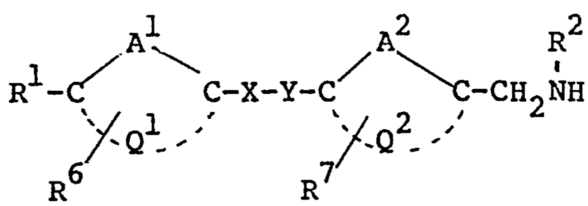 [II]

wherein $A^1$, $A^2$, $Q^1$, $Q^2$, X, Y, $R^1$, $R^2$, $R^6$ and $R^7$ are as defined in claim 1,

or its protected compound with a compound represented by the general formula

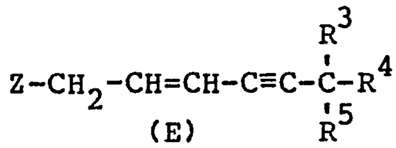 [III]

wherein Z represents a leaving group, and $R^3$, $R^4$, and $R^5$ are as defined in claim 1 and then as required, eliminating the protective group, or reacting a compound represented by the general formula

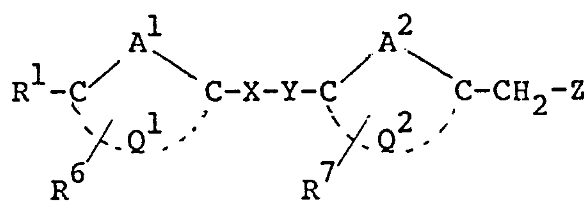 [IV]

wherein $A^1$, $A^2$, $Q^1$, $Q^2$, X, Y, $R^1$, $R^6$, $R^7$ and Z are as defined above, or its protected derivative with a substituted amine derivative represented by the general formula

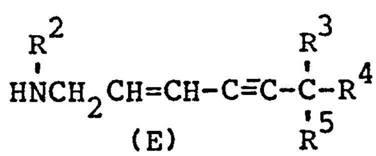 [V]

wherein $R^2$, $R^3$, $R^4$ and $R^5$ are as defined above, and then, as required, eliminating the protective group, and then as required, converting the product into its nontoxic salt.

(10) A process for producing the substituted allylamine derivative of general formula [I] given in claim 1 and its nontoxic salt, which comprises reacting a substituted allylamine derivative of the general formula

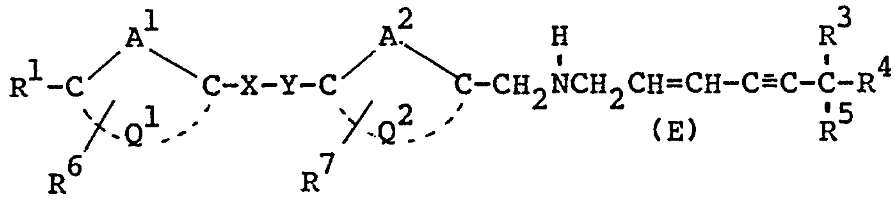 [VI]

wherein $A^1$, $A^2$, $Q^1$, $Q^2$, X, Y, $R^1$, $R^3$, $R^4$, $R^5$, $R^6$ and $R^7$ are as defined in claim 1,

or its protected derivative with a compound represented by the general formula

$$Z-R^2 \qquad [VII]$$

wherein $R^2$ is as defined in claim 1, and Z represents a leaving group,

then as required, eliminating the protective group, and then as required, converting the product into a nontoxic salt.

(11) A process for producing a substituted allylamine derivative represented by the general formula

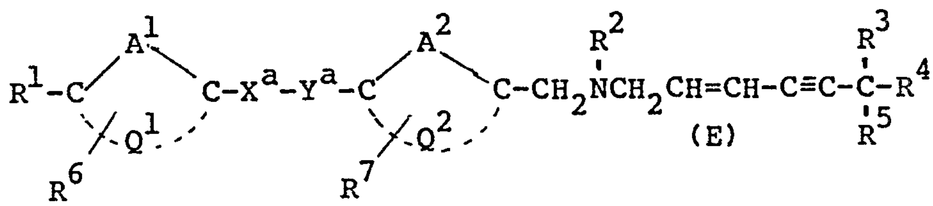 [$I^a$]

wherein $A^1$, $A^2$, $Q^1$, $Q^2$, $X^a$, $Y^a$, $R^1$, $R^2$, $R^3$, $R^4$, $R^5$, $R^6$ and $R^7$ are as defined below,

and its nontoxic salt, which comprises reacting a compound represented by the general formula

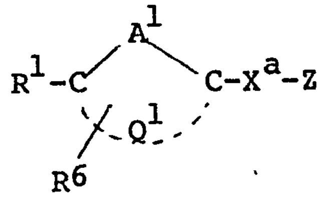 [VIII]

wherein $X^a$ represents a carbonyl group or a group of the formula $-CHR^a-$ (where $R^a$ represents a hydrogen atom or a lower alkyl group), Z represents a leaving group, and $A^1$, $Q^1$, $R^1$ and $R^6$ are as defined in claim 1,

or its protective derivative with a substituted amine derivative represented by the general formula

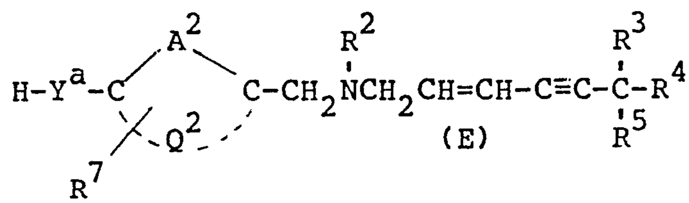

[IX]

wherein $Y^a$ represents an oxygen atom, a sulfur atom, or a group of the formula $-NR^b-$ (where $R^b$ represents a hydrogen atom or a lower alkyl group, and $A^2$, $Q^2$, $R^2$, $R^3$, $R^4$, $R^5$ and $R^7$ are as defined in claim 1,

or its protected compound, then as required, eliminating the protective group, and then as required converting the product into a nontoxic salt.

(12) A process for producing a substituted allylamine derivative represented by the general formula

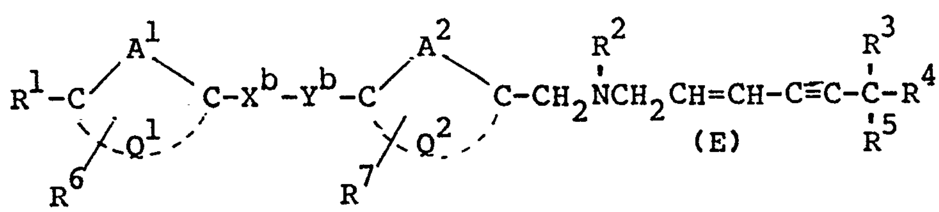

[$I^b$]

wherein $A^1$, $A^2$, $Q^1$, $Q^2$, $X^b$, $Y^b$, $R^1$, $R^2$, $R^3$, $R^4$, $R^5$, $R^6$ and $R^7$ are as defined below,

and its nontoxic salt, which comprises reacting a compound represented by the general formula

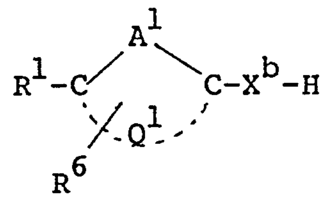

[X]

wherein $X^b$ represents an oxygen atom, a sulfur atom, or a group of the formula $-NR^b-$ (where $R^b$ represents a hydrogen atom or a lower alkyl group), and $A^1$, $Q^1$, $R^1$ and $R^6$ are as defined in claim 1,

or its protected derivative with a substituted amine derivative represented by the general formula

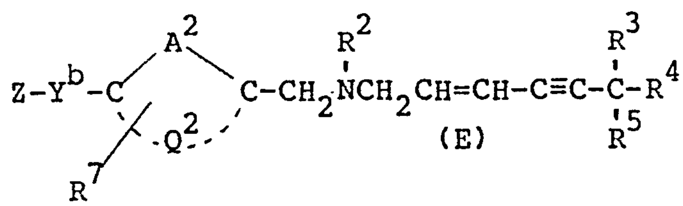

[XI]

wherein $Y^b$ represents a carbonyl group or a group of the formula $-CHR^a-$ (where $R^a$ represents a hydrogen atom or a lower alkyl group), Z represents a leaving group, and $A^2$, $Q^2$, $R^2$, $R^3$, $R^4$, $R^5$ and $R^7$ are as defined in claim 1,

or its protected group, then as required, eliminating the protective group, and then as required, converting the product into a nontoxic salt.

(13) A process for producing a substituted allylamine derivative represented by the general formula

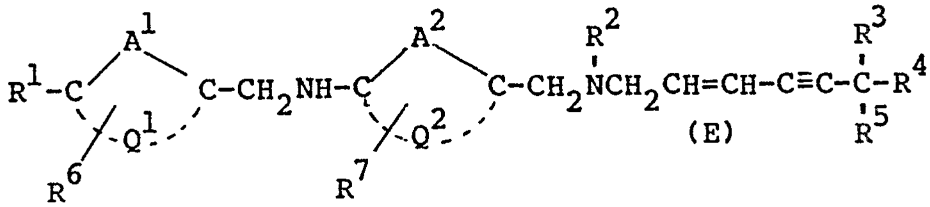

$[I^c]$

wherein $A^1$, $A^2$, $Q^1$, $Q^2$, $R^1$, $R^2$, $R^3$, $R^4$, $R^5$, $R^6$ and $R^7$ are as defined below,

and its nontoxic salt, which comprises reacting a compound represented by the general formula

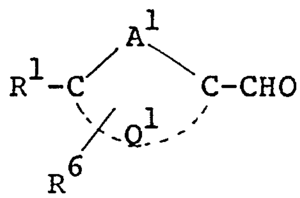

[XII]

wherein $A^1$, $Q^1$, $R^1$ and $R^6$ are as defined in claim 1,

with a compound represented by the general formula

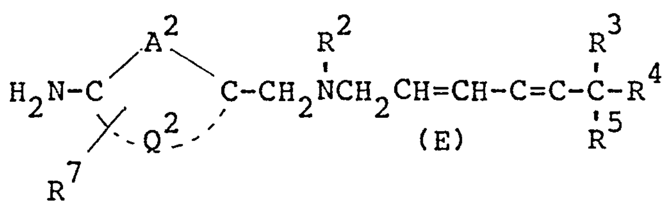

[XIII]

wherein $A^2$, $Q^2$, $R^2$, $R^3$, $R^4$, $R^5$ and $R^7$ are as defined in claim 1,

and thereafter reducing the product.

(14) An agent for treating hypercholesterolemia comprising the substituted allylamine derivative of general formula [I] or its nontoxic salt set forth in claim 1.

(15) An agent for treating hyperlipemia comprising the substituted allylamine derivative of general formula [I] or its nontoxic salt set forth in claim 1.

(16) An agent for treating arteriosclerosis comprising the substituted allylamine derivative of general formula [I] or its nontoxic salt set forth in claim 1.

# INTERNATIONAL SEARCH REPORT

International Application No PCT/JP89/00522

**I. CLASSIFICATION OF SUBJECT MATTER** (if several classification symbols apply, indicate all) [6]

According to International Patent Classification (IPC) or to both National Classification and IPC

Int. $Cl^4$ C07D207/325, 207/33, 213/24, 233/56, 239/26, 249/06, 261/08, 263/32, 271/10, 275/02, 277/22, 307/38, 333/06, 413/04, 417/04, A61K31/33

**II. FIELDS SEARCHED**

Minimum Documentation Searched [7]

| Classification System | Classification Symbols |
|---|---|
| IPC | C07D207/325-33, 213/24-54, 233/56-61, 239/26, 249/06, 261/08, 263/32, 271/10, 275/02, 277/22, 307/38, 333/06, 413/04, 417/04, A61K31/33 |

Documentation Searched other than Minimum Documentation to the Extent that such Documents are Included in the Fields Searched [8]

**III. DOCUMENTS CONSIDERED TO BE RELEVANT** [9]

| Category * | Citation of Document, [11] with indication, where appropriate, of the relevant passages [12] | Relevant to Claim No. [13] |
|---|---|---|
| A | Chemical Abstracts. 100 (11):85436C (DE, A1, 3316093 (Sandog-Patent-G.m.b.H.) 10 November 1983 (10. 11. 83)) | 1 - 16 |

* Special categories of cited documents: [10]

"A" document defining the general state of the art which is not considered to be of particular relevance

"E" earlier document but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step

"Y" document of particular relevance; the claimed invention cannot be considered to involve an inventive step when the document is combined with one or more other such documents, such combination being obvious to a person skilled in the art

"&" document member of the same patent family

**IV. CERTIFICATION**

| Date of the Actual Completion of the International Search | Date of Mailing of this International Search Report |
|---|---|
| June 29, 1989 (29. 06. 89) | August 21, 1989 (21. 08. 89) |
| **International Searching Authority** | **Signature of Authorized Officer** |
| Japanese Patent Office | |